# EUROPÄISCHE PATENTANMELDUNG

(11) **EP 4 098 643 A1**
(43) Veröffentlichungstag der Anmeldung: **07.12.2022**
(21) Anmeldenummer: 21177066.4
(22) Anmeldetag: 01.06.2021
(51) Int. Cl.: C07C 13/52, C07F 17/02, C23C 18/42

(54) **EDELMETALLKOMPLEXE MIT DIHYDROGUAJAZULENYL-LIGANDEN UND DEREN VERWENDUNG**

(71) Anmelder: UMICORE AG & Co. KG, 63457 Hanau-Wolfgang (DE)
(72) Erfinder: Vollgraff, Tobias, 35037 Marburg (DE); SUNDERMEYER, Joerg, 35041 Marburg (DE); DOPPIU, Angelino, 63500 Seligenstadt (DE)
(74) Vertreter: Clauswitz, Kai-Uwe Wolfram

(57) **Zusammenfassung**

Die Erfindung betrifft ein Verfahren zur Herstellung von Komplexen der Edelmetalle, insbesondere von Platin, welche wenigstens einen Organo-dihydroazulenyl-Liganden aufweisen. Gegenstand der Erfindung sind außerdem Komplexe der Edelmetalle, insbesondere von Platin, welche wenigstens einen Organo-dihydroazulenyl-Liganden aufweisen. Ferner betrifft die Erfindung die Verwendung der vorgenannten Metallkomplexe als Präkatalysatoren oder Katalysatoren in einer chemischen Reaktion oder als Präkursorverbindungen zur Herstellung einer Schicht, welche ein Edelmetall, insbesondere Platin enthält, oder einer Metallschicht bestehend aus einem Edelmetall, insbesondere Platin, insbesondere auf wenigstens einer Oberfläche eines Substrats. Außerdem ist Gegenstand der Erfindung ein Substrat, insbesondere erhältlich nach einem solchen Verfahren. Zudem ist Gegenstand der Erfindung eine vernetzbare Silikonzusammensetzung, umfassend wenigstens eine Verbindung mit aliphatischen Kohlenstoff-Kohlenstoff-Mehrfachbindungen, wenigstens eine Verbindung mit Si-gebundenen Wasserstoffatomen und wenigstens einen Platin(IV)-Komplex des vorgenannten Typs. Weiterhin betrifft die Erfindung neue Alkalimetall-organo-dihydroazulenide, welche zur Herstellung von Metallkomplexen, insbesondere des vorgenannten Typs, verwendbar sind.

## Beschreibung

Homoleptische und heteroleptische Metallkomplexe mit anionischen Liganden, welche von Bicyclo[5.3.0]decapentaen (Azulen) oder einfach oder mehrfach substituiertem Azulen, d. h. Azulenderivaten, abgeleitet sind, sowie Verfahren zu ihrer Herstellung sind bekannt.

Azulen und Azulenderivate, welche z. B. in 4-Position anstelle eines H-Atoms einen Alkyl- oder Arylsubstituenten aufweisen, gehören zu den *ortho*-anellierten aromatischen Ringsystemen. Sie liegen als neutrale Zwitterionen vor.

Anionische Liganden, die von erdölbasiertem Azulen oder von üblicherweise erdölbasierten Azulenderivaten abgeleitet sind, können beispielsweise durch Addition eines Alkalimetallorganyls, z. B. Methyllithium oder Phenyllithium, in 4-Position, in 6-Position oder in 8-Position eines Azulenmoleküls oder eines Azulenderivats hergestellt werden. Das jeweilige Produkt ist ein Alkalimetall-dihydroazulenid, welches in 4-Position, 6-Position oder 8-Position zusätzlich zu einem H-Atom eine Organylgruppe trägt, z. B. eine Methylgruppe oder eine Phenylgruppe. Mithin liegt in C4-Position oder in C6-Position oder in C8-Position des Azulengerüsts eine RCH-Gruppe vor, wobei R eine Organylgruppe ist.

Es sei angemerkt, dass Azulen in 4-Position, 6-Position und 8-Position eine vergleichbare Elektrophilie aufweist. Daher kann es zur Bildung von Regioisomeren kommen. Weil Azulen zudem eine C₂ᵥ-Symmetrie aufweist, handelt es sich bei den Produkten der Additionsreaktion um ein Alkalimetall-4-organo-dihydroazulenid und/oder ein Alkalimetall-6-organo-dihydroazulenid. Das jeweilige Anion kann als 4-Organo-dihydroazulenyl-Anion oder als 6-Organo-dihydroazulenyl-Anion bezeichnet werden. Genauer gesagt handelt es sich um ein 4-Organo-3a,4-dihydroazulenyl-Anion oder ein 6-Organo-3a,6-dihydroazulenyl-Anion.

Unabhängig vom Substitutionsmuster wird die Aromatizität durch die Alkyl- oder Aryl-Addition auf den Fünfring beschränkt. Dabei geht in der Regel die für Azulen typische blaue Farbe verloren.

Bei den vorgenannten Organo-dihydroazulenyl-Anionen, beispielsweise einem vom Naturstoff 7-*iso-*Propyl-1,4-dimethylazulen (= Guajazulen) abgeleiteten Organo-dihydroguajazulenyl-Anion, handelt es sich um Cyclopentadienyl-artige Monoanionen bzw. Derivate des Cyclopentadienyl-Anions (Cp⁻). Daher eignen sie sich ebenso wie das Cyclopentadienyl-Anion unter anderem als Liganden für die Darstellung von *Sandwich*-Komplexen vom Metallocen-Typ. Lithium-Dihydroazulenide besitzen - insbesondere hinsichtlich Herstellung und Lagerung - gegenüber Lithium-Cyclopentadienid (LiCp) einige Vorteile.

Nachteilig an der Chemie des Cyclopentadienyl-Liganden, eines der wichtigsten Liganden der Metallorganik, ist zum einen, dass sie erdölbasiert ist. Die Bereitstellung von LiCp umfasst zunächst das thermische Cracken von Dicyclopentadien in sein Monomer Cyclopentadien in Gegenwart eines Katalysators, z. B. Eisenpulver. Nach diesem ersten Schritt muss Cyclopentadien zügig verbraucht oder im Gefrierschrank gelagert werden. Ansonsten dimerisiert es rasch wieder zu Dicyclopentadien. In einem zweiten Schritt erfolgt die Umsetzung von Cyclopentadien mit einer starken Base, z. B. einem Lithiumalkyl, üblicherweise dem relativ kostenintensiven *n*-Butyllithium.

Beispiele für Übergangsmetallkomplexe, welche Dihydroazulenyl-Anionen, d. h. Cyclopentadienyl-artige Monoanionen bzw. Cyclopentadienyl-Derivate als Liganden aufweisen, sind unter anderem in einem Überblicksartikel von M. R. Churchill beschrieben. (Prog. Inorg. Chem. 1970, 54 - 98, Kapitel IV., Abschnitt C.)

Hafner und Weldes (Liebigs Ann. Chem. 1957, 606, 90 - 99) untersuchten das Verhalten von Azulen gegenüber metallorganischen Verbindungen. Ausgehend von Methyllithium und Azulen (äquimolar) erhielten sie das Dietherat von Lithium-4-methyl-dihydro-azulen. Die Reaktion verlief exotherm. Zudem wurden Butyllithium, Phenyllithium sowie natrium- und kaliumorganische Verbindungen mit Azulen umgesetzt und die entsprechenden in 4-Stellung substituierten Azulene erhalten.

Erst kürzlich publizierte die Gruppe um Edelmann (J. Richter, P. Liebing, F. T. Edelmann, Inorg. Chim. Acta 2018, 475,18 - 27) ihre Ergebnisse betreffend die Synthese von Metallocen-Komplexen der frühen Übergangsmetalle Titan und Zirkonium und des Lanthanoids Neodym, welche Dihydroazulenyl-Liganden aufweisen. Die im Rahmen der Metallkomplex-Synthesen eingesetzten Lithium-Dihydroazulenide, nämlich Lithium-4-methyl-dihydroazulenid, Lithium-7-*iso-*propyl-1,4,8-trimethyl-dihydroazulenid und Lithium-7-*iso*-propyl-1,4-dimethyl-8-phenyl-dihydroazulenid, wurden analog zu der Methode von Hafner und Weldes (Liebigs Ann. Chem. 1957, 606, 90 - 99) dargestellt, d. h. ausgehend von Azulen oder Guajazulen und Methyl- oder Phenyllithium. Um solvensfreie Produkte zu erhalten, wurden die isolierten Lithium-Dihydroazulenide jedoch mit n-Pentan anstelle von Diethylether gewaschen.

Die Autoren stellen zusammenfassend fest, dass ihnen Dihydroazulenyl-Anionen und ähnliche von Azulen abgeleitete Anionen als Liganden für die Darstellung von Metallocenen früher Übergangsmetalle und der Lanthanoide zunächst vielversprechend erschienen. Allerdings seien gemäß den von ihnen beschriebenen Syntheseprotokollen die Metallocen-Komplexe in den meisten Fällen nur als Isomerengemische erhältlich. Mithin sei der synthetische Wert von Dihydroazulenyl-Liganden für die metallorganische Chemie bzw. davon abgeleiteter Mono- und Bisdihydroazulenyl-Komplexe deutlich gemindert.

An den von der Gruppe um Edelmann dargestellten Komplexverbindungen ist zum einen nachteilig, dass es sich in vielen Fällen um gemischte Zirkonocene handelt, nämlich um Zirkonocen-Dichloride. Aufgrund des Vorhandenseins von Chlorid-Liganden können die Möglichkeiten der weiteren Verwendung dieser Metallkomplexe eingeschränkt sein. Zum anderen werden die von Edelmann und Mitarbeitern beschriebenen Komplexe nur in geringen Ausbeuten von 19 % bis 48 % erhalten. Weiterhin unvorteilhaft ist, dass die Mehrzahl der Metallkomplexe - insbesondere die unter Verwendung von Guajazulen hergestellten - relativ hohe Schmelzpunkte aufweist, nämlich oberhalb von 150 °C, zum Teil sogar oberhalb von 200 °C. Dies ist mit Blick auf eine mögliche Verwendung der auf Guajazulen basierenden Komplexe, z. B. als Präkursoren in einem Gasphasenabscheidungsprozess, insbesondere einem Niedrigtemperatur-Gasphasenabscheidungsverfahren, von Nachteil.

Insgesamt sind die literaturbekannten Synthesewege für die Herstellung von Metallkomplexen mit Organo-dihydroazulenyl-Liganden unter technologischen, ökologischen und (atom-)ökonomischen Gesichtspunkten als unbefriedigend einzustufen. Dies gilt konsequenterweise auch für die mittels der vorbekannten Verfahren herstellbaren Komplexverbindungen. Zudem wurden bisher keine Metallkomplexe der für eine Vielzahl von Anwendungen relevanten Edelmetalle, beispielsweise von Platin, zur Verfügung gestellt, welche wenigstens einen Organo-dihydroazulenyl-Liganden aufweisen.

Der Erfindung liegt daher die Aufgabe zugrunde, diese und weitere Nachteile des Standes der Technik zu überwinden und ein Verfahren zur Herstellung von Komplexen der Edelmetalle, insbesondere von Platin, zur Verfügung zu stellen, welche wenigstens einen Organo-dihydroazulenyl-Liganden aufweisen. Mittels dieses Verfahrens sollen Metallkomplexe des vorgenannten Typs einfach, reproduzierbar und vergleichsweise kostengünstig in hoher Reinheit und guter Ausbeute herstellbar sein. Das Verfahren soll sich zudem dadurch auszeichnen, dass es - mit vergleichbarer Ausbeute und Reinheit der Zielverbindungen - auch im industriellen Maßstab durchgeführt werden kann. Außerdem sollen Komplexe der Edelmetalle, insbesondere von Platin, welche wenigstens einen Organo-dihydroazulenyl-Liganden aufweisen, zur Verfügung gestellt werden. Gegenstand der vorliegenden Erfindung ist ferner die Verwendung solcher Komplexe. Weiterhin sollen neue Alkalimetall-organo-dihydroazulenide bereitgestellt werden, welche zur Herstellung von Metallkomplexen, insbesondere des vorgenannten Typs, verwendbar sind.

Die Hauptmerkmale der Erfindung ergeben sich aus den Patentansprüchen.

Gelöst wird die Aufgabe durch eine Verbindung gemäß der allgemeinen Formel 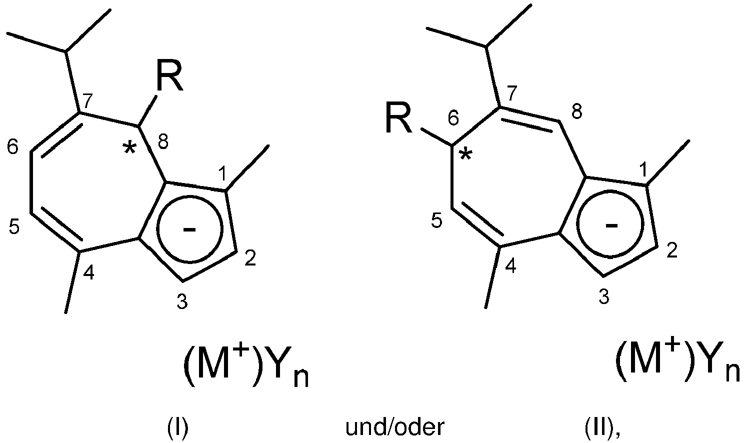 wobei
- M⁺ ein Alkalimetallkation ist,
- R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
- Y ein Neutralligand ist, welcher über wenigstens ein Donoratom an M⁺ gebunden oder koordiniert ist, wobei H₂O ausgenommen ist,
   und
- *n* = 0, 1, 2, 3 oder 4 ist,
wobei
die Verbindungen Lithium-7-*iso*-propyl-1,4,8-trimethyl-dihydroazulenid und Lithium-7-*iso*-propyl-1,4-dimethyl-8-phenyl-dihydroazulenid und deren THF-Addukte und DME-Addukte ausgenommen sind.

Bei 7-*iso*-Propyl-1,4-dimethylazulen (= Guajazulen, im Folgenden auch abgekürzt mit Gua) handelt es sich um einen Naturstoff, der in Kamillenöl und anderen ätherischen Ölen enthalten und daher in großen Mengen kostengünstig zugänglich ist. Synthetisch kann es aus dem Guajol des Guajakholzöls (Guajakharz) hergestellt werden. Guajazulen ist eine intensiv blaue Substanz mit entzündungshemmender Wirkung.

Mit THF ist Tetrahydrofuran gemeint, DME ist die Abkürzung für 1,2-Dimethoxyethan.

Im Falle der Verbindung gemäß Formel I stellt das Kohlenstoffatom C8 ein Stereozentrum dar, im Falle der Verbindung gemäß Formel II stellt das Kohlenstoffatom C6 ein Stereozentrum dar. Die Stereozentren sind in den beiden oben gezeigten Strukturformeln sowie in einigen ausgewählten nachfolgend dargestellten Strukturformeln mit einem Sternchen (*) gekennzeichnet.

Die hier beanspruchten Verbindungen werden vorliegend als Alkalimetalldihydroguajazulenide, Alkalimetall-R-dihydroguajazulenide oder Alkalimetall-organodihydroguajazulenide bezeichnet, es sei denn, dass ein bestimmtes Regioisomer gemeint ist, also ein Alkalimetall-8-R-dihydroguajazulenid (Formel I) oder ein Alkalimetall-6-R-dihydroguajazulenid (Formel II). Die beanspruchten Alkalimetalldihydroguajazulenide können isomerenrein oder als Mischung der beiden Regioisomere gemäß Formel I und II vorliegen.

Die Verbindungen gemäß den allgemeinen Formeln I und II weisen jeweils ein Organo-dihydroguajazulenyl-Anion bzw. R-Dihydroguajazulenyl-Anion (GuaR)¹⁻ auf, welches in 8-Position oder in 6-Position des Guajazulengerüsts zusätzlich zu einem H-Atom einen Organylrest R trägt. Bei dem R-Dihydroguajazulenyl-Anion (GuaR)¹⁻ kann es sich also um ein 7-*iso*-Propyl-1,4-dimethyl-8-R-dihydroazulenyl-Anion bzw. 8-R-Dihydroguajazulenyl-Anion (Gua-8-R)¹⁻ gemäß Formel I oder um ein 7-*iso*Propyl-1,4-dimethyl-6-R-dihydroazulenyl-Anion bzw. 6-R-Dihydroguajazulenyl-Anion (Gua-6-R)¹⁻ gemäß Formel II handeln. Anders ausgedrückt: In C8-Position oder in C6-Position des Guajazulengerüsts liegt eine RCH-Gruppe vor. Infolge der Addition eines Organyl-Anions (R)¹⁻ wird die Aromatizität auf den Fünfring beschränkt, wobei in der Regel die für Azulen und dessen Derivate typische blaue Farbe verloren geht. Das Organo-dihydroguajazulenyl-Anion (GuaR)¹⁻ stellt ein Derivat des Cyclopentadienyl-Anions bzw. ein Cyclopentadienyl-artiges Monoanion dar.

Bei den beiden ausgenommenen Verbindungen handelt es sich jeweils um ein Alkalimetall-dihydroguajazulenid gemäß Formel I, wobei M⁺ jeweils Li⁺ ist und R = Methyl oder Phenyl. Diese Verbindungen, deren THF-Solvate und das DME-Addukt von Lithium-7-*iso*-propyl-1,4,8-trimethyl-dihydroazulenid wurden von Edelmann und Mitarbeitern beschrieben (vgl. J. Richter, P. Liebing, F. T. Edelmann Inorg. Chim. Acta 2018, 475, 18 - 27) und sind - genau wie die DME-Solvate der beiden ausgenommenen Verbindungen - nicht Gegenstand der vorliegenden Erfindung. Mischungen der beiden vorgenannten Verbindungen oder der THF-Solvate dieser Verbindungen oder des DME-Addukts von Lithium-7-*iso*-propyl-1,4,8-trimethyl-dihydroazulenid mit dem jeweiligen Regioisomer gemäß Formel II wurden von den Autoren nicht beschrieben.

Die beanspruchten Verbindungen können adduktfrei bzw. solvensfrei vorliegen, nämlich wenn *n* = 0 ist, oder als Solvens-Addukte bzw. Solvate mit einem (*n* = 1), zwei (*n* = 2), drei (*n* = 3) oder vier (*n* = 4) Neutralliganden Y. In Lösung erfolgt regelmäßig eine Solvatation der Alkalimetallionen M⁺, insbesondere wenn das verwendete Lösungsmittel ein Alkoxyalkan ist oder wenigstens ein Alkoxyalkan umfasst. Dann liegen in Lösung insbesondere solvatisierte Lithium-Ionen vor, wobei Y = ein Alkoxyalkan und *n* = 4 ist, z. B. Li(OC₂H₅)₄ oder Li(thf)₄. Zudem können isolierte Verbindungen gemäß den Formeln I und II als Solvens-Addukte vorliegen, wobei das jeweilige Alkalimetallkation M⁺ komplexiert ist, z. B. durch einen Kronenether.

Mit dem Begriff "Alkoxyalkan" ist gemäß der vorliegenden Erfindung jeder Sauerstoff enthaltende Ether gemeint, beispielsweise auch Glycol-dialkylether und Kronenether. Als Glycol-dialkylether werden auch terminal dialkylierte Mono-, Di- oder Trialkylenglycoldialkylether verstanden. Kronenether sind makrocyclische Polyether, deren Ring aus mehreren Ethyloxy-Einheiten (-CH₂-CH₂-O-) besteht. Sie besitzen die Fähigkeit zur Komplexierung von Kationen unter Bildung von Coronaten. Bei Übereinstimmung von Innendurchmesser eines Kronenethers und lonenradius eines Metallkations werden äußerst stabile Komplexe gebildet. Beispielsweise ist der Kronenether [18]-Krone-6 ein sehr guter Ligand für ein Kalium-Ion, während sich z. B. der Kronenether [15]-Krone-5 besonders gut für die Komplexierung eines Natrium-Ions eignet. Ein Lithium-Ion kann z. B. sehr gut von dem Kronenether [12]-Krone-4 komplexiert werden. Über den Austausch der Sauerstoff-Atome gegen andere Heteroatome, z. B. Stickstoff, Phosphor oder Schwefel, sind Aza-, Phospha- oder Thia-Derivate der Kronenether zugänglich.

Ob die hier beanspruchten Verbindungen gemäß der allgemeinen Formel I oder II als Solvens-Addukte, insbesondere Alkoxyalkan-Addukte, vorliegen, hängt von verschiedenen Faktoren ab, z. B. von dem für die Darstellung von Verbindungen gemäß der allgemeinen Formel I oder II verwendeten Lösungsmittel sowie der Art des Alkalimetallkations M⁺. Wurde beispielsweise im Rahmen der Synthese, einschließlich der Aufreinigung, ein Solvens verwendet, welches ein oder mehrere Alkoxyalkane umfasste oder daraus bestand, oder zum Kristallisieren ein Alkoxyalkan verwendet, beispielsweise ein Kronenether, und wenigstens ein Lithium-Kationen aufweisendes Alkylierungs- oder Arylierungsreagenz eingesetzt, so wird aller Wahrscheinlichkeit nach ein Solvens-Addukt Li(alkoxyalkan)*ₙ*(GuaR) vorliegen, wobei *n* = 1 oder 2 ist. Sofern ein solches Solvens-Addukt abschließend mit einem nicht-etherischen Lösungsmittel, wie z. B. Pentan oder Hexan, gewaschen wurde, liegt üblicherweise eine solvensfreie Verbindung gemäß der allgemeinen Formel Li(GuaR) vor.

Als Rest R können auch primäre, sekundäre, tertiäre Alkyl-, Alkenyl- und Alkinylreste mit 2, 3, 4, 5, 6, 7, 8 oder 9 Kohlenstoffatomen vorgesehen sein sowie cyclische Alkylreste mit 4, 5, 6, 7, 8 oder 9 Kohlenstoffatomen.

Besonders vorteilhaft ist, dass die hier beanspruchten Verbindungen umfassend Cyclopentadienyl-artige Monoanionen (GuaR)¹⁻ bei Raumtemperatur eine gute bis sehr gute Langzeitstabilität aufweisen. Während einer Lagerung für mehrere Monate bei Raumtemperatur werden weder Zersetzungsreaktionen noch eine Oligomerisierung oder Polymerisierung beobachtet. Dies ist besonders mit Blick auf die weitere Verwendung der Verbindungen gemäß den allgemeinen Formeln I und II, insbesondere als Liganden-Präkursoren zur Herstellung von *Sandwich-* und Halb*sandwich*-Komplexen, vorteilhaft. Weiterhin ist die Herstellung von Alkalimetall-dihydroguajazuleniden gemäß den allgemeinen Formeln I und II, umfassend Cyclopentadienyl-artige Monoanionen (GuaR)¹⁻, im Vergleich zur Bereitstellung von LiCp weniger arbeits- und zeitaufwändig. Insbesondere können die hier beanspruchten Verbindungen, welche Cyclopentadienyl-artige Liganden umfassen, unter Verwendung von kostengünstigen nachwachsenden Rohstoffen hergestellt werden. Guajazulen ist partialsynthetisch zugänglich, nämlich ausgehend von dem Naturstoff Guajol und anderen Azulenbildnern durch einfache Dehydratisierung und Dehydrierung (T. Shono, N. Kise, T. Fujimoto, N. Tominaga, H. Morita, J. Org. Chem. 1992, 57, 26, 7175 - 7187; CH 314 487 A (B. Joos) 29.01.1953).

Die hier beschriebenen Verbindungen gemäß den Formeln I und II, umfassend Cyclopentadienyl-artige Monoanionen (GuaR)¹⁻, sind einfach und reproduzierbar und - je nach Wahl der Edukte - nachhaltig und vergleichsweise kostengünstig erhältlich. Zudem sind eine hohe Reinheit von 97 %, vorteilhaft von mehr als 97 %, insbesondere von mehr als 98 % oder 99 %, und gute Ausbeuten von üblicherweise ≥ 60% sowie gute Raum-Zeit-Ausbeuten realisierbar. Mithin eignen sie sich als Liganden-Präkursoren zur Herstellung von *Sandwich-* und Halb*sandwich-*Komplexen, auch im industriellen Maßstab. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, z. B. von Lösungsmitteln, mittels gaschromatographischer Verfahren (GC), ggf. mit Massenspektrometrie-Kopplung (GC-MS), bestimmt werden kann.

Mit Reinheit ist gemäß der vorliegenden Erfindung die Abwesenheit unerwünschter Verunreinigungen, insbesondere durch Edukte, Nebenprodukte, Luftsauerstoff, Wasser, sauerstoffhaltige Verbindungen, Halbmetalle, Metalle und Lösungsmittel, gemeint. Die Reinheit kann mit Blick auf die spätere Verwendung der Verbindungen gemäß Formel I und Formel II, insbesondere als Liganden-Präkursoren zur Herstellung von *Sandwich-* und Halb*sandwich*-Komplexen, von Bedeutung sein.

Unter "Raum-Zeit-Ausbeute" wird hier eine innerhalb eines Reaktionsbehältnisses bzw. Reaktionsgefäßes pro Raum und Zeit gebildete Produktmenge verstanden, also eine pro Volumen und Zeit erhaltene Masse eines Produktes. Als Einheit wird beispielsweise kg/L*h gewählt.

Die Begriffe Reaktionsbehältnis und Reaktionsgefäß werden im Zusammenhang mit der vorliegenden Erfindung synonym verwendet und sind nicht auf ein Volumen, eine Materialbeschaffenheit, eine Ausstattung oder eine Form beschränkt. Geeignete Reaktionsgefäße sind z. B. Glaskolben, emaillierte Reaktoren, Rührkesselreaktoren, Druckbehälter, Röhrenreaktoren, Mikroreaktoren und Durchflussreaktoren.

Eine Ausführungsform der hier beanspruchten Verbindungen sieht vor, dass R ausgewählt ist aus der Gruppe bestehend aus
- primären, sekundären, tertiären Alkyl-, Alkenyl- und Alkinylresten mit 1 bis 6 Kohlenstoffatomen,
- cyclischen Alkylresten mit 3 bis 6 Kohlenstoffatomen,
- einem Benzylrest,
- einkernigen Arylresten und mehrkernigen Arylresten
   und
- einkernigen Heteroarylresten und mehrkernigen Heteroarylresten.

Als Rest R können also auch primäre, sekundäre, tertiäre Alkyl-, Alkenyl- und Alkinylreste mit 2, 3, 4 oder 5 Kohlenstoffatomen vorgesehen sein sowie cyclische Alkylreste mit 4 oder 5 Kohlenstoffatomen.

In einer anderen Ausführungsvariante der Verbindungen gemäß der allgemeinen Formel I oder II ist R ausgewählt aus der Gruppe bestehend aus Me, Et, *n*-Pr, *i*-Pr, *n-*Bu, *i*-Bu, *s*-Bu, *t*-Bu, *n*-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, Cyclopentyl, Cyclohexyl, Phenyl, Tolyl, Benzyl und Cumyl, und deren Isomeren. Gemäß einer noch anderen vorteilhaften Ausführungsform ist R ausgewählt aus der Gruppe bestehend aus Me, Et, *n*-Pr, *i*-Pr, *n*-Bu, *i*-Bu, *s*-Bu, *t*-Bu, Cyclohexyl, Phenyl, Tolyl, Benzyl und Cumyl, und deren Isomeren. Besonders vorteilhaft ist R ausgewählt aus der Gruppe bestehend aus Me, Et, *n*-Pr, *i*-Pr, *n*-Bu, *i*-Bu, *s*-Bu, *t*-Bu, insbesondere ist R = Me.

Gemäß einer weiteren Ausführungsform der hier beanspruchten Verbindungen liegt
i. eine isomerenreine Verbindung gemäß der allgemeinen Formel I oder der allgemeinen Formel II vor
   oder
ii. ein Isomerengemisch vor, welches ein erstes Regioisomer gemäß Formel I und ein zweites Regioisomer gemäß Formel II enthält, und insbesondere aus dem ersten Regioisomer gemäß Formel I und dem zweiten Regioisomer gemäß Formel II besteht.

Während die Kohlenstoffatome C4, C6 und C8 von Bicyclo[5.3.0]decapentaen (= Azulen) bekanntermaßen eine vergleichbare Nucleophilie aufweisen, besitzen die Kohlenstoffatome C6 und C8 von Guajazulen eine unterschiedliche Nucleophilie, nämlich aufgrund der asymmetrischen Substitution in C1-, C4- und C7-Position. Daher können die hier beanspruchten Verbindungen gemäß Formel I und II - in Abhängigkeit vom jeweils nucleophil angreifenden Organyl-Anion (R)¹⁻ - nicht nur als Isomerengemische vorliegen, welche die beiden Regioisomere gemäß Formel I und II enthalten oder daraus bestehen, sondern auch als isomerenreine Verbindungen gemäß einer der Formeln I oder II.

"Isomerenrein" bedeutet im Rahmen der vorliegenden Erfindung, dass das gewünschte Produkt isomerenrein anfällt bzw. angefallen ist oder das gewünschte Isomer nach Aufreinigung mit einem Anteil von ≥ 90 %, bevorzugt von ≥ 95 %, besonders bevorzugt von ≥ 99 % vorliegt. Dabei ist die Isomerenreinheit beispielsweise mittels Kernresonanzspektroskopie bestimmt, insbesondere mittels ¹H-NMR-Spektroskopie.

Im Falle einer isomerenreinen Verbindung liegt entweder ein Alkalimetall-8-R-dihydroguajazulenid (Formel I) oder ein Alkalimetall-6-R-dihydroguajazulenid (Formel II) vor. Mit anderen Worten: Es liegt nur ein Regioisomer vor, welches entweder in C8-Position (Formel I) oder in C6-Position (Formel II) des Guajazulengerüsts eine RCH-Gruppe und somit ein Chiralitätszentrum aufweist.

Liegt ein Isomerengemisch vor, welches das erste Regioisomer gemäß Formel I und das zweite Regioisomer gemäß Formel II enthält, und insbesondere aus dem ersten Regioisomer gemäß Formel I und dem zweiten Regioisomer gemäß Formel II besteht, so liegt ein Gemisch enthaltend ein Alkalimetall-8-R-dihydroguajazulenid (Formel I) und ein Alkalimetall-6-R-dihydroguajazulenid (Formel II) vor, insbesondere bestehend aus einem Alkalimetall-8-R-dihydroguajazulenid (Formel I) und einem Alkalimetall-6-R-dihydroguajazulenid (Formel II). Dabei ist R in Formel I identisch mit R in Formel II. Mit anderen Worten: Das erste Regioisomer weist eine RCH-Gruppe in C8-Position (Formel I) des Guajazulengerüsts auf, und das zweite Regioisomer weist eine RCH-Gruppe in C6-Position (Formel II) des Guajazulengerüsts auf.

Eine noch andere Variante der hier beanspruchten Verbindungen sieht vor, dass ein Isomerenverhältnis erstes Regioisomer : zweites Regioisomer ≥ 80 : 20 und < 90 : 10 beträgt, vorteilhaft zwischen 81 : 19 und 89 : 11, insbesondere zwischen 82 : 18 und 88 : 12, beispielsweise 83 : 17 oder 84 : 16 oder 85 : 15 oder 86 : 14 oder 87 : 13. Dabei ist das Isomerenverhältnis beispielsweise mittels Kernresonanzspektroskopie bestimmt, insbesondere mittels ¹H-NMR-Spektroskopie.

Gemäß einer weiteren Ausführungsform der hier beanspruchten Verbindungen gemäß Formel I und II ist das Alkalimetallkation M⁺ ausgewählt aus der Gruppe bestehend aus Li+, Na⁺ und K+.

Nach einer anderen vorteilhaften Ausführungsvariante liegt ein Isomerengemisch bestehend aus einem ersten Regioisomer gemäß Formel I.1 und einem zweiten Regioisomer gemäß Formel II.1 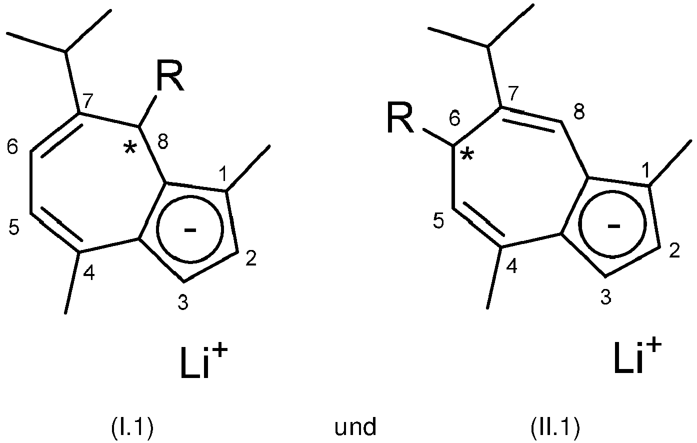 vor, wobei M⁺ = Li⁺ ist. R ist insbesondere eine Methylgruppe (Me). In einer alternativen oder ergänzenden Ausführungsform beträgt das Isomerenverhältnis erstes Regioisomer : zweites Regioisomer ≥ 80 : 20 und < 90 : 10, vorteilhaft zwischen 81 : 19 und 89 : 11, insbesondere zwischen 82 : 18 und 88 : 12, beispielsweise 83 : 17 oder 84 : 16 oder 85 : 15 oder 86 : 14 oder 87 : 13. In einer alternativen oder ergänzenden Ausführungsform ist eine Anzahl *n* des Neutralliganden Y über wenigstens ein Donoratom an M⁺ gebunden oder koordiniert, wobei H₂O ausgenommen ist und *n* = 1, 2, 3 oder 4 ist. Dabei kann Y beispielsweise ein Alkoxyalkan und *n* = 4 sein und das Alkalimetallkation, insbesondere das Lithium-Kation, als Solvat, z. B. Li(OC₂H₅)₄ oder Li(thf)₄, oder Solvens-Addukt, z. B. in Li(OC₂H₅)₂(GuaR), vorliegen.

Eine weitere Ausführungsform der hier beanspruchten Verbindungen gemäß Formel I und Formel II sieht vor, dass der Neutralligand Y ein aprotisch-polares Lösungsmittel ist. Vorteilhaft ist das aprotisch-polare Lösungsmittel ausgewählt aus der Gruppe bestehend aus Alkoxyalkanen, Thioethern und tertiären Aminen, insbesondere aus der Gruppe bestehend aus Alkoxyalkanen und Thioethern. Dann kann die jeweilige Verbindung gemäß Formel I oder Formel II in Abhängigkeit von der Wahl des Neutralliganden Y, z. B. im Falle von Diethylether, THF, Thiophen oder Triethylamin, kristallin vorliegen.

Der Begriff "Alkoxyalkan" ist bereits weiter oben definiert. Der Begriff "Thioether" umfasst sowohl nicht-cyclische als auch cyclische Thioether.

In einer anderen Variante der hier beanspruchten Verbindungen ist der Neutralligand Y ein Kronenether, welcher ausgewählt ist aus der Gruppe bestehend aus makrocyclischen Polyethern und deren Aza-, Phospha- und Thia-Derivaten, wobei ein Innendurchmesser des Kronenethers und ein lonenradius des jeweiligen Alkalimetallkations M⁺ miteinander korrespondieren. Dann kann die jeweilige Verbindung gemäß Formel I oder Formel II kristallin vorliegen.

Gemäß einer weiteren Ausführungsform der hier beanspruchten Verbindungen ist als Neutralligand Y ein Glycol-dialkylether vorgesehen, ausgewählt aus der Gruppe bestehend aus einem Monoethylenglycoldialkylether, einem Diethylenglycoldialkylether, einem Triethylengylcoldialkylether, einem Monopropylenglycoldialkylether, einem Dipropylenglycoldialkylether, einem Tripropylenglycoldialkylether, einem Monooxomethylendialkylether, einem Dioxomethylendialkylether und einem Trioxomethylendialkylether, deren Isomerenmischungen, und Mischungen davon.

In einer weiteren Ausführungsform der hier beanspruchten Verbindungen ist der als Neutralligand Y vorgesehene Glycol-dialkylether ausgewählt aus der Gruppe bestehend aus Ethylenglycoldimethylether CH₃-O-CH₂CH₂-O-CH₃, Ethylenglycoldiethylether CH₃CH₂-O-CH₂CH₂-O-CH₂CH₃, Ethylenglycoldi-*n-*propylether CH₃CH₂CH₂-O-CH₂CH₂-O-CH₂CH₂CH₃, Ethylenglycoldi-*iso*-propylether (CH₃)₂CH-O-CH₂CH₂-O-CH(CH₃)₂, Ethylenglycoldi-*n*-butylether CH₃CH₂CH₂CH₂-O-CH₂CH₂-O-CH₂CH₂CH₂CH₃, Ethylenglycoldi-*n*-pentylether CH₃CH₂CH₂CH₂CH₂-O-CH₂CH₂-O-CH₂CH₂CH₂CH₂CH₃, Ethylenglycoldi-*n*-hexylether CH₃CH₂CH₂CH₂CH₂CH₂-O-CH₂CH₂-O-CH₂CH₂CH₂CH₂CH₂CH₃, Ethylenglycoldiphenylether C₆H₅-O-CH₂CH₂-O-C₆H₅, Ethylenglycoldibenzylether C₆H₅CH₂-O-CH₂CH₂-O-CH₂C₆H₅, Diethylenglycoldimethylether CH₃-O-CH₂CH₂-O-CH₂CH₂-O-CH₃, Diethylenglycoldiethylether CH₃CH₂-O-CH₂CH₂-O-CH₂CH₂-O-CH₂CH₃, Diethylenglycoldi-*n*-propylether CH₃CH₂CH₂-O-CH₂CH₂-O-CH₂CH₂-O-CH₂CH₂CH₃, Diethylenglycoldi-*iso*-propylether (CH₃)₂CH-O-CH₂CH₂-O-CH₂CH₂-O-CH(CH₃)₂, Diethylenglycoldi-*n*-butylether CH₃CH₂CH₂CH₂-O-CH₂CH₂-O-CH₂CH₂-O-CH₂CH₂CH₂CH₃, Diethylenglycoldi-*n*-pentylether CH₃CH₂CH₂CH₂CH₂-O-CH₂CH₂-O-CH₂CH₂-O-CH₂CH₂CH₂CH₂CH₃, Diethylenglycoldi-*n*-hexylether CH₃CH₂CH₂CH₂CH₂CH₂-O-CH₂CH₂-O-CH₂CH₂-O-CH₂CH₂CH₂CH₂CH₂CH₃, Diethylenglycoldiphenylether C₆H₅-O-CH₂CH₂-O-CH₂CH₂-O-C₆H₅, Diethylenglycoldibenzylether C₆H₅CH₂-O-CH₂CH₂-O-CH₂CH₂-O-CH₂C₆H₅, Propylenglycoldimethylether CH₃-O-CH₂CH₂CH₂-O-CH₃, Propylenglycoldiethylether CH₃CH₂-O-CH₂CH₂CH₂-O-CH₂CH₃, Propylenglycoldi-*n*-propylether CH₃CH₂CH₂-O-CH₂CH₂CH₂-O-CH₂CH₂CH₃, Propylenglycoldi-*n*-butylether CH₃CH₂CH₂CH₂-O-CH₂CH₂CH₂-O-CH₂CH₂CH₂CH₃, Propylenglycoldi-*n*-pentylether CH₃CH₂CH₂CH₂CH₂-O-CH₂CH₂CH₂-O-CH₂CH₂CH₂CH₂CH₃, Propylenglycoldi-*n-*hexylether CH₃CH₂CH₂CH₂CH₂CH₂-O-CH₂CH₂CH₂-O-CH₂CH₂CH₂CH₂CH₂CH₃, Propylenglycoldiphenylether C₆H₅-O-CH₂CH₂CH₂-O-C₆H₅, Propylenglycoldibenzylether C₆H₅CH₂-O-CH₂CH₂CH₂-O-CH₂C₆H₅, *iso*-Propylenglycoldimethylether CH₃-O-CH₂-CH(CH₃)-O-CH₃, *iso*-Propylenglycoldiethylether CH₃CH₂-O-CH₂-CH(CH₃)-O-CH₂CH₃, *iso*-Propylenglycoldi-*n*-propylether CH₃CH₂CH₂-O-CH₂-CH(CH₃)-O-CH₂CH₂CH₃, *iso*-Propylenglycoldi-*iso*-propylether (CH₃)₂CH-O-CH₂-CH(CH₃)-O-CH(CH₃)₂, *iso*-Propylenglycoldi-*n*-butylether CH₃CH₂CH₂CH₂-O-CH₂-CH(CH₃)-O-CH₂CH₂CH₂CH₃, *iso*-Propylenglycoldi-*n*-pentylether CH₃CH₂CH₂CH₂CH₂-O-CH₂-CH(CH₃)-O-CH₂CH₂CH₂CH₂CH₃, *iso*-Propylenglycoldi-*n*-hexylether CH₃CH₂CH₂CH₂CH₂CH₂-O-CH₂-CH(CH₃)-O-CH₂CH₂CH₂CH₂CH₂CH₃, *iso*-Propylenglycoldiphenylether C₆H₅-O-CH₂-CH(CH₃)-O-C₆H₅, *iso-*Propylenglycoldibenzylether C₆H₅CH₂-O-CH₂-CH(CH₃)-O-CH₂C₆H₅, Dipropylenglycoldimethylether CH₃OCH₂CH₂CH₂OCH₂CH₂CH₂OCH₃, Di-*iso-*propylenglycoldi-*n*-propylether CH₃CH₂CH₂-O-CH₂CH(CH₃)-OCH₂CH(CH₃)-O-CH₂CH₂CH₃, Tripropylenglycoldimethylether CH₃OCH₂CH₂CH₂OCH₂CH₂CH₂OCH₂CH₂CH₂OCH₃, Dipropylenglycoldibutylether CH₃CH₂CH₂CH₂OCH₂CH₂CH₂OCH₂CH₂CH₂OCH₂CH₂CH₂CH₃, Tripropylenglycoldibutylether CH₃CH₂CH₂CH₂OCH₂CH₂CH₂OCH₂CH₂CH₂OCH₂CH₂CH₂OCH₂CH₂CH₂CH₃, sowie deren Mischungen. Die angegebenen Glycolether können auch als Isomerengemische vorliegen.

Eine weitere Ausführungsform der hier beanspruchten Verbindungen sieht vor, dass der Neutralligand Y ein Ether ist. Beispielsweise kann der Ether ein nicht-cyclischer oder ein cyclischer Ether sein ausgewählt aus der Gruppe bestehend aus Dialkylethern, Cyclopentylmethylether, Tetrahydrofuran, 2-Methyltetrahydrofuran, 3-Methyltetrahydrofuran, Tetrahydropyran, 1,4-Dioxan, 1,2-Dimethoxyethan, und deren Isomeren, und Mischungen davon, insbesondere aus der Gruppe bestehend aus Diethylether, Methyl-*tert*-butylether, Di-*n*-propylether, Di-*iso-*propylether, Di-*n-*butylether, Di-*iso*-butylether, Di-*tert*-butylether, Tetrahydrofuran, 2-Methyltetrahydrofuran, 3-Methyltetrahydrofuran, Tetrahydropyran, 1,4-Dioxan, 1,2-Dimethoxyethan, und deren Isomeren, und Mischungen davon.

Die Aufgabe wird außerdem gelöst durch die Verwendung einer Verbindung gemäß der allgemeinen Formel 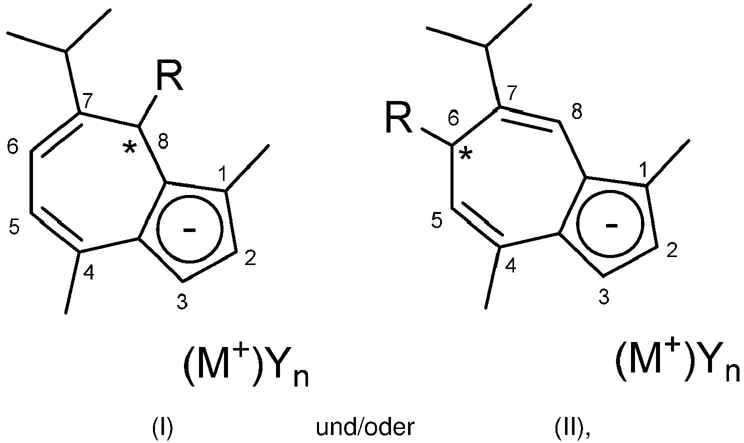 wobei
- M⁺ ein Alkalimetallkation ist,
- R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
- Y ein Neutralligand ist, welcher über wenigstens ein Donoratom an M⁺ gebunden oder koordiniert ist, wobei H₂O ausgenommen ist,
   und
- *n* = 0, 1, 2, 3 oder 4 ist,
oder gemäß einer der weiter oben beschriebenen Ausführungsformen,
zur Herstellung eines Platin(IV)-Komplexes gemäß der allgemeinen Formel 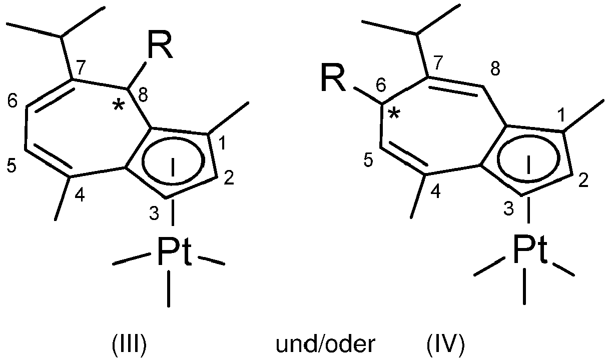 wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten.

Bei der vorgenannten Verwendung einer Verbindung gemäß der allgemeinen Formel I und/oder der allgemeinen Formel II zur Herstellung eines Platin(IV)-Komplexes gemäß der allgemeinen Formel III und/oder IV handelt es sich um ein Verfahren zur Herstellung eines Platin(IV)-Komplexes gemäß der allgemeinen Formel 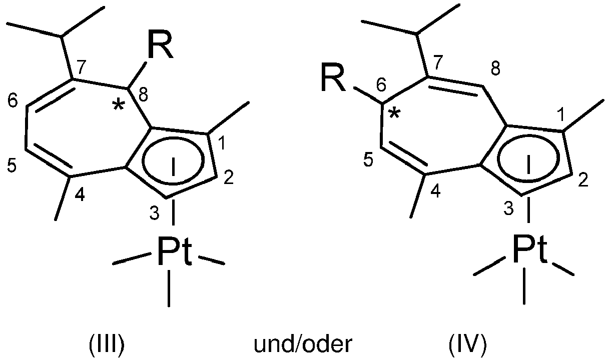 wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
unter Verwendung einer Verbindung gemäß der allgemeinen Formel 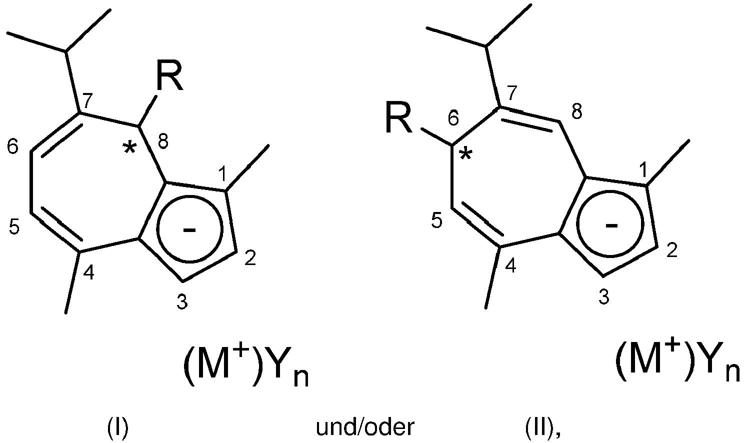 wobei
- M⁺ ein Alkalimetallkation ist,
- R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
- Y ein Neutralligand ist, welcher über wenigstens ein Donoratom an M⁺ gebunden oder koordiniert ist, wobei H₂O ausgenommen ist,
   und
- *n* = 0, 1, 2, 3 oder 4 ist,
oder gemäß einer der weiter oben beschriebenen Ausführungsformen.

Dabei umfasst das Verfahren die folgenden Schritte:
A. Zurverfügungstellung
   - der Verbindung gemäß der allgemeinen Formel I und/oder der allgemeinen Formel II
      und
   - einer Platinvorstufe,
B. Synthese des Platin(IV)-Komplexes gemäß der allgemeinen Formel III und/oder der allgemeinen Formel IV
   unter Verwendung der Verbindung gemäß der allgemeinen Formel I und/oder der allgemeinen Formel II als Edukt
   in einem Lösungsmittel S_{P},
C. Optionale Isolierung des in Schritt B. synthetisierten Platin(IV)-Komplexes gemäß der allgemeinen Formel III und/oder der allgemeinen Formel IV.

Dabei umfassen die allgemeinen Formeln III und IV jeweils sowohl die Monomere als auch etwaige Oligomere, insbesondere Dimere, und Lösungsmitteladdukte. Zudem liegen die mittels des beanspruchten Verfahrens herstellbaren Verbindungen gemäß den allgemeinen Formeln III und IV insbesondere jeweils als ein Diastereomerengemisch vor. Mit anderen Worten: Im Falle des Platin(IV)-Komplexes gemäß Formel III stellt das Kohlenstoffatom C8 ein Stereozentrum dar, im Falle des Platin(IV)-Komplexes gemäß Formel IV stellt das Kohlenstoffatom C6 ein Stereozentrum dar.

Alternativ kann - insbesondere in Abhängigkeit vom gewählten Substituenten R - ein Diastereomerengemisch erhalten werden, welches sowohl das Diastereomerengemisch der Verbindung gemäß Formel III als auch das Diastereomerengemisch der Verbindung gemäß Formel IV umfasst, insbesondere daraus besteht.

In jedem der vorgenannten Fälle liegt jedes Diastereomer als ein Enantiomerengemisch vor, wobei die Diastereomere unabhängig voneinander jeweils als ein Racemat vorliegen können.

Dem Fachmann ist bekannt, welche Platinvorstufen kommerziell erhältlich oder - ggf. auch *in situ -* darstellbar sind und welche Reaktionsbedingungen, z. B. Stöchiometrie der Edukte, Lösungsmittel, Reaktionstemperatur, Reaktionsdauer, und Arbeitsschritte, inklusive ggf. erforderlichem Lösungsmittelwechsel, Isolierung und ggf. Aufreinigung, in Schritt B. für die Synthese des Platin(IV)-Komplexes gemäß Formel III und/oder Formel IV erforderlich sind.

Die Reihenfolge, in der ein Reaktionsbehältnis mit den Edukten, d. h. dem Alkalimetall-dihydroguajazulenid gemäß Formel I und/oder Formel II und einer Platinvorstufe, beschickt wird, ist frei wählbar. Das schließt auch die Möglichkeit mit ein, die Schritte A. und B. bzw. Teilschritte davon, also sämtliche die Herstellung der jeweiligen Zielverbindung betreffenden Schritte, in einem einzigen Schritt durchzuführen, also sämtliche Edukte und Lösungsmittel zeitgleich oder nahezu zeitgleich in das Reaktionsbehältnis einzubringen.

Die Begriffe Reaktionsbehältnis und Reaktionsgefäß sind bereits weiter oben definiert.

Das hierin beschriebene Verfahren zur Herstellung heteroleptischer Platin(IV)-Komplexe gemäß den Formeln III und IV kann als diskontinuierliches Verfahren oder als kontinuierliches Verfahren durchgeführt werden.

Das jeweils verwendete Alkalimetall-dihydroguajazulenid kann isomerenrein oder als Mischung der beiden Regioisomere gemäß den Formeln I und II vorliegen, d. h. als Gemisch umfassend ein Alkalimetall-8-R-dihydroguajazulenid (Formel I) und ein Alkalimetall-6-R-dihydroguajazulenid (Formel II) oder bestehend aus einem Alkalimetall-8-R-dihydroguajazulenid (Formel I) und einem Alkalimetall-6-R-dihydroguajazulenid (Formel II). Zudem können die verwendeten Verbindungen gemäß Formel I und Formel II adduktfrei bzw. solvensfrei vorliegen, nämlich wenn *n* = 0, oder als Solvens-Addukte bzw. Solvate mit einem (*n* = 1), zwei (*n* = 2), drei (*n* = 3) oder vier (*n* = 4) Neutralliganden Y. Eine Auswahl an Neutralliganden Y ist in nicht einschränkender Weise weiter oben angegeben.

Bei dem Lösungsmittel S_{P} kann es sich auch um ein Lösungsmittelgemisch handeln.

Überraschenderweise sind mittels der hier beanspruchten Verwendung einer Verbindung gemäß der allgemeinen Formel I und/oder II bzw. mittels des hier beschriebenen Verfahrens unter Verwendung der vorgenannten Verbindungen Platin(IV)-Komplexe gemäß der allgemeinen Formel III und/oder IV, insbesondere als Diastereomerengemische, in hoher Reinheit von 97 %, vorteilhaft von mehr als 97 %, insbesondere von mehr als 98 % oder 99 %, und guter Ausbeute von üblicherweise ≥ 60 erhältlich sowie in guter Raum-Zeit-Ausbeute. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, z. B. von Lösungsmitteln, mittels gaschromatographischer Verfahren (GC), ggf. mit Massenspektrometrie-Kopplung (GC-MS), bestimmt werden kann.

Eine Definition des Begriffes "Raum-Zeit-Ausbeute" ist bereits weiter oben gegeben.

Ein mittels des hier beschriebenen Verfahrens in hoher Reinheit bzw. hochrein erhältlicher oder erhaltener Platin(IV)-Komplex weist einen Gesamtgehalt an Verunreinigungen, umfassend insbesondere Verunreinigungen durch Edukte, Nebenprodukte, Luftsauerstoff, Wasser, sauerstoffhaltige Verbindungen, Halbmetalle, Metalle, beispielsweise Platin(0), gegebenenfalls in Form von Platin(0)-Nanopartikeln und/oder Platin(0) enthaltenden Nanopartikeln, und Lösungsmittel, von unter 1000 ppm, idealerweise von unter 100 ppm auf. Die Reinheit kann mit Blick auf die spätere Verwendung der mittels dieses Verfahrens darstellbaren Platin(IV)-Komplexe von Bedeutung sein.

Überraschend ist insbesondere, dass sich stark reduzierende Organo-dihydroguajazulenyl-Liganden (GuaR)¹⁻, nämlich der 8-R-Dihydroguajazulenyl-Ligand (Gua-8-R)¹⁻ und der 6-R-Dihydroguajazulenyl-Ligand (Gua-6-R)¹⁻ für die Darstellung von Komplexen der Edelmetalle, insbesondere von Platin, beispielsweise ausgehend von Platin(IV)-Präkursorverbindungen wie Trimethylplatin(IV)-iodid, eignen. Diese Komplexe, z. B. der Halb*sandwich*-Komplex [PtMe₃(GuaMe)], sind zudem vorteilhafterweise in guter Ausbeute und hoher Reinheit erhältlich.

Die vorgenannten Tatsachen sind deswegen überraschend, weil dem Fachmann bekannt ist, dass stark reduzierende R-Dihydroguajazulenyl-Liganden (GuaR)¹⁻, wie z. B. der 8-R-Dihydroguajazulenyl-Ligand (Gua-8-R)¹⁻, insbesondere für die Komplexbildung mit frühen Übergangsmetallen, z. B. Titan und Zirkonium, prädestiniert sind. (vgl. J. Richter, P. Liebing, F. T. Edelmann Inorg. Chim. Acta 2018, 475,18 - 27).

Die hier als Edukt verwendeten Verbindungen gemäß den Formeln I und II, umfassend Cyclopentadienyl-artige Monoanionen (GuaR)¹⁻, sind einfach, reproduzierbar, nachhaltig und vergleichsweise kostengünstig erhältlich. Zudem sind eine hohe Reinheit, gute Ausbeuten sowie Raum-Zeit-Ausbeuten realisierbar. Mithin eignen sie sich auch für den Einsatz in industriellen Prozessen. Besonders vorteilhaft ist, dass die im Rahmen des hier beanspruchten Verfahrens verwendeten Verbindungen gemäß den Formeln I und II, umfassend Cyclopentadienyl-artige Monoanionen (GuaR)¹⁻, bei Raumtemperatur eine gute bis sehr gute Langzeitstabilität aufweisen. Während einer Lagerung für mehrere Monate bei Raumtemperatur werden weder Zersetzungsreaktionen noch eine Oligomerisierung oder Polymerisierung beobachtet. Weiterhin ist die Herstellung der verwendeten Alkalimetall-dihydroguajazuleniden gemäß den allgemeinen Formeln I und II im Vergleich zur Bereitstellung von LiCp weniger arbeits- und zeitaufwändig. Insbesondere können die hier als Edukte eingesetzten Verbindungen, welche Cyclopentadienyl-artige Liganden umfassen, unter Verwendung von kostengünstigen nachwachsenden Rohstoffen hergestellt werden. Denn Guajazueln ist partialsynthetisch zugänglich, und zwar ausgehend von dem Naturstoff Guajol und anderen Azulenbildnern durch einfache Dehydratisierung und Dehydrierung (T. Shono, N. Kise, T. Fujimoto, N. Tominaga, H. Morita, J. Org. Chem. 1992, 57, 26, 7175 - 7187; CH 314 487 A (B. Joos) 29.01.1953).

Nach alledem sind mittels des hier beanspruchten Verfahrens Platin(IV)-Komplexe erhältlich, welche eine kostengünstige und nachhaltige Alternative zu vorbekannten Katalysatoren bzw. Präkatalysatoren wie [PtMe₃(Cp)] und [PtMe₃(MeCp)] darstellen. So wird das zur Herstellung von Verbindungen gemäß den Formeln III und IV, z. B. [PtMe₃(GuaMe)], erforderliche Guajazulen unter Verwendung nachwachsender Rohstoffe anstelle von Erdöl synthetisiert. Mithin sind das hier beschriebene Verfahren und die damit darstellbaren Verbindungen sowohl aus ökonomischer als auch aus ökologischer Sicht besonders vorteilhaft gegenüber den vorgenannten Cyclopentadienyl-Anionen enthaltenden Platin(IV)-Verbindungen.

Gemäß einer Ausführungsform der hier beanspruchten Verwendung bzw. des hier beanspruchten Verfahrens umfasst das Lösungsmittel S_{P} wenigstens ein Lösungsmittel, welches ausgewählt ist aus der Gruppe bestehend aus aprotisch-polaren Lösungsmitteln, aliphatischen Kohlenwasserstoffen, aromatischen Kohlenwasserstoffen, siliciumorganischen Verbindungen, und Mischungen davon. Vorteilhaft ist das Lösungsmittel S_{P} ausgewählt aus der Gruppe bestehend aus aprotisch-polaren Lösungsmitteln, aliphatischen Kohlenwasserstoffen, insbesondere mit 1 bis 30 Kohlenstoffatomen, aromatischen Kohlenwasserstoffen, siliciumorganischen Verbindungen, und Mischungen davon.

Das aprotisch-polare Lösungsmittel ist beispielsweise ein Ether oder umfasst wenigstens einen Ether. Der Ether kann ausgewählt sein aus der Gruppe bestehend aus Tetrahydrofuran, Methyltetrahydrofuran, 1,4-Dioxan, 1,2-Dimethoxyethan, Diethylether, Methyl-*tert*-butylether, Di-*n*-propylether, Di*iso*propylether, Cyclopentylmethylether, und deren Isomeren, und Mischungen davon.

Unter aliphatischen Kohlenwasserstoffen werden vorliegend acyclische, cyclische, gesättigte und ungesättigte Kohlenwasserstoffe verstanden. Der aliphatische Kohlenwasserstoff kann auch 2, 3, 4, 5, 6, 7, 8, 9, 10, 11, 12, 13, 14, 15, 16, 17, 18, 19, 20, 21, 22, 23, 24, 25, 26, 27, 28 oder 29 Kohlenstoffatome aufweisen. Vorteilhaft weist der aliphatische Kohlenwasserstoff 1 bis 20 Kohlenstoffatome auf, vorteilhafter 1 bis 18 Kohlenstoffatome, insbesondere 1 bis 16 Kohlenstoffatome.

Mit aromatischen Kohlenwasserstoffen sind insbesondere Benzol und seine Derivate, z. B. Toluol und Xylol, gemeint.

Gemäß einer weiteren alternativen oder ergänzenden Ausführungsform der hier beanspruchten Verwendung bzw. des hier beschriebenen Verfahrens ist die siliciumorganische Verbindung ein Silikon.

Mit dem Begriff "Silikon" sind im Rahmen der vorliegenden Erfindung polymere und oligomere Siloxane gemeint. Siloxane sind gesättigte Silicium-Sauerstoff-Hydride mit unverzweigten oder verzweigten Ketten, in welchen Silicium- und Sauerstoffatome alternieren. Mithin ist jedes Siliciumatom ist durch einzelne Sauerstoffatome von seinen nächsten Siliciumnachbarn getrennt. Unverzweigte Siloxane besitzen die allgemeine Struktur H₃Si[OSiH₂]ₘOSiH₃. Ein Beispiel für ein verzweigtes Siloxan ist H₃Si[OSiH₂]ₘOSiH[OSiH₂OSiH₃]₂. Vorliegend sind Hydrocarbyl-Derivate mit eingeschlossen. Beispiele für Hydrocarbyl-Derivate sind lineare Siloxane wie Hexamethyldisiloxan, Octamethyltrisiloxan, Decamethyltetrasiloxan und Polydimethylsiloxan sowie cyclische Siloxane wie Octamethylcyclotetrasiloxan, Decamethylcyclopentasiloxan und Dodecamethylcyclohexasiloxan.

Gemäß einer anderen Ausführungsvariante sind die in einem Lösungsmittelgemisch S_{P} enthaltenen Solventien miteinander mischbar.

Im Zusammenhang mit der vorliegenden Erfindung werden zwei Lösungsmittel als mischbar bezeichnet, wenn sie wenigstens während der jeweiligen Umsetzung mischbar sind, also nicht als zwei Phasen vorliegen.

In einer anderen Ausführungsvariante der hier beschriebenen Verwendung bzw. des hier beanspruchten Verfahrens ist R ausgewählt aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten. Als Rest R können also auch primäre, sekundäre, tertiäre Alkyl-, Alkenyl- und Alkinylreste mit 2, 3, 4, 5, 6, 7, 8 oder 9 Kohlenstoffatomen vorgesehen sein sowie cyclische Alkylreste mit 4, 5, 6, 7, 8 oder 9 Kohlenstoffatomen. Gemäß einer anderen Ausführungsform des Verfahrens ist R ausgewählt aus der Gruppe bestehend aus primären, sekundären, tertiären Alkyl-, Alkenyl- und Alkinylresten mit 1 bis 6 Kohlenstoffatomen, cyclischen Alkylresten mit 3 bis 6 Kohlenstoffatomen, einem Benzylrest, einkernigen Arylresten, mehrkernigen Arylresten, einkernigen Heteroarylresten und mehrkernigen Heteroarylresten. Als Rest R können also auch primäre, sekundäre, tertiäre Alkyl-, Alkenyl- und Alkinylreste mit 2, 3, 4 oder 5 Kohlenstoffatomen vorgesehen sein sowie cyclische Alkylreste mit 4 oder 5 Kohlenstoffatomen. In einer noch anderen Variante des Verfahrens ist R ausgewählt aus der Gruppe bestehend aus Me, Et, *n*-Pr, *i*-Pr, *n-*Bu, *i*-Bu, *s*-Bu, *t*-Bu, *n*-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, Cyclopentyl, Cyclohexyl, Phenyl, Tolyl, Benzyl und Cumyl, und deren Isomeren. Gemäß einer anderen vorteilhaften Ausführungsform ist R ausgewählt aus der Gruppe bestehend aus Me, Et, *n*-Pr, *i*-Pr, *n*-Bu, *i*-Bu, *s*-Bu, *t*-Bu, Cyclohexyl, Phenyl, Tolyl, Benzyl und Cumyl, und deren Isomeren, besonders vorteilhaft ist R ausgewählt aus der Gruppe bestehend aus Me, Et, *n*-Pr, *i*-Pr, *n*-Bu, *i*-Bu, *s*-Bu, *t*-Bu, insbesondere ist R = Me.

In einer weiteren Ausführungsform der hier beanspruchten Verwendung bzw. des hier beschriebenen Verfahrens ist vorgesehen, dass in Schritt A.
- eine isomerenreine Verbindung gemäß Formel I oder Formel II
   oder
- ein Isomerengemisch,
   umfassend ein erstes Regioisomer gemäß Formel I und ein zweites Regioisomer gemäß Formel II, insbesondere bestehend aus einem ersten Regioisomer gemäß Formel I und einem zweiten Regioisomer gemäß Formel II,
zur Verfügung gestellt wird.

Der Begriff "isomerenrein" ist bereits weiter oben definiert. Bei der isomerenreinen Verbindung handelt es sich um ein Alkalimetall-8-R-dihydroguajazulenid gemäß Formel I oder ein Alkalimetall-6-R-dihydroguajazulenid gemäß Formel II.

Bei dem ersten Regioisomer des Isomerengemisches handelt es sich um ein Alkalimetall-8-R-dihydroguajazulenid gemäß Formel I. Das zweite Regioisomer des Isomerengemisches ist ein Alkalimetall-6-R-dihydroguajazulenid gemäß Formel II. In einer alternativen oder ergänzenden Ausführungsform der hier beanspruchten Verwendung bzw. des hier beschriebenen Verfahrens beträgt ein Isomerenverhältnis erstes Regioisomer : zweites Regioisomer ≥ 80 : 20 und < 90 : 10, vorteilhaft zwischen 81 : 19 und 89 : 11, insbesondere zwischen 82 : 18 und 88 : 12, beispielsweise 83 : 17 oder 84 : 16 oder 85 : 15 oder 86 : 14 oder 87 : 13.

Die folgende Reaktionsgleichung gilt für den Fall, dass in Schritt A. des hier beschriebenen Verfahrens ein Isomerengemisch eingesetzt wird, bestehend aus einem ersten Regioisomer gemäß Formel I und einem zweiten Regioisomer gemäß Formel II. Dann wird als Produkt ein Diastereomerengemisch des Pt(IV)-Halb*sandwich*-Komplexes [PtMe₃(GuaR)] erhalten, welches die vorliegend maximal mögliche Anzahl an Konfigurationsisomeren enthält. Es liegen dann vier diastereromere Enantiomerenpaare vor wie nachfolgende Abbildung zeigt. Dabei ist (GuaR)¹⁻ = 7-*iso*-Propyl-1,4-dimethyl-8-R-dihydroazulenyl-Anion (Gua-8-R)¹⁻ (unterer Teil der Abbildung, linke Hälfte) bzw. 7-*iso*-Propyl-1,4-dimethyl-6-R-dihydroazulenyl-Anion (Gua-6-Me)¹⁻ (unterer Teil der Abbildung, rechte Hälfte).

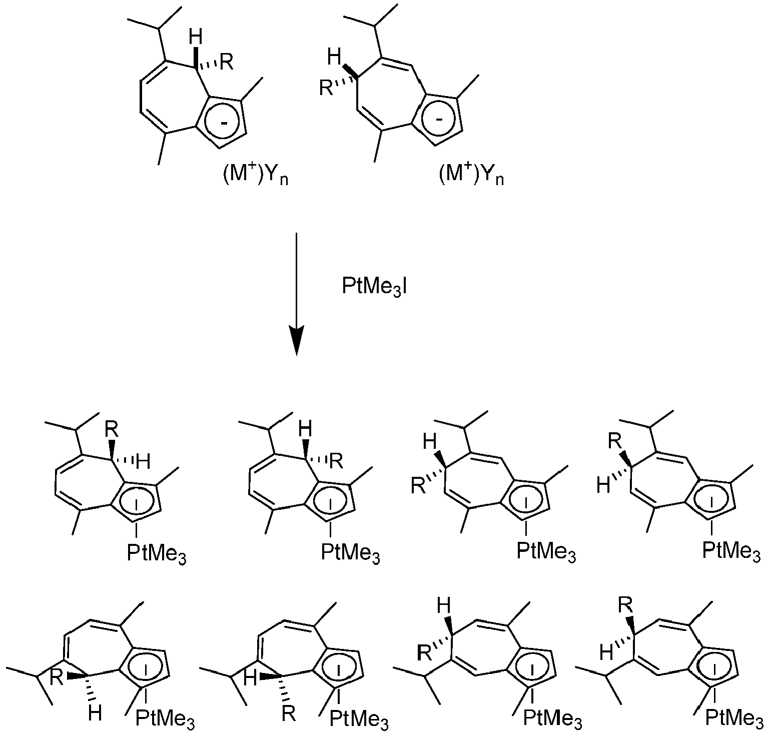

In der oberen Reihe der Produkte sind von links nach rechts gezeigt: [PtMe₃(Gua-8-exo-R)] (III.D1), [PtMe₃(Gua-8-*endo*-R)] (III.D2), [PtMe₃(Gua-6-*endo*-R)] (IV.D3), [PtMe₃(Gua-6-*exo*-R)] (IV.D4). In der unteren Reihe der Produkte ist -jeweils direkt unterhalb jedem der vorgenannten Diastereomere - das jeweils zugehörige Enantiomer gezeigt.

Alternativ oder ergänzend zu Trimethylplatin(IV)-iodid ([PtMe₃I]), welches wie in der Literatur beschrieben im Festkörper als Tetramer [PtMe₃I]₄ vorliegt, können Trimethylplatin(IV)-bromid ([PtMe₃Br]) und/oder Trimethylplatin(IV)-chlorid ([PtMe₃CI]) als Platinvorstufen vorgesehen sein. Letztere liegen im Festkörper ebenfalls als Tetramere [PtMe3Br]4 bzw. [PtMe₃Cl]₄ vor.

Gemäß einer weiteren Ausführungsform der hier beschriebenen Verwendung bzw. des hier beanspruchten Verfahrens zur Herstellung von Platin(IV)-Komplexen gemäß der allgemeinen Formel III und/oder der allgemeinen Formel IV werden in Schritt A. zuvor isolierte und ggf. aufgereinigte Verbindungen gemäß der allgemeinen Formel I und/oder der allgemeinen Formel II eingesetzt.

Die Verbindung gemäß Formel I und/oder Formel II kann in Schritt A. als Substanz, d. h. als Feststoff oder Flüssigkeit, zur Verfügung gestellt werden oder als Lösung oder Suspension in einem, insbesondere aprotisch-polaren, Lösungsmittel. Letzteres ist vorteilhafterweise mit dem in Schritt B. vorgesehen Lösungsmittel S_{P} mischbar oder identisch.

Der Begriff "mischbar" ist bereits weiter oben definiert.

Eine andere Ausführungsform der hier beschriebenen Verwendung bzw. des beanspruchten Verfahrens zur Herstellung von Platin(IV)-Komplexen gemäß Formel III und/oder Formel IV sieht vor, dass die Zurverfügungstellung in Schritt A. eine *in situ*-Herstellung der Verbindung gemäß der allgemeinen Formel I und/oder gemäß der allgemeinen Formel II umfasst. Dabei erfolgt die *in situ*-Herstellung durch Umsetzung von Guajazulen mit einem Alkalimetallorganyl RM in einem Lösungsmittel S_{L}, welches insbesondere ein aprotisch-polares Lösungsmittel umfasst oder ist. R ist wie weiter oben definiert, und M ist ein Alkalimetall, vorteilhaft Li, Na oder K.

Im Zusammenhang mit der vorliegenden Erfindung bedeutet der Ausdruck *"in situ* erzeugt/hergestellt" bzw. *"in situ*-Herstellung", dass die Edukte, welche zur Synthese einer auf diese Weise herzustellenden Verbindung erforderlich sind, in einer geeigneten Stöchiometrie in einem Lösungsmittel oder Lösungsmittelgemisch zur Reaktion gebracht werden und das dabei entstehende Produkt nicht isoliert wird. Vielmehr wird die Lösung oder die Suspension, welches die *in situ* erzeugte Verbindung umfasst, in der Regel direkt, d. h. ohne Isolierung und/oder weitere Aufreinigung, weiterverwendet. Dabei kann die *in situ*-Herstellung einer Verbindung in dem für ihre Weiterverwendung vorgesehenen Reaktionsgefäß erfolgen oder in einem davon unterschiedlichen Reaktionsbehältnis. Die Begriffe Reaktionsbehältnis und Reaktionsgefäß sind bereits weiter oben definiert worden.

Gemäß einer anderen Ausführungsform der hier beschriebenen Verwendung bzw. des beanspruchten Verfahrens umfasst die Zurverfügungstellung in Schritt A. eine *in situ*-Herstellung der Verbindung gemäß der allgemeinen Formel I und/oder gemäß der allgemeinen Formel II, wobei ein molares Verhältnis Guajazulen : Alkalimetallorganyl RM wenigstens 1,00 : 2,00 beträgt. Es kann also beispielsweise auch 1,00 : 1,00 betragen. In einer anderen Ausführungsform des Verfahrens beträgt das molare Verhältnis Guajazulen : Alkalmetallorganyl RM zwischen 1,00 : 2,00 und 2,00 : 1,00, vorteilhafterweise zwischen 1,00 : 1,75 und 1,75 : 1,00, insbesondere zwischen 1,00 : 1,50 und 1,50 : 1,00, beispielsweise 1,00 : 1,95 oder 1,95 : 1,00 oder 1,00 : 1,90 oder 1,90 : 1,00 oder 1,00 : 1,85 oder 1,85 : 1,00 oder 1,00 : 1,80 oder 1,80 : 1,00 oder 1,00 : 1,70 oder 1,70 : 1,00 oder 1,00 : 1,65 oder 1,65 : 1,00 oder 1,00 : 1,60 oder 1,60 : 1,00 oder 1,00 : 1,55 oder 1,55 : 1,00 oder 1,00 : 1,45 oder 1,45 : 1,00 oder 1,00 : 1,40 oder 1,40 : 1,00 oder 1,00 : 1,35 oder 1,35 : 1,00 oder 1,00 : 1,30 oder 1,30 : 1,00 oder 1,00 : 1,25 oder 1,25 : 1,00 oder 1,00 : 1,20 oder 1,20 : 1,00 oder 1,00 : 1,15 oder 1,15 : 1,00 oder 1,00 : 1,10 oder 1,10 : 1,00 oder 1,00 : 1,05 oder 1,05 : 1,00. In einer vorteilhaften Ausführungsform beträgt das molare Verhältnis Guajazulen : Alkalimetallorganyl RM 1,00 : 1,00.

Eine weitere Variante der beanspruchten Verwendung bzw. des beanspruchten Verfahrens sieht vor, dass die *in situ*-Herstellung der Verbindung gemäß der allgemeinen Formel I und/oder gemäß der allgemeinen Formel II in Schritt A. in einem Lösungsmittel oder Lösungsmittelgemisch S_{L} durchgeführt, welches insbesondere mit dem Lösungsmittel S_{P} aus Schritt B. identisch oder mischbar ist. Eine Definition des Begriffes "mischbar" ist bereits weiter oben gegeben. Dann kann - die chemische Inertheit des Lösungsmittels S_{L} und des in Schritt B. vorgesehenen Lösungsmittel S_{P} vorausgesetzt - auf einen Lösungsmittelwechsel verzichtet werden, was aus (verfahrens)ökonomischer und ökologischer Sicht besonders vorteilhaft ist.

In einer weiteren Variante sind die Lösungsmittel S_{P} und S_{L} chemisch inert.

Im Zusammenhang mit der vorliegenden Erfindung ist mit dem Begriff "chemisch inertes Lösungsmittel" ein Lösungsmittel gemeint, welches unter den jeweiligen Verfahrensbedingungen chemisch nicht reaktiv ist. Mithin reagiert das inerte Lösungsmittel unter den jeweiligen Reaktionsbedingungen, einschließlich der Aufreinigungs- und/oder Isolierungsschritte, nicht mit einem potenziellen Reaktionspartner, insbesondere nicht mit einem Edukt und/oder einem Zwischenprodukt und/oder einem Produkt und/oder einem Nebenprodukt, und nicht mit einem anderen Lösungsmittel, Luft oder Wasser.

Beispielsweise ist das aprotisch-polare Lösungsmittel S_{L} ein Ether oder umfasst wenigstens einen Ether. Der Ether kann ausgewählt sein aus der Gruppe bestehend aus Tetrahydrofuran, Methyltetrahydrofuran, 1,4-Dioxan, 1,2-Dimethoxyethan, Diethylether, Methyl-*tert*-butylether, Di-*n*-propylether, Di*iso*propylether, Cyclopentylmethylether, und deren Isomeren, und Mischungen davon.

Gemäß einer alternativen oder ergänzenden Ausführungsform der hier beanspruchten Verwendung bzw. des hier beschriebenen Verfahrens umfasst oder ist das Lösungsmittel S_{L} wenigstens eine siliciumorganische Verbindung, insbesondere ein Silikon. Der Begriff "Silikon" ist bereits weiter oben definiert. Zudem sind in nicht einschränkender Weise Beispiele für Silikone angegeben.

In einer anderen Variante des Verfahrens zur Herstellung von Platin(IV)-Komplexen gemäß den Formeln III und IV ist vorgesehen, dass die Synthese in Schritt B. wenigstens eine salzmetathetische Umsetzung umfasst.

Eine noch andere Ausführungsform des hier beanspruchten Verfahrens sieht vor, dass die in Schritt A. zur Verfügung zu stellende Platinvorstufe ausgewählt ist aus der Gruppe bestehend aus Trimethylplatin(IV)-iodid, Trimethylplatin(IV)-bromid und Trimethylplatin(IV)-chlorid, und Mischungen davon, wobei die Zurverfügungstellung
i. als Lösung, welche die Platinvorstufe und ein Lösungsmittel S_{A} umfasst, welches insbesondere mit dem Lösungsmittel S_{P} mischbar oder identisch ist,
   oder
ii. als Feststoff
   erfolgt.

Das Lösungsmittel S_{A} ist vorteilhafterweise ein aprotisch-polares Lösungsmittel oder Lösungsmittelgemisch. Besonders vorteilhaft ist es, wenn das Lösungsmittel S_{A} einen Ether umfasst oder ein Ether ist. Dabei ist der Ether zum Beispiel ausgewählt aus der Gruppe bestehend aus Tetrahydrofuran, Methyltetrahydrofuran, 1,4-Dioxan, 1,2-Dimethoxyethan, Diethylether, Methyl-*tert*-butylether, Di-*n*-propylether, Di*iso*propylether, Cyclopentylmethylether, und deren Isomeren, und Mischungen davon.

Eine andere Ausführungsform der Verwendung bzw. des Verfahrens sieht vor, dass das Alkalimetallkation M⁺ ausgewählt ist aus der Gruppe bestehend aus Li⁺, Na⁺ und K+.

Gemäß einer weiteren Variante des hier beanspruchten Verfahrens ist vorgesehen, dass der Neutralligand Yein aprotisch-polares Lösungsmittel ist. Vorteilhaft ist das aprotisch-polare Lösungsmittel ausgewählt aus der Gruppe bestehend aus Alkoxyalkanen, Thioethern und tertiären Aminen, insbesondere aus der Gruppe bestehend aus Alkoxyalkanen und Thioethern. Die Begriffe "Alkoxyalkan" und "Thioether" sind bereits weiter oben definiert.

In einer anderen Variante des hier beanspruchten Verfahrens ist der Neutralligand Y ein Kronenether, welcher ausgewählt ist aus der Gruppe bestehend aus makrocyclischen Polyethern und deren Aza-, Phospha- und Thia-Derivaten, wobei ein Innendurchmesser des Kronenethers und ein lonenradius des jeweiligen Alkalimetallkations M⁺ miteinander korrespondieren. Eine Definition des Begriffes "Kronenether" ist bereits weiter oben gegeben.

Gemäß einer weiteren Ausführungsform des hier beanspruchten Verfahrens ist als Neutralligand Y ein Glycol-dialkylether vorgesehen, ausgewählt aus der Gruppe bestehend aus einem Monoethylenglycoldialkylether, einem Diethylenglycoldialkylether, einem Triethylengylcoldialkylether, einem Monopropylenglycoldialkylether, einem Dipropylenglycoldialkylether, einem Tripropylenglycoldialkylether, einem Monooxomethylendialkylether, einem Dioxomethylendialkylether und einem Trioxomethylendialkylether, deren Isomerenmischungen, und Mischungen davon. Eine Auswahl verwendbarer Glykoldialkylether ist in nicht einschränkender Weise weiter oben angegeben.

Eine weitere Ausführungsform des hier beanspruchten Verfahrens sieht vor, dass der Neutralligand Y ein Ether ist. Beispielsweise kann der Ether ein nicht-cyclischer oder ein cyclischer Ether sein ausgewählt aus der Gruppe bestehend aus Dialkylethern, Cyclopentylmethylether, Tetrahydrofuran, 2-Methyltetrahydrofuran, 3-Methyltetrahydrofuran, Tetrahydropyran, 1,4-Dioxan, 1,2-Dimethoxyethan, und deren Isomeren, und Mischungen davon, insbesondere aus der Gruppe bestehend aus Diethylether, Methyl-*tert*-butylether, Di-*n*-propylether, Di-*iso-*propylether, Di-*n-*butylether, Di-*iso*-butylether, Di-*tert*-butylether, Tetrahydrofuran, 2-Methyltetrahydrofuran, 3-Methyltetrahydrofuran, Tetrahydropyran, 1,4-Dioxan, 1,2-Dimethoxyethan, und deren Isomeren, und Mischungen davon.

Besonders vorteilhaft ist, dass die Verwendung von Verbindungen gemäß der allgemeinen Formel I und/oder der allgemeinen Formel II zur Herstellung von Platin(IV)-Komplexen gemäß der allgemeinen Formel III und/oder der allgemeinen Formel IV bzw. das Verfahren zur Herstellung solcher Platin(IV)-Komplexe unter Verwendung von Verbindungen gemäß der allgemeinen Formel I und/oder der allgemeinen Formel II die Darstellung hochreiner Platin(IV)-Komplexe ermöglichen. Insbesondere kann mittels des hier beschriebenen Verfahrens eine Vielzahl von, insbesondere bei Raumtemperatur flüssigen, Diastereomerenmischungen von Platin(IV)-Komplexen in guter Ausbeute von üblicherweise ≥ 60 %, vorteilhaft ≥ 70 %, sowie in guter Raum-Zeit-Ausbeute zur Verfügung gestellt werden. Ein Beispiel für ein solches Diastereomerengemisch ist [PtMe₃(GuaMe)]. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, z. B. von Lösungsmitteln, mittels gaschromatographischer Verfahren (GC), ggf. mit Massenspektrometrie-Kopplung (GC-MS), bestimmt werden kann.

Definitionen des Ausdrucks "hochreiner Platin(IV)-Komplex" bzw. "Platin(IV)-Komplex in hoher Reinheit" sowie des Begriffes "Raum-Zeit-Ausbeute" sind weiter oben angegeben.

Mittels des hier beschriebenen Verfahrens ist beispielsweise der Platin(IV)-Halb*sandwich*-Komplex [PtMe₃(GuaMe)] herstellbar, z. B. ausgehend von der Platinvorstufe [PtMe₃I]₄ und einem Isomerengemisch eines Lithiumdihydroguajazulenids, welches aus einem ersten Regioisomer gemäß Formel I.1 und einem zweiten Regioisomer gemäß Formel II.1 besteht:

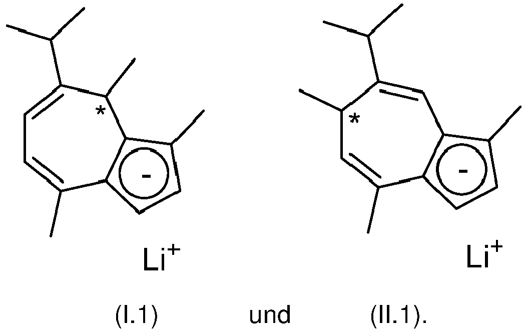

Die Reaktionsgleichung für die Darstellung von [PtMe₃(GuaMe)] lautet dann:

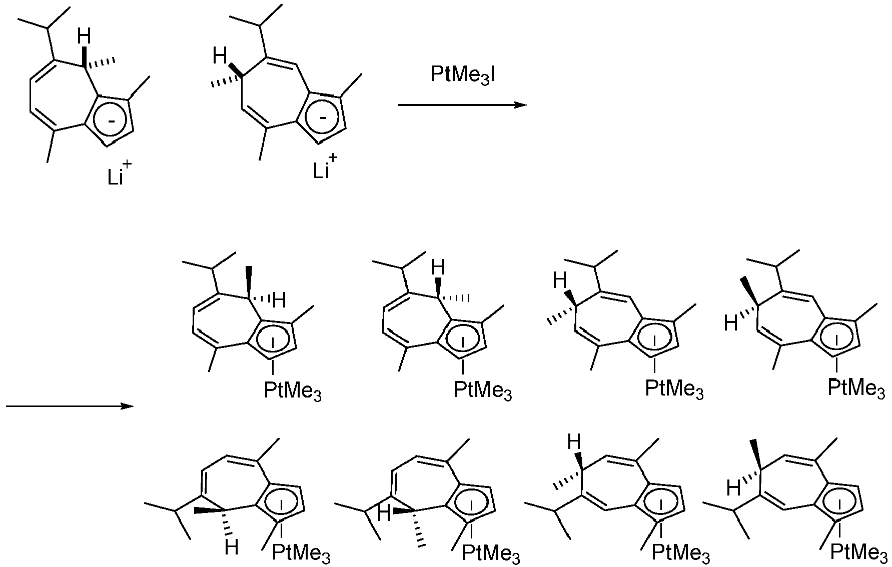

Bei dieser Reaktionsführung wird der Platin(IV)-Komplex [PtMe₃(GuaMe)] als Diastereomerengemisch bestehend aus acht Konfigurationsisomeren, die sich durch das Vorliegen planarer und zentraler Chiralität ergeben, nämlich vier Diastereomeren sowie deren Enantiomeren, erhalten, und zwar in Form eines Öls. Die Ausbeute beträgt üblicherweise > 75%, beispielsweise 77 %.

Ist eine Aufreinigung des erhaltenen Öls gewünscht oder erforderlich, kann diese vorteilhafterweise mittels einer einfachen Sublimation und/oder Destillation erfolgen. Eine weitere - je nach Wahl der Edukte, Reaktionsbedingungen und Lösungsmittel zur Herstellung des Platin(IV)-Komplexes und/oder der Verwendung des Platin(IV)-Komplexes nicht zwingend erforderliche - Aufreinigung des Produktes kann mittels Säulenchromatographie erfolgen, beispielsweise über Silica oder Al₂O₃ (neutral) mit einem aprotisch-unpolaren Lösungsmittel, z. B. Hexan, als Eluent. Unter Lichtausschluss ist die monomere Verbindung [PtMe₃(GuaMe)] für mehrere Monate bei Raumtemperatur lagerbar. Währenddessen werden weder Zersetzungsreaktionen noch eine Oligomerisierung oder Polymerisierung beobachtet.

Lediglich die Dünnschichtchromatographie liefert Hinweise auf die vier möglichen Diastereomere [PtMe₃(Gua-6-*endo*-Me)], [PtMe₃(Gua-6-*exo*-Me)], [PtMe₃(Gua-8-*endo-Me*)] und [PtMe₃(Gua-8-*exo*-Me)], welche jeweils als Enantiomergemische vorliegen. Mittels ¹H-NMR-Spektroskopie werden ausschließlich Signale der Diastereomere detektiert, welche ausgehend von dem Lithium-dihydroguajazulenid gemäß Formel I.1 erhalten werden, also die Diastereomere gemäß der Formel [PtMe₃(Gua-8-Me)] bzw. gemäß den Formeln [PtMe₃(Gua-8-*exo*-Me)] (III.D1.1) und [PtMe₃(Gua-8-*endo*-Me)] (III.D2.1). Letztere sind nachstehend gezeigt:

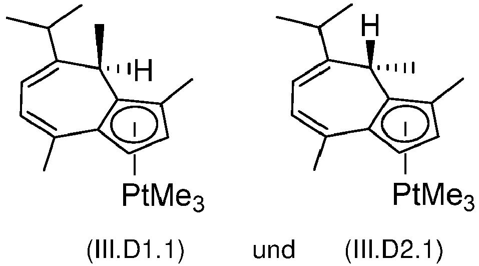

Dabei stellt das Diastereomer gemäß Formel III.D1.1 das Haupt-Diastereomer dar. Mittels ¹H-NMR-Spektroskopie wurde beispielsweise ein Diastereomerenverhältnis *d.r.* (englisch: *diastereomeric ratio*) erstes Diastereomer (III.D1.1) : zweites Diastereomer von 68 : 32 (III.D2.1) ermittelt. Die ausgehend von dem Lithium-dihydroguajazulenid gemäß Formel II.1 erhaltenen Diastereomere gemäß der Formel [PtMe₃(Gua-6-Me)] bzw. gemäß den Formeln PtMe₃(Gua-6-*endo*-Me)] (IV.D3.1) und [PtMe₃(Gua-6-*exo*-Me)] (IV.D4.1) wurden nur mittels Dünschichtchromatographie detektiert. Sie sind nachstehend dargestellt:

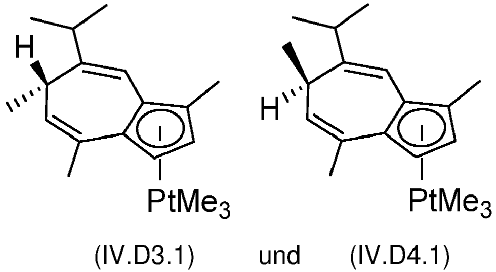

Die Tatsache, dass der mittels des hier beschriebenen Verfahrens erhältliche Platin(IV)-Komplex in isolierter Form bei Raumtemperatur als Flüssigkeit vorliegt, ist insbesondere mit Blick auf eine Verwendung als Platinpräkursor-Verbindung für Gasphasenabscheidungsprozesse, insbesondere Niedrigtemperatur-Gasphasenabscheidungsverfahren, von Vorteil. Erwähnenswert ist zudem, dass der erhaltene Komplex [PtMe₃(GuaMe)] eine relativ hohe thermische Stabilität aufweist. Laut thermogravimetrischer Analyse (TGA) erfolgt ein 3%-Abbau bei einer Temperatur von 180 °C.

Darüber hinaus kann diese bei Raumtemperatur flüssige Platin(IV)-Verbindung vorteilhaft als Präkatalysator und/oder Katalysator in der Katalyse eingesetzt werden, z. B. für lichtinduzierte Platin-katalysierte Hydrosilylierungsreaktionen, die Hydrierung ungesättigter Verbindungen und Polymerisationsreaktionen, bei denen die Aktivierung durch ultraviolette oder sichtbare Strahlung erfolgt. Denn aufgrund der Tatsache, dass der Platin(IV)-Komplex [PtMe₃(GuaMe)] in Form eines Öls erhalten wird, ist er - im Vergleich zu vorbekannten, Cyclopentadienyl-Anionen enthaltenden und häufig wachsartig vorliegenden Platin(IV)-Verbindungen wie [PtMe₃(MeCp)] - einfacher handhabbar. Die Platin(IV)-Verbindung [PtMe₃(GuaMe)] zeigt zudem eine Absorption im Vis-Bereich. Das stellt im Rahmen lichtinduzierter Platin-katalysierter Reaktionen, beispielsweise Hydrosilylierungsreaktionen, einen weiteren Vorteil dar. Denn dabei ist regelmäßig die Verwendung von UV/Vis-Licht vorgesehen, wodurch üblicherweise besondere Sicherheitsmaßnahmen erforderlich sind, um das Hautkrebsrisiko zu reduzieren. Solche Sicherheitsmaßnahmen sind bei Verwendung von [PtMe₃(GuaMe)] nicht zwingend notwendig.

Die nach der Synthese in Schritt B. vorliegende Lösung, umfassend die in Lösung befindliche Zielverbindung gemäß der allgemeinen Formel III und/oder Formel IV, kann unmittelbar mit einem oder mehreren weiteren Reaktanden umgesetzt werden. Alternativ wird nach der Umsetzung ein Schritt C. durchgeführt wird, welcher eine Isolierung des Platin(IV)-Komplexes gemäß Formel III und/oder Formel IV umfasst:
- als Lösung, welche den Platin(IV)-Komplex gemäß Formel III und/oder gemäß Formel IV und das Lösungsmittel S_{P} umfasst,
   oder
- als Feststoff oder Flüssigkeit.

Die Isolierung des Platin(IV)-Komplexes gemäß Formel III und/oder Formel IV als Lösung, als Feststoff oder als Flüssigkeit kann einen oder mehrere Verfahrensschritte umfassen, wie z. B. einen oder mehrere Filtrationsschritte, die Reduzierung des Volumens der Mutterlauge, d. h. Einengen, z. B. mittels "*bulbto-bulb*", die Zugabe eines Lösungsmittels und/oder einen Lösungsmittelaustausch, um eine Fällung des Produktes aus der Mutterlauge zu erzielen und/oder Verunreinigungen und/oder Edukte zu entfernen, eine Sublimation, eine Destillation, eine säulenchromatographische Aufreinigung, Waschen und Trocknen des Produktes. Gegebenenfalls auch eine Filtration über einem Reinigungsmedium, wie z. B. Aktivkohle oder Silica, z. B. Celite^{®}, durchgeführt werden. Die vorgenannten Schritte können jeweils in unterschiedlichen Reihenfolgen und Häufigkeiten vorgesehen sein.

Die Aufgabe wird außerdem gelöst durch
▪ einen Platin(IV)-Komplex gemäß der allgemeinen Formel 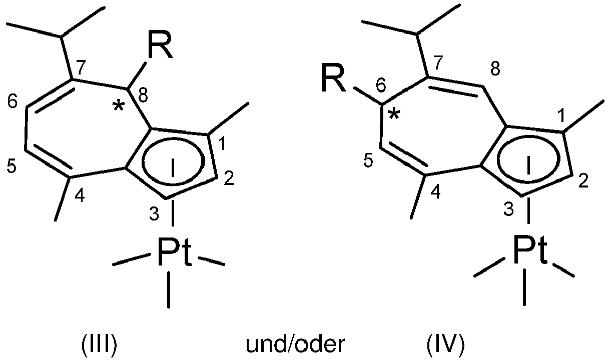 oder
▪ eine Lösung oder eine Suspension, umfassend einen Platin(IV)-Komplex gemäß der allgemeinen Formel 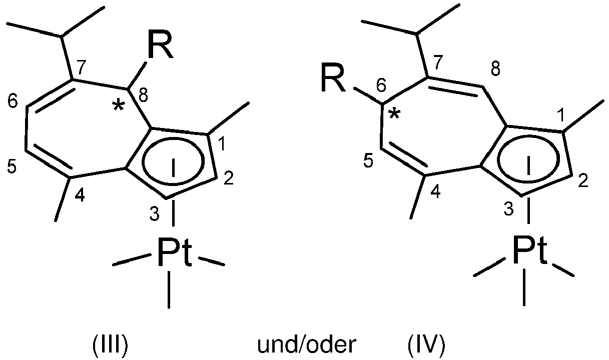
und ein Lösungsmittel, welches insbesondere mit dem Lösungmittel S_{P} mischbar oder identisch ist,
wobei
R jeweils ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten.

Zudem wird die Aufgabe gelöst durch
▪ einen Platin(IV)-Komplex gemäß der allgemeinen Formel 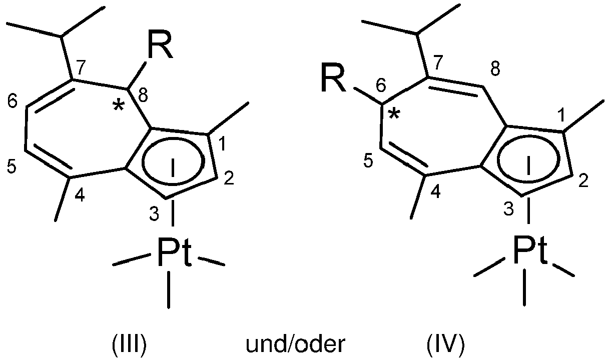 oder
▪ eine Lösung oder eine Suspension, umfassend einen Platin(IV)-Komplex gemäß der allgemeinen Formel 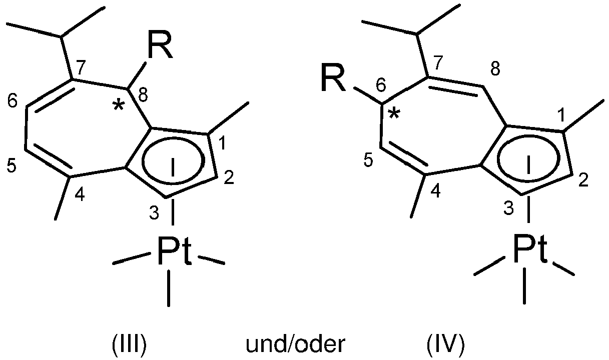 und ein Lösungsmittel, welches insbesondere mit dem Lösungmittel S_{P} mischbar oder identisch ist,
   wobei
   R jeweils ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
   erhalten oder erhältlich nach einem Verfahren zur Herstellung solcher Platin(IV)-Komplexe gemäß einer der weiter oben beschriebenen Ausführungsformen.

Dabei umfassen die allgemeinen Formeln III und IV jeweils sowohl die Monomere als auch etwaige Oligomere, insbesondere Dimere, und Lösungsmitteladdukte. Zudem liegen die beanspruchten Verbindungen gemäß den allgemeinen Formeln III und IV insbesondere jeweils als ein Diastereomerengemisch vor. Mit anderen Worten: Im Falle des Platin(IV)-Komplexes gemäß Formel III stellt das Kohlenstoffatom C8 ein Stereozentrum dar, im Falle des Platin(IV)-Komplexes gemäß Formel IV stellt das Kohlenstoffatom C6 ein Stereozentrum dar.

Alternativ kann - insbesondere in Abhängigkeit vom gewählten Substituenten R - ein Diastereomerengemisch erhalten werden, welches sowohl das Diastereomerengemisch der Verbindung gemäß Formel III als auch das Diastereomerengemisch der Verbindung gemäß Formel IV umfasst, insbesondere daraus besteht.

In jedem der vorgenannten Fälle liegt jedes Diastereomer als ein Enantiomerengemisch vor, wobei die Diastereomere unabhängig voneinander jeweils als ein Racemat vorliegen können.

Die Platin(IV)-Komplexe gemäß den allgemeinen Formeln III und IV werden üblicherweise solvensfrei, d. h. nicht als Solvensaddukte, erhalten bzw. liegen in der Regel solvensfrei vor. Mittels des weiter oben beschriebenen Verfahrens zur Herstellung solcher Platin(IV)-Komplexe können aber auch Lösungsmitteladdukte dieser Metallkomplexe erhalten werden. Im Falle eines solchen Adduktes ist das Lösungsmittel insbesondere mit dem - im Rahmen des weiter oben beschriebenen Verfahrens verwendeten - Lösungsmittel S_{P} identisch, insbesondere wenn es sich bei dem Lösungsmittel S_{P} um ein Alkoxyalkan handelt oder das Lösungsmittel S_{P} ein Alkoxyalkan umfasst.

Die hier beanspruchten Platin(IV)-Komplexe gemäß den allgemeinen Formeln III und IV weisen jeweils ein Organo-dihydroguajazulenyl-Anion bzw. R-Dihydroguajazulenyl-Anion (GuaR)¹⁻ auf, welches in 8-Position oder in 6-Position des Guajazulengerüsts zusätzlich zu einem H-Atom einen Organylrest R trägt. Bei dem R-Dihydroguajazulenyl-Anion (GuaR)¹⁻ kann es sich also um ein 7-*iso*-Propyl-1,4-dimethyl-8-R-dihydroazulenyl-Anion bzw. 8-R-Dihydroguajazulenyl-Anion (Gua-8-R)¹⁻ gemäß Formel III oder um ein 7-*iso*-Propyl-1,4-dimethyl-6-R-dihydroazulenyl-Anion bzw. 6-R-Dihydroguajazulenyl-Anion (Gua-6-R)¹⁻ gemäß Formel IV handeln. Anders ausgedrückt: In C8-Position oder in C6-Position des Guajazulengerüsts liegt eine RCH-Gruppe vor. Infolge der Addition eines Organyl-Anions (R)¹⁻ wird die Aromatizität auf den Fünfring beschränkt, wobei in der Regel die für Azulen und dessen Derivate typische blaue Farbe verloren geht. Das Organo-dihydroguajazulenyl-Anion (GuaR)¹⁻ stellt ein Derivat des Cyclopentadienyl-Anions bzw. ein Cyclopentadienyl-artiges Monoanion dar.

Aufgrund des Vorhandenseins eines R-Dihydroguajazulenyl-Anions, also eines Cyclopentadienyl-artigen Monoanions, eignen sich die Verbindungen gemäß den allgemeinen Formeln III und IV insbesondere als Präkatalysatoren und/oder als Katalysatoren für chemische Reaktionen, in welchen ansonsten Platin(IV)-Komplexe eingesetzt werden, die Cyclopentadienyl-Liganden aufweisen. Dies ist besonders vorteilhaft, weil die Zurverfügungstellung eines R-Dihydroguajazulenyl-Liganden im Vergleich zur Bereitstellung des Cyclopentadienyl-Liganden weniger arbeits- und zeitaufwändig ist. Insbesondere können die hier als Edukte eingesetzten Verbindungen, welche Cyclopentadienyl-artige Liganden umfassen, unter Verwendung von kostengünstigen nachwachsenden Rohstoffen hergestellt werden. Denn Guajazulen ist partialsynthetisch zugänglich, und zwar ausgehend von dem Naturstoff Guajol und anderen Azulenbildnern durch einfache Dehydratisierung und Dehydrierung (T. Shono, N. Kise, T. Fujimoto, N. Tominaga, H. Morita, J. Org. Chem. 1992, 57, 26, 7175 - 7187; CH 314 487 A (B. Joos) 29.01.1953). Infolgedessen fallen auch der Syntheseaufwand und die Herstellungskosten für die hier beanspruchten Platin(IV)-Komplexe sowie Lösungen oder Suspensionen, umfassend eine solche Verbindung und ein Lösungsmittel, welches insbesondere mit dem Lösungmittel S_{P} mischbar oder identisch ist, geringer aus als für analoge Platin(IV)-Cp-Komplexe. Mithin stellen die hier beschriebenen Platin(IV)-Komplexe sowie deren Lösungen und Suspensionen insbesondere mit Blick auf eine industrielle Anwendung eine relativ kostengünstige und insbesondere nachhaltige Alternative für Platin(IV)-Cyclopentadienyl-Komplexe dar.

Bei Guajazulen handelt es sich um einen Naturstoff, der in Kamillenöl und anderen ätherischen Ölen enthalten und daher vorteilhafterweise in großen Mengen kostengünstig zugänglich ist. Synthetisch kann es aus dem Guajol des Guajakholzöls (Guajakharz) hergestellt werden. Guajazulen ist eine intensiv blaue Substanz mit entzündungshemmender Wirkung.

In einer Ausführungsvariante der hier beanspruchten Verbindungen gemäß Formel III und/oder Formel IV ist R ausgewählt aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten. Als Rest R können also auch primäre, sekundäre, tertiäre Alkyl-, Alkenyl- und Alkinylreste mit 2, 3, 4, 5, 6, 7, 8 oder 9 Kohlenstoffatomen vorgesehen sein sowie cyclische Alkylreste mit 4, 5, 6, 7, 8 oder 9 Kohlenstoffatomen. Gemäß einer anderen Ausführungsform der beanspruchten Verbindungen gemäß Formel III und/oder Formel IV ist R ausgewählt aus der Gruppe bestehend aus primären, sekundären, tertiären Alkyl-, Alkenyl- und Alkinylresten mit 1 bis 6 Kohlenstoffatomen, cyclischen Alkylresten mit 3 bis 6 Kohlenstoffatomen, einem Benzylrest, einkernigen Arylresten, mehrkernigen Arylresten, einkernigen Heteroarylresten und mehrkernigen Heteroarylresten. Als Rest R können also auch primäre, sekundäre, tertiäre Alkyl-, Alkenyl- und Alkinylreste mit 2, 3, 4 oder 5 Kohlenstoffatomen vorgesehen sein sowie cyclische Alkylreste mit 4 oder 5 Kohlenstoffatomen. In einer noch anderen Variante der beanspruchten Verbindungen ist R ausgewählt aus der Gruppe bestehend aus Me, Et, *n*-Pr, *i*-Pr, *n*-Bu, *i*-Bu, *s*-Bu, *t*-Bu, *n*-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, Cyclopentyl, Cyclohexyl, Phenyl, Tolyl, Benzyl und Cumyl, und deren Isomeren. Gemäß einer weiteren vorteilhaften Ausführungsform ist R ausgewählt aus der Gruppe bestehend aus Me, Et, *n*-Pr, *i*-Pr, *n*-Bu, *i*-Bu, *s*-Bu, *t*-Bu, Cyclohexyl, Phenyl, Tolyl, Benzyl und Cumyl, und deren Isomeren, besonders vorteilhaft ist R ausgewählt aus der Gruppe bestehend aus Me, Et, *n*-Pr, *i*-Pr, *n*-Bu, *i*-Bu, *s*-Bu, *t*-Bu, insbesondere ist R = Me.

Gemäß einer weiteren Ausführungsform der beanspruchten Platin(IV)-Komplexe oder Lösungen oder Suspensionen liegt ein Diastereomerengemisch vor, umfassend
- ein erstes Diastereomer gemäß Formel III.D1 und ein zweites Diastereomer gemäß Formel III.D2, 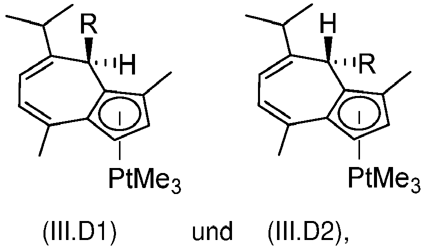 und/oder
- ein drittes Diastereomer gemäß Formel IV.D3 und ein viertes Diastereomer gemäß Formel IV.D4, 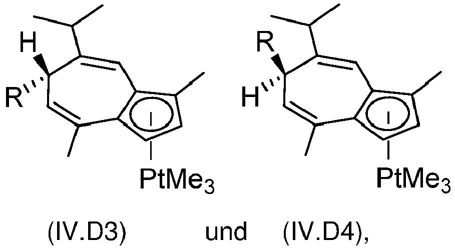
wobei jedes Diastereomer als ein Enantiomerengemisch vorliegt.

Dabei weisen das erste und das zweite Diastereomer jeweils in C8-Position des Guajazulengerüsts eine RCH-Gruppe auf, und das dritte und das vierte Diastereomer weisen jeweils in C6-Position des Guajazulengerüsts eine RCH-Gruppe auf. Mit anderen Worten: Im Falle des ersten Diastereomers (III.D1) und des zweiten Diastereomers (III.D2) stellt das Kohlenstoffatom C8 jeweils ein Stereozentrum dar, im Falle des dritten Diastereoemers (IV.D3) und des vierten Diastereomers (IV.D4) stellt jeweils das Kohlenstoffatom C6 ein Stereozentrum dar. Zudem liegt jedes der vier möglichen Diastereomere jeweils als ein Enantiomerengemisch vor. Mithin weist ein Diastereomerengemisch eines hier beanspruchten Platin(IV)-Komplexes wenigstens vier Stereoisomere auf, nämlich zwei Diastereomere sowie deren insgesamt zwei Enantiomere. Anders ausgedrückt: Ein hier beschriebener Platin(IV)-Komplex kann als ein Gemisch von vier oder acht Konfigurationsisomeren vorliegen, also als eine Mischung von zwei oder vier Diastereomeren zuzüglich eines Enantiomers pro Diastereomer.

In einer anderen Ausführungsvariante der hier beanspruchten Platin(IV)-Komplexe oder Lösungen oder Suspensionen liegt wenigstens ein Diastereomer als ein Racemat vor. Die Diastereomere können also unabhängig voneinander jeweils als Racemat vorliegen.

Nach einer noch anderen Ausführungsform der hier beschriebenen Platin(IV)-Komplexe oder Lösungen oder Suspensionen liegt ein Gemisch zweier Diastereomere vor, wobei ein Diastereomerenverhältnis
- erstes Diastereomer: zweites Diastereomer
   oder
- drittes Diastereomer: viertes Diastereomer
   ≥ 60 : 40 und < 90 : 10 beträgt, vorteilhaft zwischen 61 : 39 und 89 : 11, noch vorteilhafter zwischen 62 : 38 und 88 : 12, insbesondere zwischen 63 : 37 und 87 : 13, beispielsweise 64 : 36 oder 65 : 35 oder 66 : 34 oder 67 : 33 oder 68 : 32 oder 69 : 31 oder 70 : 30 oder 71 : 29 oder 72 : 28 oder 73 : 27 oder 74 : 26 oder 75 : 25 oder 76 : 24 oder 77 : 23 oder 78 : 22 oder 79 : 21 oder 80 : 20 oder 81 : 19 oder 82 : 18 oder 83 : 17 oder 84 : 16 oder 85 : 15 oder 86 : 14 oder 87 : 13.

Dabei ist das jeweilige Diastereomerenverhältnis d.r. beispielsweise mittels Kernresonanzspektroskopie bestimmt, insbesondere mittels ¹H-NMR-Spektroskopie.

Gemäß einer weiteren vorteilhaften Ausführungsform der hier beanspruchten Platin(IV)-Komplexe oder Lösungen oder Suspensionen liegt ein Diastereomerengemisch vor, bestehend aus dem ersten Diastereomer gemäß Formel III.D1 und dem zweiten Diastereomer gemäß Formel III.D2, 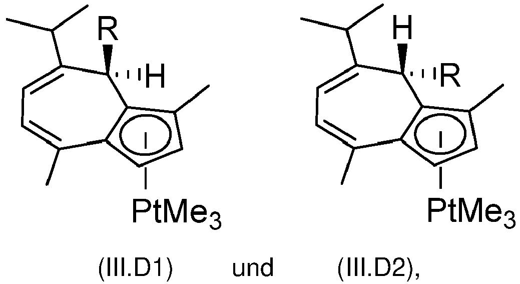 wobei jedes Diastereomer als ein Enantiomerengemisch vorliegt.

Bei dem vorgenannten Diastereomerengemisch handelt es sich um das Diastereomerengemisch des Platin(IV)-Komplexes gemäß Formel III, als um das Diastereomerengemisch der Verbindung [PtMe₃(Gua-8-R)]. Dieses Diastereomerengemisch besteht aus genau vier Konfigurationsisomeren gemäß der nachfolgenden Abbildung, nämlich aus den beiden Diastereomeren [PtMe₃(Gua-8-exo-R)] und [PtMe₃(Gua-8-*endo*-R)] (obere Reihe, von links nach rechts) und deren Enantiomeren (untere Reihe, von links nach rechts).

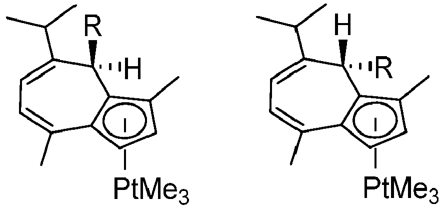

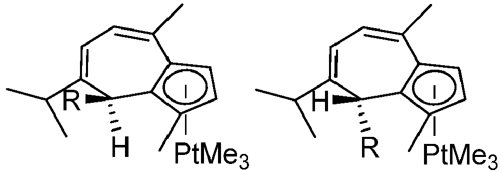

Gemäß einer weiteren vorteilhaften Ausführungsform der hier beanspruchten Platin(IV)-Komplexe oder Lösungen oder Suspensionen ist R = Me. Dabei liegt der Platin(IV)-Komplex als ein Gemisch zweier Diastereomere, nämlich eines ersten Diastereomers gemäß Formel III.D1.1 und eines zweiten Diastereomers gemäß Formel III.D2.1, vor, insbesondere in isolierter Form als bei Raumtemperatur flüssiges Diastereomerengemisch. Die nachfolgende Abbildung zeigt die beiden vorgenannten Diastereomere:

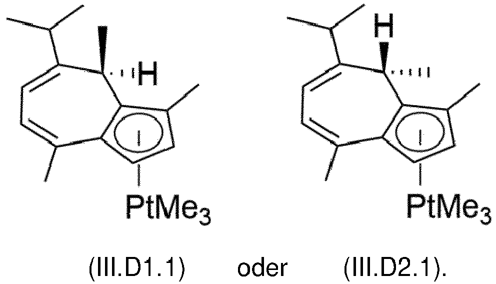

Gemäß einer alternativen oder ergänzenden Ausführungsform beträgt das Diastereomerenverhältnis erstes Diastereomer (Formel III.D1.1) : zweites Diastereomer (Formel III.D2.1) zwischen 65 : 35 und 75 : 25, beispielsweise 68 : 32, wobei jedes Diastereomer als ein Enantiomerengemisch vorliegt, unabhängig voneinander jeweils optional als ein Racemat.

Nach einer anderen Ausführungsform der hier beanspruchten Platin(IV)-Komplexe oder Lösungen oder Suspensionen ist R = Me und der Platin(IV)-Komplex [PtMe₃(GuaMe)] liegt als ein Gemisch vierer Diastereomere vor, insbesondere in isolierter Form als bei Raumtemperatur flüssiges Diastereomerengemisch, bestehend aus vier diastereomeren Enantiomerenpaaren, wie nachfolgende Abbildung zeigt. Dabei ist (GuaMe)¹⁻ = 7-*iso-*Propyl-1,4,8-trimethyl-dihydroazulenyl-Anion (Gua-8-Me)¹⁻ (linke Hälfte der Abbildung) bzw. 7-*iso*-Propyl-1,4,6-trimethyl-dihydroazulenyl-Anion (Gua-6-Me)¹⁻ (rechte Hälfte der Abbildung).

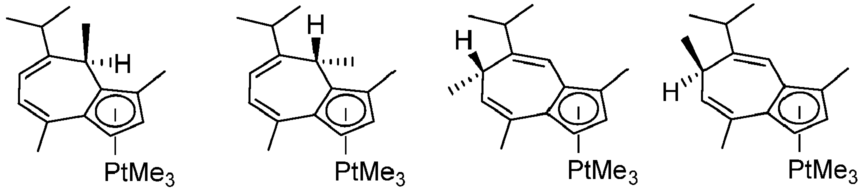

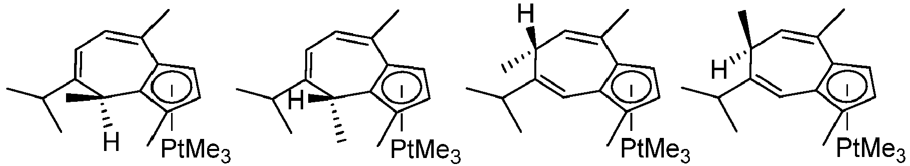

Die Abbildung zeigt in der oberen Reihe von links nach rechts: [PtMe₃(Gua-8-*exo-*Me)] (III.D1.1), [PtMe₃(Gua-8-*endo*-Me)] (III.D2.1), [PtMe₃(Gua-6-*endo*-Me)] (IV.D3.1), [PtMe₃(Gua-6-*exo*-Me)] (IV.D4.1). In der unteren Reihe ist - jeweils direkt unterhalb jedem der vorgenannten Diastereomere - das jeweils zugehörige Enantiomer gezeigt. Gemäß einer alternativen oder ergänzenden Ausführungsvariante beträgt das Diastereomerenverhältnis erstes Diastereomer (Formel III.D1.1) : zweites Diastereomer (Formel III.D2.1) zwischen 65 : 35 und 75 : 25 beträgt, beispielsweise 68 : 32, wobei jedes Diastereomer optional als ein Racemat vorliegt.

Die Tatsache, dass der hier beschriebene Platin(IV)-Komplex [PtMe₃(GuaMe)] in isolierter Form als bei Raumtemperatur flüssiges Diastereomerengemisch vorliegt, ist insbesondere mit Blick auf eine Verwendung als Platinpräkursor-Verbindung für Gasphasenabscheidungsprozesse, insbesondere Niedrigtemperatur-Gasphasenabscheidungsverfahren, von Vorteil. Erwähnenswert ist zudem, dass der Komplex [PtMe₃(GuaMe)] eine relativ hohe thermische Stabilität aufweist. Laut thermogravimetrischer Analyse (TGA) erfolgt ein 3%-Abbau bei einer Temperatur von 180 °C.

Darüber hinaus kann diese bei Raumtemperatur flüssige Platin(IV)-Verbindung vorteilhaft als Präkatalysator und/oder Katalysator in der Katalyse eingesetzt werden, z. B. für lichtinduzierte Platin-katalysierte Hydrosilylierungsreaktionen, die Hydrierung ungesättigter Verbindungen und Polymerisationsreaktionen, bei denen die Aktivierung durch ultraviolette oder sichtbare Strahlung erfolgt. Denn aufgrund der Tatsache, dass der Platin(IV)-Komplex [PtMe₃(GuaMe)] in Form eines Öls erhalten wird bzw. vorliegt, ist er - im Vergleich zu vorbekannten, Cyclopentadienyl-Anionen enthaltenden und häufig wachsartig vorliegenden Platin(IV)-Verbindungen wie [PtMe₃(MeCp)] - einfacher handhabbar. Die Platin(IV)-Verbindung [PtMe₃(GuaMe)] zeigt zudem eine Absorption im Vis-Bereich. Das stellt im Rahmen lichtinduzierter Platin-katalysierter Reaktionen, beispielsweise Hydrosilylierungsreaktionen, einen weiteren Vorteil dar. Denn dabei ist regelmäßig die Verwendung von UV/Vis-Licht vorgesehen, wodurch üblicherweise besondere Sicherheitsmaßnahmen erforderlich sind, um das Hautkrebsrisiko zu reduzieren. Solche Sicherheitsmaßnahmen sind bei Verwendung von [PtMe₃(GuaMe)] nicht zwingend notwendig. Weiterhin vorteilhaft ist, dass die monomere Verbindung [PtMe₃(GuaMe)] unter Lichtausschluss für mehrere Monate bei Raumtemperatur lagerbar ist. Währenddessen werden weder Zersetzungsreaktionen noch eine Oligomerisierung oder Polymerisierung beobachtet.

Gemäß einer vorteilhaften Ausführungsform weist der isolierte Platin(IV)-Komplex gemäß der Formel III und/oder der Formel IV einen Gesamtgehalt an Verunreinigungen, umfassend insbesondere Verunreinigungen durch Edukte, Nebenprodukte, Luftsauerstoff, Wasser, sauerstoffhaltige Verbindungen, Halbmetalle, Metalle, insbesondere Platin(0), gegebenenfalls in Form von Platin(0)-Nanopartikeln und/oder Platin(0) enthaltenden Nanopartikeln, und Lösungsmittel, , von unter 1000 ppm, idealerweise von unter 100 ppm, auf.

Die Platin(IV)-Komplexe gemäß der Formel III und/oder IV sowie deren Lösungen oder Suspensionen in einem Lösungsmittel, welches insbesondere mit dem Lösungmittel S_{P} mischbar oder identisch ist, z. B. S_{L}, sind - insbesondere mittels des weiter oben beschriebenen Verfahrens zur Herstellung solcher Platin(IV)-Komplexe, Lösungen und Suspensionen - vorteilhafterweise auf einfache, reproduzierbare und vergleichsweise kostengünstige Weise in hoher Reinheit von 97 %, vorteilhaft von mehr als 97 %, insbesondere von mehr als 98 % oder 99 %, und guter Ausbeute von üblicherweise ≥ 60 %, vorteilhaft ≥ 70 %, erhältlich sowie in guter Raum-Zeit-Ausbeute. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, z. B. von Lösungsmitteln, mittels gaschromatographischer Verfahren (GC), ggf. mit Massenspektrometrie-Kopplung (GC-MS), bestimmt werden kann.

Definitionen der Begriffe "Platin(IV)-Komplex in hoher Reinheit" und "Raum-Zeit-Ausbeute" sind bereits weiter oben gegeben.

Nach alledem eignen sich die hier beanspruchten Platin(IV)-Komplexe gemäß den allgemeinen Formeln III und IV sowie Lösungen oder Suspensionen, umfassend wenigstens einen solchen Platin(IV)-Komplex, als qualitativ hochwertige Edukte für weitere Umsetzungen und/oder Anwendungen, auch im industriellen Maßstab.

Zudem wird die Aufgabe gelöst durch
▪ einen Platin(IV)-Komplex gemäß Formel 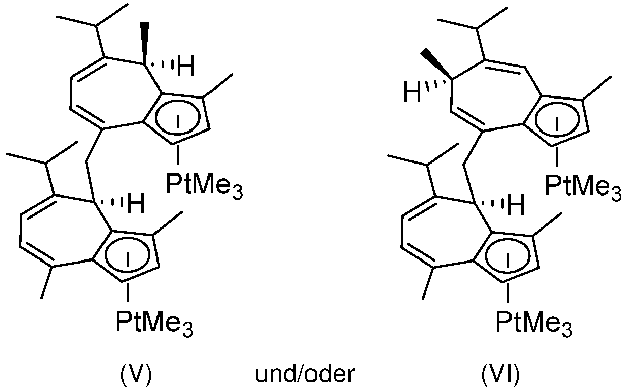 oder
▪ eine Lösung oder eine Suspension, umfassend einen Platin(IV)-Komplex gemäß Formel V und/oder Formel VI und ein Lösungsmittel S_{D}, welches ein aprotisch-unpolares oder ein aprotisch-polares Lösungsmittel umfasst oder ist.

Bei den Platin(IV)-Komplexen gemäß Formel V und Formel VI handelt es sich jeweils um das Produkt einer photoinduzierten, insbesondere tageslichtinduzierten, Dimerisierung. Die Verbindungen gemäß Formel V und Formel VI weisen jeweils zwei 4,8-verknüpfte Organo-dihydroguajazulenyl-Radikalanionen auf. Dabei trägt jeweils das über sein C4-Atom an der Verknüpfung beteiligte Radikalanion in 8-Position oder in 6-Position des Guajazulengerüsts zusätzlich zu einem H-Atom einen Organylrest R, insbesondere einen Methylrest. Die Formeln V und VI umfassen jeweils auch Lösungsmitteladdukte.

Gemäß einer Ausführungsform der hier beanspruchten Platin(IV)-Komplexe gemäß Formel V und Formel VI oder Lösungen oder Suspensionen, umfassend wenigstens einen solchen Platin(IV)-Komplex, umfasst das Lösungsmittel S_{D} wenigstens ein Lösungsmittel, welches ausgewählt ist aus der Gruppe bestehend aus aprotisch-polaren Lösungsmitteln, aliphatischen Kohlenwasserstoffen, aromatischen Kohlenwasserstoffen, siliciumorganischen Verbindungen, und Mischungen davon. Vorteilhaft ist das Lösungsmittel S_{D} ausgewählt aus der Gruppe bestehend aus aprotisch-polaren Lösungsmitteln, aliphatischen Kohlenwasserstoffen, insbesondere mit 1 bis 30 Kohlenstoffatomen, aromatischen Kohlenwasserstoffen, siliciumorganischen Verbindungen, und Mischungen davon.

Das aprotisch-polare Lösungsmittel ist beispielsweise ein Ether oder umfasst wenigstens einen Ether. Der Ether kann ausgewählt sein aus der Gruppe bestehend aus Tetrahydrofuran, Methyltetrahydrofuran, 1,4-Dioxan, 1,2-Dimethoxyethan, Diethylether, Methyl-*tert*-butylether, Di-*n*-propylether, Di*iso*propylether, Cyclopentylmethylether, und deren Isomeren, und Mischungen davon.

Eine Definition des Begriffes "aliphatische Kohlenwasserstoffe" ist bereits weiter oben gegeben. Der aliphatische Kohlenwasserstoff kann auch 2, 3, 4, 5, 6, 7, 8, 9, 10, 11, 12, 13, 14, 15, 16, 17, 18, 19, 20, 21, 22, 23, 24, 25, 26, 27, 28 oder 29 Kohlenstoffatome aufweisen. Vorteilhaft weist der aliphatische Kohlenwasserstoff 1 bis 20 Kohlenstoffatome auf, vorteilhafter 1 bis 18 Kohlenstoffatome, insbesondere 1 bis 16 Kohlenstoffatome.

Mit aromatischen Kohlenwasserstoffen sind insbesondere Benzol und seine Derivate, z. B. Toluol und Xylol, gemeint.

Gemäß einer weiteren alternativen oder ergänzenden Ausführungsform der hier beanspruchten Platin(IV)-Komplexe oder Lösungen oder Suspensionen, umfassend wenigstens einen solchen Platin(IV)-Komplex, ist die siliciumorganische Verbindung ein Silikon. Eine Definition des Begriffes "Silikon" ist bereits weiter oben angegeben. Zudem sind dort in nicht einschränkender Weise Beispiele für Silikone aufgeführt.

Ein Beispiel für ein aprotisch-polares Lösungsmittel ist Dimethylsulfoxid (DMSO).

Gemäß einer anderen Ausführungsvariante sind die in einem Lösungsmittelgemisch S_{D} enthaltenen Solventien miteinander mischbar. Der Ausdruck "mischbar" ist bereits weiter oben definiert.

Eine weitere vorteilhafte Ausführungsform sieht vor, dass das Lösungsmittel S_{D} mit dem weiter oben definierten Lösungsmittel S_{P} mischbar oder identisch ist.

Die Dimerisierungsreaktion erfolgt in Lösung, insbesondere in einem der oben genannten aprotisch-unpolaren Lösungsmittel S_{D} oder in einem aprotisch-polaren Lösungsmittel S_{D} wie beispielsweise DMSO, infolge einer lichtinduzierten Methaneliminierung gemäß der folgenden Bruttogleichung:

2 [PtMe₃(GuaMe)] - *hv →* [Me₃Pt(Gua--Gua)PtMe₃] + CH₄ ↑

Das Homodimer gemäß Formel V ist ausgehend von einer Lösung eines Diastereomerengemisches, bestehend aus den beiden Diastereomeren PtMe₃(Gua-8-exo-Me)] (III.D1.1) und [PtMe₃(Gua-8-*endo*-Me)] (III.D2.1) erhältlich. Der Platin(IV)-Komplex gemäß Formel V wurde mittels massenspektrometrischer Untersuchung identifiziert. Das Heterodimer gemäß Formel VI ist ausgehend von einer Lösung eines Diastereomerengemisches, bestehend aus den vier Diastereomeren [PtMe₃(Gua-8-*exo*-Me)] (III.D1.1), [PtMe₃(Gua-8-*endo*-Me)] (III.D2.1), [PtMe₃(Gua-6-*endo-Me*)] (IV.D3.1), [PtMe₃(Gua-6-*exo*-Me)] (IV.D4.1), erhältlich. Der Platin(IV)-Komplex gemäß Formel VI wurde mittels Röntgenstrukturanalyse und massenspektrometischer Untersuchung identifiziert.

Als Lösungsmittel ist in beiden Fällen eines der oben genannten aprotisch-unpolaren Lösungsmittel S_{D} oder ein aprotisch-polares Lösungsmittel S_{D} einsetzbar, beispielsweise DMSO. Die Reaktionszeit beträgt - in Abhängigkeit von den Reaktionsbedingungen, insbesondere der Konzentration des jeweiligen Diastereomerengemisches in dem gewählten Lösungsmittel und der Intensität des zur Bestrahlung genutzten sichtbaren Lichtes, 1 Stunde bis 72 Stunden.

Die Aufgabe wird auch gelöst durch die Verwendung
▪ wenigstens eines Platin(IV)-Komplexes gemäß der allgemeinen Formel 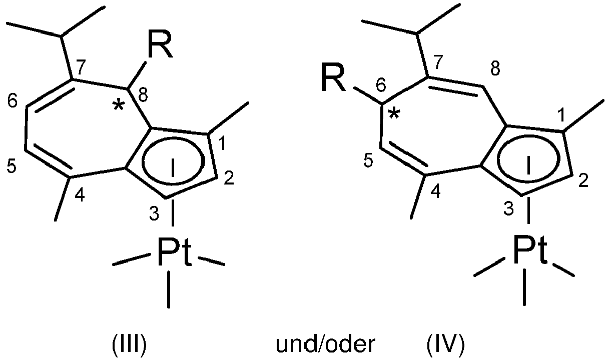 und/oder
   gemäß Formel 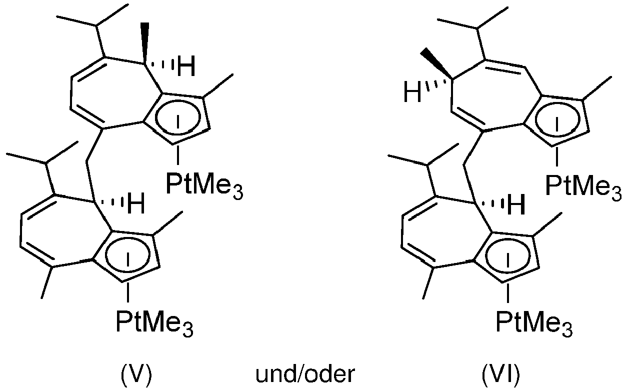 oder
▪ wenigstens einer Lösung oder Suspension, umfassend einen Platin(IV)-Komplex gemäß der allgemeinen Formel 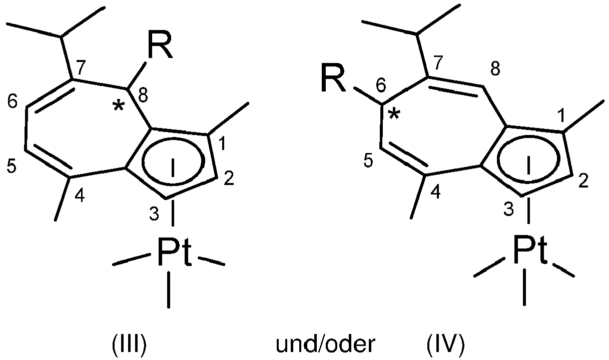 und ein Lösungsmittel, welches insbesondere mit dem Lösungmittel S_{P} mischbar oder identisch ist,
   und/oder
   umfassend einen Platin(IV)-Komplex gemäß Formel 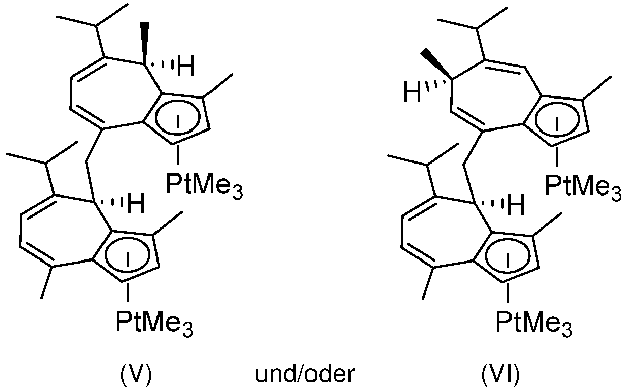 und ein Lösungsmittel S_{D}, welches ein aprotisch-unpolares oder ein aprotisch-polares Lösungsmittel umfasst oder ist, insbesondere mit dem Lösungmittel S_{P} mischbar oder identisch ist,
   wobei
   R jeweils ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
   - gemäß einer Ausführungsform der weiter oben beschriebenen Platin(IV)-Komplexe
      oder
   - gemäß einer Ausführungsform der weiter oben beschriebenen Lösungen oder Suspensionen, umfassend einen solchen Platin(IV)-Komplex,
      oder
   - erhalten oder erhältlich nach einem Verfahren zur Herstellung solcher Platin(IV)-Komplexe oder Lösungen oder Suspensionen gemäß einer der weiter oben beschriebenen Ausführungsformen,
      als
      i. Präkatalysator oder Präkatalysator enthaltende Lösung oder Suspension in einer chemischen Reaktion
         und/oder
      ii. Katalysator oder Katalysator enthaltende Lösung oder Suspension in einer chemischen Reaktion
         und/oder
      iii. Präkursorverbindung oder Präkursorverbindung enthaltende Lösung oder Suspension zur Herstellung wenigstens einer Schicht bestehend aus Platin oder wenigstens einer Schicht, welche Platin enthält, auf wenigstens einer Oberfläche eines Substrats.
      Zum einen handelt es sich bei der vorgenannten Verwendung um ein Verfahren zur Durchführung einer chemischen Reaktion unter Verwendung
▪ wenigstens eines Platin(IV)-Komplexes gemäß der allgemeinen Formel 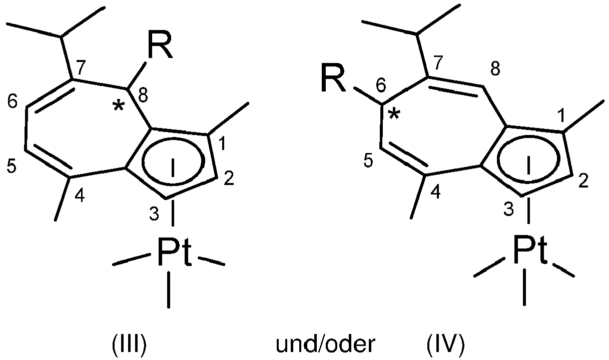 und/oder
   gemäß Formel 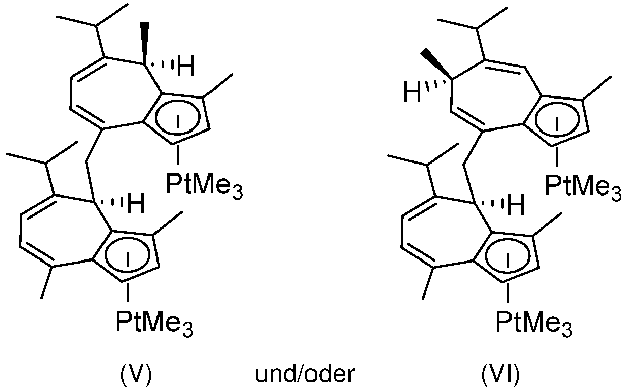 oder
▪ wenigstens einer Lösung oder Suspension, umfassend einen Platin(IV)-Komplex gemäß der allgemeinen Formel 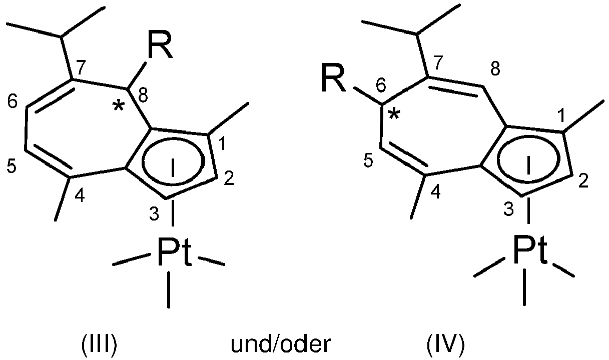 und ein Lösungsmittel, welches insbesondere mit dem Lösungmittel S_{P} mischbar oder identisch ist,
   und/oder
   gemäß Formel 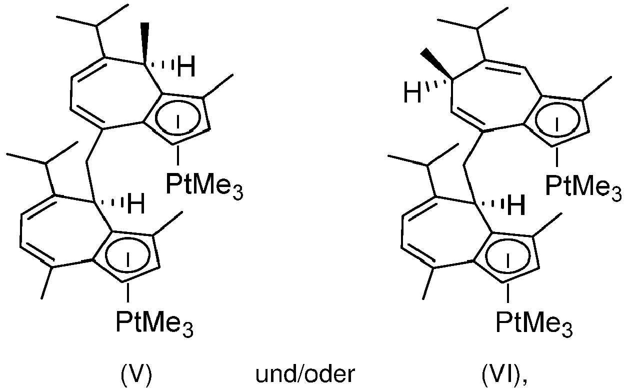 und ein Lösungsmittel S_{D}, welches ein aprotisch-unpolares oder ein aprotisch-polares Lösungsmittel umfasst oder ist, insbesondere mit dem Lösungmittel S_{P} mischbar oder identisch ist,
   wobei
   R jeweils ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
   - gemäß einer Ausführungsform der weiter oben beschriebenen Platin(IV)-Komplexe
      oder
   - gemäß einer Ausführungsform der weiter oben beschriebenen Lösungen oder Suspensionen, umfassend einen solchen Platin(IV)-Komplex,
      oder
   - erhalten oder erhältlich nach einem Verfahren zur Herstellung solcher Platin(IV)-Komplexe oder Lösungen oder Suspensionen gemäß einer der weiter oben beschriebenen Ausführungsformen,
umfassend die Schritte:
A) Zurverfügungstellung des wenigstens einen Platin(IV)-Komplexes gemäß der allgemeinen Formel III und/oder IV und/oder gemäß Formel V und/oder VI
   oder
   der wenigstens einen Lösung oder Suspension, umfassend einen Platin(IV)-Komplex gemäß der allgemeinen Formel III und/oder IV und ein Lösungsmittel, welches insbesondere mit dem Lösungmittel S_{P} mischbar oder identisch ist, und/oder umfassend einen Platin(IV)-Komplex gemäß Formel V und/oder VI und ein Lösungsmittel S_{D}, welches ein aprotisch-unpolares oder ein aprotisch-polares Lösungsmittel umfasst oder ist, insbesondere mit dem Lösungmittel S_{P} mischbar oder identisch ist,
   und
B) Durchführung der chemischen Reaktion unter Verwendung des wenigstens einen Platin(IV)-Komplexes gemäß der allgemeinen Formel III und/oder IV und/oder gemäß Formel V und/oder VI als Präkatalysator und/oder als Katalysator.

Dabei umfassen die allgemeinen Formeln III und IV jeweils sowohl die Monomere als auch etwaige Oligomere, insbesondere Dimere, und Lösungsmitteladdukte. Die Formeln V und VI umfassen jeweils auch Lösungsmitteladdutke.

Zudem liegen die hier zu verwendenden Verbindungen gemäß den allgemeinen Formeln III und IV insbesondere jeweils als ein Diastereomerengemisch vor. Mit anderen Worten: Im Falle des Platin(IV)-Komplexes gemäß Formel III stellt das Kohlenstoffatom C8 ein Stereozentrum dar, im Falle des Platin(IV)-Komplexes gemäß Formel IV stellt das Kohlenstoffatom C6 ein Stereozentrum dar. Alternativ kann - insbesondere in Abhängigkeit vom gewähltem Substituent R - ein Diastereomerengemisch vorliegen, welches sowohl das Diastereomerengemisch der Verbindung gemäß Formel III als auch das Diastereomerengemisch der Verbindung gemäß Formel IV umfasst, insbesondere daraus besteht. In jedem der vorgenannten Fälle liegt jedes Diastereomer als ein Enantiomerengemisch vor, wobei die Diastereomere unabhängig voneinander jeweils als ein Racemat vorliegen können.

Die Platin(IV)-Komplexe gemäß den allgemeinen Formeln III, IV, V und VI werden üblicherweise solvensfrei, d. h. nicht als Solvensaddukte, erhalten bzw. liegen in der Regel solvensfrei vor. Mittels der weiter oben beschriebenen Verfahren zur Herstellung solcher Platin(IV)-Komplexe, insbesondere gemäß Formel III und Formel IV, können aber auch Lösungsmitteladdukte dieser Metallkomplexe erhalten werden. Im Falle eines solchen Adduktes ist das Lösungsmittel insbesondere mit dem - im Rahmen des weiter oben beschriebenen Verfahrens verwendeten - Lösungsmittel S_{P} identisch, wenn es sich bei dem Lösungsmittel S_{P} um ein Alkoxyalkan handelt oder das Lösungsmittel S_{P} wenigstens ein Alkoxyalkan enthält.

Insbesondere mit Blick auf eine Verwendung als Platinpräkursor-Verbindungen in Gasphasenabscheidungsprozessen ist es vorteilhaft, wenn die Platin(IV)-Komplexe gemäß der allgemeinen Formel III und/oder IV und/oder V und/oder VI insbesondere Alkoxyalkan-frei vorliegen.

Als chemische Reaktionen, welche unter Verwendung des wenigstens einen Platin(IV)-Komplexes gemäß der allgemeinen Formel III und/oder gemäß der allgemeinen Formel IV und/oder gemäß der allgemeinen Formel IV und/oder gemäß der allgemeinen Formel V als Präkatalysator und/oder als Katalysator durchgeführt werden können, seien hier in nicht einschränkender Weise beispielhaft lichtinduzierte Platin-katalysierte Hydrosilylierungsreaktionen und die Hydrierung ungesättigter Verbindungen und Polymerisationsreaktionen, bei denen die Katalysatoraktivierung durch ultraviolette oder sichtbare Strahlung erfolgt, genannt.

Zum anderen handelt es sich bei der vorgenannten Verwendung um ein Verfahren zur Herstellung
i. wenigstens einer Schicht bestehend aus Platin
   oder
ii. wenigstens einer Schicht, welche Platin enthält,
   auf wenigstens einer Oberfläche eines Substrats unter Verwendung
   ▪ wenigstens eines Platin(IV)-Komplexes gemäß der allgemeinen Formel 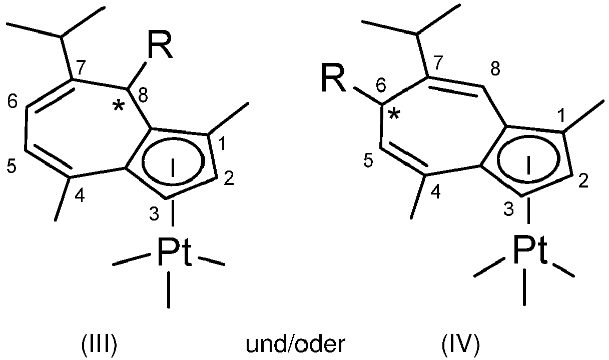 oder
   ▪ wenigstens einer Lösung oder Suspension, umfassend einen Platin(IV)-Komplex gemäß der allgemeinen Formel 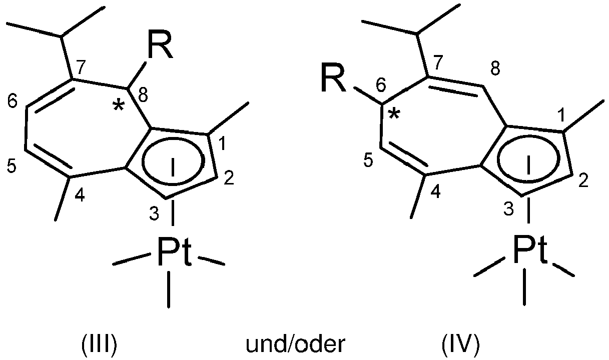 und ein Lösungsmittel, welches insbesondere mit dem Lösungmittel S_{P} mischbar oder identisch ist,
      wobei
      R jeweils ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
      - gemäß einer Ausführungsform der weiter oben beschriebenen Platin(IV)-Komplexe
         oder
      - gemäß einer Ausführungsform der weiter oben beschriebenen Lösungen oder Suspensionen, umfassend einen solchen Platin(IV)-Komplex,
         oder
      - erhalten oder erhältlich nach einem Verfahren zur Herstellung solcher Platin(IV)-Komplexe oder solcher Lösungen oder Suspensionen gemäß einer der weiter oben beschriebenen Ausführungsformen,
umfassend die Schritte:
A) Zurverfügungstellung
   des wenigstens einen Platin(IV)-Komplexes gemäß der allgemeinen Formel III und/oder IV
   oder
   der wenigstens einen Lösung oder Suspension, umfassend einen Platin(IV)-Komplex gemäß der allgemeinen Formel III und/oder IV und ein Lösungsmittel, welches insbesondere mit dem Lösungmittel S_{P} mischbar oder identisch ist,
   und
B) Abscheidung
   i. der wenigstens einen Schicht bestehend aus Platin
      oder
   ii. der wenigstens einen Schicht, welche Platin enthält,
      auf der wenigstens einen Oberfläche des Substrats unter Verwendung des wenigstens einen Platin(IV)-Komplexes gemäß der allgemeinen Formel III und/oder IV als Präkursorverbindung.

Bei den hier beschriebenen Verwendungen bzw. den hier beanspruchten Verfahren zur Durchführung einer chemischen Reaktion oder zur Herstellung wenigstens einer Platinschicht oder wenigstens einer Schicht, welche Platin enthält, können die Platin(IV)-Komplexe gemäß einer Ausführungsform der weiter oben beschriebenen Platin(IV)-Komplexe als Feststoffe, Flüssigkeiten, Lösungen oder Suspensionen eingesetzt werden. Alternativ können die Platin(IV)-Komplexe oder Lösungen oder Suspensionen, umfassend einen Platin(IV)-Komplex und wenigstens ein Lösungsmittel, welches insbesondere mit dem Lösungmittel S_{P} mischbar oder identisch ist, z. B. S_{L}, jeweils erhalten oder erhältlich nach einem Verfahren zur Herstellung solcher Platin(IV)-Komplexe bzw. Lösungen oder Suspensionen gemäß einer der weiter oben beschriebenen Ausführungsformen als Feststoff, Flüssigkeit, Lösung oder Suspension eingesetzt werden. Aufgrund ihrer hohen Reinheit eignen sich die vorgenannten Pt(IV)-Komplex-Verbindungen gemäß den Formeln III, IV, V und VI, insbesondere gemäß den Formeln III und IV, sowohl für den Einsatz als Präkatalysatoren und/oder als Katalysatoren in einer Vielzahl von Reaktionen, welche durch Platin katalysiert werden, als auch als Platinpräkursor-Verbindungen in Gasphasenabscheidungsprozessen, insbesondere Niedrigtemperatur-Gasphasenabscheidungsverfahren. Einige dieser Platin(IV)-Komplexe liegen vorteilhaft nicht nur in hoher Reinheit, sondern auch bei Raumtemperatur flüssig vor.

Eine Definition des Ausdrucks "Platin(IV)-Komplex in hoher Reinheit" ist weiter oben angegeben.

Die Platin(IV)-Komplexe gemäß den allgemeinen Formeln III und IV weisen jeweils ein Organo-dihydroguajazulenyl-Anion bzw. R-Dihydroguajazulenyl-Anion (GuaR)¹-auf, welches in 8-Position oder in 6-Position des Guajazulengerüsts zusätzlich zu einem H-Atom einen Organylrest R trägt. Bei dem R-Dihydroguajazulenyl-Anion (GuaR)¹- kann es sich also um ein 7-*iso*-propyl-1,4-dimethyl-8-R-dihydroazulenyl-Anion bzw. 8-R-Dihydroguajazulenyl-Anion (Gua-8-R)¹⁻ gemäß Formel III oder um ein 7-iso-propyl-1,4-dimethyl-6-R-dihydroazulenyl-Anion bzw. 6-R-Dihydroguajazulenyl-Anion (Gua-6-R)¹⁻ gemäß Formel IV handeln. Anders ausgedrückt: In C8-Position oder in C6-Position des Guajazulengerüsts liegt eine RCH-Gruppe vor. Die Platin(IV)-Komplexe gemäß Formel V und Formel VI weisen jeweils zwei 4,8-verknüpfte Organo-dihydroguajazulenyl-Radikalanionen auf. Dabei trägt jeweils das über sein C4-Atom an der Verknüpfung beteiligte Radikalanion in 8-Position oder in 6-Position des Guajazulengerüsts zusätzlich zu einem H-Atom einen Organylrest R, insbesondere einen Methylrest. Infolge der Addition eines Organyl-Anions (R)¹⁻ wird die Aromatizität auf den Fünfring beschränkt, wobei in der Regel die für Azulen und dessen Derivate typische blaue Farbe verloren geht. Das Organo-dihydroguajazulenyl-Anion (GuaR)¹⁻ stellt ein Derivat des Cyclopentadienyl-Anions bzw. ein Cyclopentadienyl-artiges Monoanion dar.

Aufgrund des Vorhandenseins eines R-Dihydroguajazulenyl-Anions, also eines Cyclopentadienyl-artigen Monoanions, eignen sich die Verbindungen gemäß den allgemeinen Formeln III und IV insbesondere als Präkatalysatoren und/oder Katalysatoren für chemische Reaktionen, in welchen ansonsten Platin(IV)-Komplexe eingesetzt werden, die Cyclopentadienyl-Liganden aufweisen. Dasselbe gilt für die Platin(IV)-Komplexe gemäß Formel V und Formel VI, welche jeweils zwei 4,8-verknüpfte Organo-dihydroguajazulenyl-Radikalanionen aufweisen. Besonders vorteilhaft ist, dass die Zurverfügungstellung eines R-Dihydroguajazulenyl-Liganden im Vergleich zur Bereitstellung des Cyclopentadienyl-Liganden weniger arbeits- und zeitaufwändig ist. Insbesondere können die hier als Edukte eingesetzten Verbindungen, welche Cyclopentadienyl-artige Liganden umfassen, unter Verwendung von kostengünstigen nachwachsenden Rohstoffen hergestellt werden. Denn Guajazulen ist partialsynthetisch zugänglich, nämlich ausgehend von dem Naturstoff Guajol und anderen Azulenbildnern durch einfache Dehydratisierung und Dehydrierung (T. Shono, N. Kise, T. Fujimoto, N. Tominaga, H. Morita, J. Org. Chem. 1992, 57, 26, 7175 - 7187; CH 314 487 A (B. Joos) 29.01.1953). Infolgedessen fallen auch der Syntheseaufwand und die Herstellungskosten für die hier beanspruchten Platin(IV)-Komplexe sowie Lösungen oder Suspensionen, umfassend wenigstens eine solche Pt(IV)-Verbindung und ein Lösungsmittel, welches insbesondere mit dem Lösungmittel S_{P} mischbar oder identisch ist, geringer aus als für analoge Platin(IV)-Cp-Komplexe. Mithin stellen die hier beschriebenen Platin(IV)-Komplexe sowie deren Lösungen und Suspensionen insbesondere mit Blick auf eine industrielle Anwendung eine Alternative für Platin(IV)-Cp-Komplexe dar.

Bei Guajazulen handelt es sich um einen Naturstoff, der in Kamillenöl und anderen ätherischen Ölen enthalten und daher vorteilhafterweise in großen Mengen kostengünstig zugänglich ist. Synthetisch kann es aus dem Guajol des Guajakholzöls (Guajakharz) hergestellt werden. Guajazulen ist eine intensiv blaue Substanz mit entzündungshemmender Wirkung.

Die Platin(IV)-Komplexe gemäß Formel III und/oder gemäß Formel IV und/oder gemäß Formel V und/oder gemäß Formel VI, insbesondere die Platin(IV)-Komplexe gemäß Formel III und/oder Formel IV, sowie deren Lösungen oder Suspensionen in einem Lösungsmittel, welches insbesondere mit dem Lösungmittel S_{P} mischbar oder identisch ist, z. B. S_{L}, sind - insbesondere mittels eines der weiter oben beschriebenen Verfahren zur Herstellung solcher Platin(IV)-Komplexe, Lösungen und Suspensionen - vorteilhafterweise auf einfache, reproduzierbare und vergleichsweise kostengünstige Weise in hoher Reinheit von 97 %, vorteilhaft von mehr als 97 %, insbesondere von mehr als 98 % oder 99 %, und guter Ausbeutevon üblicherweise ≥ 60 %, vorteilhaft ≥ 70 %, erhältlich sowie in guter Raum-Zeit-Ausbeute. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, wie z. B. von Lösungsmitteln, mittels gaschromatographischer Verfahren (GC), ggf. mit Massenspektrometrie-Kopplung (GC-MS), bestimmt werden kann.

Definitionen der Begriffe "Platin(IV)-Komplex in hoher Reinheit" und "Raum-Zeit-Ausbeute" sind bereits weiter oben gegeben.

Ein Beispiel für einen in den hier beanspruchten Verwendungen bzw. den hier beanspruchten Verfahren zur Durchführung einer chemischen Reaktion oder zur Herstellung wenigstens einer Schicht bestehend aus Platin oder wenigstens einer Platin enthaltenden Schicht verwendeten Platin(IV)-Komplex ist der Halb*sandwich-*Komplex [PtMe₃(GuaMe)]. Dieser liegt - wie bereits weiter oben dargelegt - in isolierter Form vorteilhafterweise als bei Raumtemperatur flüssiges Diastereomerengemisch vor, bestehend aus zwei oder vier diastereomeren Enantiomerenpaaren. Dabei kann jedes der Diastereomere optional als Racemat vorliegen.

Aufgrund der Tatsache, dass der isolierte Platin(IV)-Komplex [PtMe₃(GuaMe)] in Form eines Öls vorliegt, ist er - im Vergleich zu vorbekannten, Cyclopentadienyl-Anionen enthaltenden und häufig wachsartig vorliegenden Präkatalysatoren wie [PtMe₃(MeCp)] - einfacher handhabbar. Als bei Raumtemperatur flüssig vorliegende Platin(IV)-Verbindung eignet sich [PtMe₃(GuaMe)] außerdem besonders als Platinpräkursor-Verbindung für Gasphasenabscheidungsprozesse, insbesondere Niedrigtemperatur-Gasphasenabscheidungsverfahren.

Die Platin(IV)-Verbindung [PtMe₃(GuaMe)] zeigt zudem eine Absorption im Vis-Bereich. Das stellt im Rahmen lichtinduzierter Platin-katalysierter Reaktionen, beispielsweise Hydrosilylierungsreaktionen, einen weiteren Vorteil dar. Denn dabei ist regelmäßig die Verwendung von UV/Vis-Licht vorgesehen, wodurch üblicherweise besondere Sicherheitsmaßnahmen erforderlich sind, um das Hautkrebsrisiko zu reduzieren. Solche Sicherheitsmaßnahmen sind bei Verwendung von [PtMe₃(GuaMe)] nicht zwingend notwendig.

Weiterhin vorteilhaft ist, dass die monomere Verbindung [PtMe₃(GuaMe)] unter Lichtausschluss für mehrere Monate bei Raumtemperatur lagerbar ist. Währenddessen werden weder Zersetzungsreaktionen noch eine Oligomerisierung oder Polymerisierung beobachtet. Vorteilhaft ist zudem, dass der Komplex [PtMe₃(GuaMe)] eine relativ hohe thermische Stabilität aufweist, also auch bei erhöhten Reaktions- oder Abscheidungstemperaturen verwendbar ist. Laut thermogravimetrischer Analyse (TGA) erfolgt ein 3%-Abbau bei einer Temperatur von 180 °C.

In Schritt A) der hier beschriebenen Verfahren zur Durchführung einer chemischen Reaktion oder zur Herstellung wenigstens einer Schicht bestehend aus Platin oder wenigstens einer Schicht, welche Platin enthält, kann jeweils die Zurverfügungstellung eines Platin(IV)-Komplexes oder mehrerer Platin(IV)-Komplexe vorgesehen sein. In einer Ausführungsvariante des jeweiligen Verfahrens ist vorgesehen, dass in Schritt A) wenigstens ein Platin(IV)-Komplex als Feststoff, als Flüssigkeit oder als Lösung oder Suspension, umfassend einen Platin(IV)-Komplex, zur Verfügung gestellt wird. Alternativ oder ergänzend können mehrere Platin(IV)-Komplexe unabhängig voneinander als separate Feststoffe oder als Feststoffgemische oder als separate Flüssgkeiten oder als Flüssigkeitsgemische oder als separate Lösungen oder Suspensionen, umfassend jeweils einen Platin(IV)-Komplex, oder als Lösung oder Suspensionen, umfassend mehrere Platin(IV)-Komplexe, zur Verfügung gestellt werden.

An dieser Stelle sowie im Folgenden wird auf die Angabe einer exakten Stöchiometrie der abscheidbaren Platin enthaltenden Schichten oder Filme verzichtet. Der Begriff Schicht ist gleichbedeutend mit dem Ausdruck Film und trifft keine Aussage über die Schichtdicke oder die Filmdicke. Zudem kann gemäß der vorliegenden Erfindung eine Platinschicht oder eine Schicht, welche Platin enthält, Nanopartikel eines Metalls Q, insbesondere Platin-Nanopartikel, oder Nanopartikel unterschiedlicher Metalle Q oder Nanopartikel, die jeweils mehrere Metalle Q umfassen, enthalten oder aus solchen Nanopartikeln bestehen.

Die verwendeten Platin(IV)-Komplexe sind aufgrund ihrer hohen Reinheit besonders gut als Präkursorverbindungen zur Herstellung qualitativ hochwertiger Platinschichten oder Platin enthaltender Schichten auf einer Oberfläche eines Substrats geeignet. Das ist insbesondere zurückzuführen auf ihre Herstellung nach einem Verfahren gemäß einer der vorhergehend beschriebenen Ausführungsformen, nämlich unter Verwendung von Alkalimetall-R-dihydroguajazuleniden gemäß Formel I und/oder Formel II. Zudem sind die gemäß Schritt A) zur Verfügung zu stellenden Platin(IV)-Komplexe bzw. Lösungen oder Suspensionen, umfassend solche Komplexe, nach einem Verfahren zur Herstellung solcher Verbindungen oder Lösungen oder Suspensionen gemäß einer der vorbeschriebenen Ausführungsformen relativ einfach, reproduzierbar sowievergleichsweise nachhaltig und kostengünstig darstellbar. Letzteres ermöglicht ihre Verwendung im großtechnischen Maßstab.

In einer Ausführungsvariante der hier beanspruchten Verwendungen bzw. der hier beanspruchten Verfahren zur Durchführung einer chemischen Reaktion oder zur Herstellung wenigstens einer Schicht, welche aus Platin besteht oder Platin enthält, ist R ausgewählt aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten. Als Rest R können also auch primäre, sekundäre, tertiäre Alkyl-, Alkenyl- und Alkinylreste mit 2, 3, 4, 5, 6, 7, 8 oder 9 Kohlenstoffatomen vorgesehen sein sowie cyclische Alkylreste mit 4, 5, 6, 7, 8 oder 9 Kohlenstoffatomen. Gemäß einer anderen Ausführungsform der beanspruchten Verwendungen bzw. der hier beanspruchten Verfahren ist R ausgewählt aus der Gruppe bestehend aus primären, sekundären, tertiären Alkyl-, Alkenyl- und Alkinylresten mit 1 bis 6 Kohlenstoffatomen, cyclischen Alkylresten mit 3 bis 6 Kohlenstoffatomen, einem Benzylrest, einkernigen Arylresten, mehrkernigen Arylresten, einkernigen Heteroarylresten und mehrkernigen Heteroarylresten. Als Rest R können also auch primäre, sekundäre, tertiäre Alkyl-, Alkenyl- und Alkinylreste mit 2, 3, 4 oder 5 Kohlenstoffatomen vorgesehen sein sowie cyclische Alkylreste mit 4 oder 5 Kohlenstoffatomen. In einer noch anderen Variante der beanspruchten Verwendungen bzw. der hier beanspruchten Verfahren ist R ausgewählt aus der Gruppe bestehend aus Me, Et, n-Pr, *i*-Pr, n-Bu, *i*-Bu, s-Bu, *t*-Bu, n-Pentyl, 2-Pentyl, 3-Pentyl, 2-Methylbutyl, 3-Methylbutyl, 3-Methylbut-2-yl, 2-Methylbut-2-yl, 2,2-Dimethylpropyl, Cyclopentyl, Cyclohexyl, Phenyl, Tolyl, Benzyl und Cumyl, und deren Isomeren. Gemäß einer anderen vorteilhaften Ausführungsform ist R ausgewählt aus der Gruppe bestehend aus Me, Et, n-Pr, *i*-Pr, n-Bu, *i*-Bu, s-Bu, *t*-Bu, Cyclohexyl, Phenyl, Tolyl, Benzyl und Cumyl, und deren Isomeren, besonders vorteilhaft ist R ausgewählt aus der Gruppe bestehend aus Me, Et, n-Pr, *i*-Pr, n-Bu, i-Bu, s-Bu, *t*-Bu, insbesondere ist R = Me.

In einer weiteren Ausführungsform der hier beschriebenen Verwendungen bzw. der hier beanspruchten Verfahren zur Durchführung einer chemischen Reaktion oder zur Herstellung wenigstens einer Schicht, welche aus Platin besteht oder Platin enthält, ist der Platin(IV)-Komplex ausgewählt aus der Gruppe bestehend aus [PtMe₃(GuaMe)], [PtMe₃(GuaEt)], [PtMe₃(Gua*n*Pr)], [PtMe₃(Gua/Pr)], [PtMe₃(Gua*n*Bu)], [PtMe₃(Gua/Bu)], [PtMe₃(Gua*t*Bu)], [PtMe₃(Gua*s*Bu)] und [PtMe₃(GuaPh)]. Insbesondere ist der Platin(IV)-Komplex [PtMe₃(GuaMe)]. Der Halb*sandwich*-Komplex [PtMe₃(GuaMe)] liegt - wie bereits weiter oben dargelegt - in isolierter Form vorteilhafterweise als bei Raumtemperatur flüssiges Diastereomerengemisch vor, bestehend aus zwei oder vier diastereomeren Enantiomerenpaaren, wobei jedes der Diastereomere optional als Racemat vorliegt. In einer anderen Ausführungsform des hier beanspruchten Verfahrens zur Herstellung einer Platinschicht oder einer Platin enthaltenden Schicht erfolgt die Abscheidung der Platinschicht oder der Platin enthaltenden Schicht in Schritt B) mittels eines Gasphasenabscheidungsverfahrens, insbesondere eines Niedrigtemperatur-Gasphasenabscheidungsverfahrens. Vorteilhaft erfolgt die Abscheidung der Platinschicht oder der Platin enthaltenden Schicht mittels eines *ALD*-Verfahrens oder eines MOCVD-Verfahrens, insbesondere mittels eines MOVPE-Verfahrens. Alternativ kann ein Sol-Gel-Verfahren angewendet werden, wobei das Sol beispielsweise mittels Spin- oder Dipcoating auf einer Oberfläche oder mehreren Oberflächen des Substrats abgeschieden werden kann.

Eine weitere Variante der hier beschriebenen Verwendung bzw. des beanspruchten Verfahrens sieht eine sequenzielle Abscheidung mehrerer Platinschichten und/oder Platin enthaltender Schichten auf der Oberfläche des Substrats vor. Dabei erfolgt eine Wiederholung von Schritt B), wobei die jeweiligen Platin enthaltenden Schichten und/oder Platinschichten nacheinander abgeschieden werden. Die jeweils erste Schicht wird unmittelbar auf der Oberfläche des Substrats abgeschieden, während die weiteren Schichten auf der Oberfläche der jeweils zuvor abgeschiedenen Schicht abgeschieden werden.

Das Substrat kann beispielsweise ein Nichtmetall oder mehrere Nichtedelmetalle umfassen oder aus einem Nichtmetall oder mehreren Nichtedelmetallen gefertigt sein. Alternativ oder ergänzend kann das Substrat ein nichtmetallisches Material oder mehrere nichtmetallische Materialien umfassen oder vollständig aus einem nichtmetallischen Material oder mehreren solcher Materialien bestehen. Als Substrat können z. B. Korundfolien oder dünne metallische Folien eingesetzt werden. Das Substrat kann selbst Teil eines Bauteils sein. In einer Ausführungsform der vorgenannten Verwendung eines Platin(IV)-Komplexes als Präkursorverbindung zur Herstellung einer Platinschicht oder einer Platin enthaltenden Schicht bzw. in einer Ausführungsform des Verfahrens zur Herstellung einer Platinschicht oder einer Platin enthaltenden Schicht auf einer Oberfläche eines Substrats ist das Substrat ein Wafer. Der Wafer kann Silicium, Siliciumcarbid, Germanium, Galliumarsenid, Indiumphosphid, ein Glas, wie z. B. SiO₂, und/oder einen Kunststoff, wie z. B. Silikon, umfassen oder vollständig aus einem oder mehreren dieser Materialien bestehen. Zudem kann der Wafer eine Waferschicht oder mehrere Waferschichten mit jeweils einer Oberfläche aufweisen. Die Herstellung einer Platinschicht oder einer Platin enthaltenden Schicht kann auf der Oberfläche einer Waferschicht oder mehrerer Waferschichten vorgesehen sein.

Mittels der hier beanspruchten Verwendung bzw. des hier beschriebenen Verfahrens erhaltene oder erhältliche Substrate, umfassend eine Platinschicht oder eine Platin enthaltende Schicht, ggf. umfassend Metallnanopartikel oder bestehend aus Metallnanopartikeln, sind aufgrund der hohen Reinheit der Platinschicht oder der Platin enthaltenden Schicht besonders gut für die Herstellung eines elektronischen Bauelements, insbesondere eines elektronischen Halbleiterbauelements, oder einer redoxaktiven Elektrode für eine Brennstoffzelle einsetzbar. In letzterem Fall fungiert die Platinschicht oder die Platin enthaltende Schicht als katalytische Schicht.

Die Aufgabe wird außerdem gelöst durch ein Substrat, welches auf wenigstens einer Oberfläche
i. wenigstens eine Schicht bestehend aus Platin,
   oder
ii. wenigstens eine Schicht, welche Platin enthält,
   aufweist,
   wobei die wenigstens eine Schicht bestehend aus Platin oder die wenigstens eine Schicht, welche Platin enthält, herstellbar oder hergestellt ist unter Verwendung eines Platin(IV)-Komplexes gemäß der allgemeinen Formel III und/oder IV
   oder
   einer Lösung oder Suspension, umfassend einen solchen Platin(IV)-Komplex und ein Lösungsmittel, welches insbesondere mit dem Lösungmittel S_{P} mischbar oder identisch ist,
   - gemäß einer Ausführungsform der weiter oben beschriebenen Platin(IV)-Komplexe
      oder
   - gemäß einer Ausführungsform der weiter oben beschriebenen Lösungen oder Suspensionen, umfassend einen solchen Platin(IV)-Komplex,
      oder
   - erhalten oder erhältlich nach einem Verfahren zur Herstellung solcher Platin(IV)-Komplexe oder solcher Lösungen oder Suspensionen, umfassend einen solchen Platin(IV)-Komplex, gemäß einer der weiter oben beschriebenen Ausführungsformen.

Hinsichtlich einer Auswahl verwendbarer Platin(IV)-Komplexe gemäß der allgemeinen Formel III und/oder der allgemeinen Formel IV sowie von Lösungen oder Suspensionen, umfassend einen solchen Platin(IV)-Komplex und ein Lösungsmittel, welches insbesondere mit dem Lösungmittel S_{P} mischbar oder identisch ist, wird auf die Ausführungen betreffend das weiter oben beschriebene Verfahren zur Herstellung eines solchen Substrats verwiesen.

Eine Definition des Begriffs "mischbar" ist bereits weiter oben gegeben.

Hinsichtlich der Vorteile eines solchen Substrats wird auf die Vorteile verwiesen, welche für das weiter oben beschriebene Verfahren zur Herstellung eines solchen Substrats genannt sind.

Das hier beschriebene Substrat ist insbesondere mittels des weiter oben beschriebenen Verfahrens zur Herstellung wenigstens einer Schicht bestehend aus Platin oder wenigstens einer Schicht, welche Platin enthält, auf wenigstens einer Oberfläche eines Substrats erhältlich.

Weiterhin wird die Aufgabe gelöst durch eine vernetzbare Silikonzusammensetzung, umfassend
i. wenigstens eine Verbindung ausgewählt aus der Gruppe bestehend aus Verbindungen vom Typ (a), Verbindungen vom Typ (b) und Verbindungen vom Typ (c), wobei
   - Verbindungen vom Typ (a) organische Verbindungen und siliciumorganische Verbindungen sind, umfassend jeweils wenigstens zwei Reste mit aliphatischen Kohlenstoff-Kohlenstoff-Mehrfachbindungen,
   - Verbindungen vom Typ (b) siliciumorganische Verbindungen sind, umfassend jeweils wenigstens zwei Si-gebundene Wasserstoffatome, und
   - Verbindungen vom Typ (c) siliciumorganische Verbindungen sind, umfassend jeweils SiC-gebundene Reste mit aliphatischen Kohlenstoff-Kohlenstoff-Mehrfachbindungen und Si-gebundene Wasserstoffatome,
   wobei die Silikonzusammensetzung wenigstens eine Verbindung mit aliphatischen Kohlenstoff-Kohlenstoff-Mehrfachbindungen und wenigstens eine Verbindung mit Si-gebundenen Wasserstoffatomen enthält,
   und
ii. wenigstens einen Platin(IV)-Komplex gemäß einer der Formeln III, IV, V und VI gemäß einer der weiter oben beschriebenen Ausführungsformen.

Die vernetzbare Silikonzusammensetzung ist einfach durch Mischen der jeweils gewünschten Verbindungen erhältlich, wobei die gewünschten Verbindungen in beliebiger Reihenfolge miteinander vermischt werden können.

Mittels der hier beanspruchten vernetzbaren, insbesondere additionsvernetzbaren, Silikonzusammensetzung sind Silikonelastomere herstellbar. In solchen vernetzbaren Silikonzusammensetzungen erfolgt der Vernetzungsvorgang im Allgemeinen über eine Hydrosilylierungsreaktion, bei welcher als Katalysator üblicherweise Platin oder ein anderes Metall aus der Platingruppe eingesetzt wird. Bei der katalytisch ablaufenden Reaktion werden aliphatisch ungesättigte Gruppen mit Si-gebundenem Wasserstoff zur Reaktion gebracht, um die vernetzbare Silikonzusammensetzung über den Aufbau eines Netzwerks in den elastomeren Zustand zu überführen. Nach dem Stand der Technik erfolgt die Aktivierung der eingesetzten Katalysatoren regelmäßig thermisch oder unter Verwendung von UV/Vis-Strahlung. Erstere Vorgehensweise ist üblicherweise relativ kostenintensiv. Der Einsatz von UV/Vis-Strahlung ist zwar kostengünstiger, erfordert jedoch in der Regel besondere Sicherheitsmaßnahmen, um das Hautkrebsrisiko zu reduzieren. Solche Sicherheitsmaßnahmen sind bei Verwendung einer hier beschriebenen vernetzbaren, insbesondere additionsvernetzbaren, Silikonzusammensetzung, umfassend wenigstens einen Platin(IV)-Komplex gemäß einer der Formeln III, IV, V und VI gemäß einer der weiter oben beschriebenen Ausführungsformen, insbesondere [PtMe₃(GuaMe)], nicht zwingend notwendig. Denn die weiter oben beschriebenen Platin(IV)-Komplexe, welche jeweils wenigstens einen Organo-dihydroguajazulenyl-Liganden (GuaR)¹⁻ oder zwei 4,8-verknüpfte Organo-dihydroguajazulenyl-Radikalanionen aufweisen, zeigen eine Absorption im Vis-Bereich und sind daher mittels sichtbarer Strahlung aktivierbar.

Insbesondere Platin(IV)-Komplexe gemäß Formel III und/oder gemäß Formel IV sowie deren Lösungen oder Suspensionen in einem Lösungsmittel, welches insbesondere mit dem Lösungmittel S_{P} mischbar oder identisch ist, z. B. S_{L}, sind - insbesondere mittels des weiter oben beschriebenen Verfahrens zur Herstellung solcher Platin(IV)-Komplexe, Lösungen und Suspensionen - vorteilhafterweise auf einfache, reproduzierbare und vergleichsweise kostengünstige Weise in hoher Reinheit von 97 %, vorteilhaft von mehr als 97 %, insbesondere von mehr als 98 % oder 99 %, und guter Ausbeutevon üblicherweise ≥ 60 %, vorteilhaft ≥ 70 %, erhältlich sowie in guter Raum-Zeit-Ausbeute. Im Allgemeinen kann das Endprodukt noch Reste von Lösungsmitteln oder beispielsweise Verunreinigungen aus den Edukten enthalten. Dem Fachmann ist bekannt, dass der Gehalt an Verunreinigungen, wie z. B. von Lösungsmitteln, mittels gaschromatographischer Verfahren (GC), ggf. mit Massenspektrometrie-Kopplung (GC-MS), bestimmt werden kann.

Definitionen der Begriffe "Platin(IV)-Komplex in hoher Reinheit" und "Raum-Zeit-Ausbeute" sind bereits weiter oben gegeben.

Nach alledem sind die hier beanspruchten vernetzbaren Silikonzusammensetzungen vorteilhafterweise auf einfache, reproduzierbare und vergleichsweise kostengünstige Weise in hoher Reinheit erhältlich. Vorteilhaft ist zudem, dass die Verwendung dieser Zusammensetzungen zur Herstellung von Silikonelastomeren besonders einfach, sicher und kostengünstig realisierbar ist.

Gemäß einer Ausführungsform der hier beanspruchten vernetzbaren Silikonzusammensetzung umfasst die Silikonzusammensetzung
i. jeweils wenigstens eine Verbindung vom Typ (a) und Typ (b)
   oder
ii. wenigstens eine Verbindung vom Typ (c)
   oder
iii. jeweils wenigstens eine Verbindung vom Typ (a) und Typ (c) oder
iv. jeweils wenigstens eine Verbindung vom Typ (b) und Typ (c)
   oder
v. jeweils wenigstens eine Verbindung vom Typ (a), Typ (b) und Typ (c).

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- **Fig. 1**: ein Diagramm betreffend den Umsatz der Hydrosilylierungsreaktion von 1-Octen mit Pentamethyldisiloxan unter Verwendung von 5 ppm [PtMe₃(GuaMe)] (Dreiecke) bzw. von 5 ppm [PtMe₃(CpMe)] (Quadrate) als Präkatalysator,
- **Fig. 2**: UV/Vis-Spektrum von [PtMe₃(GuaMe)], aufgenommen in Cyclohexan (10⁻⁵ mol/L).

Während der Hydrosilylierungsreaktion von 1-Octen mit Pentamethyldisiloxan unter Verwendung von 5 ppm [PtMe₃(GuaMe)] als Präkatalysator (vgl. **Beispiel** 4, NMR-Experimente mit 5 ppm Pt) wurden ¹H-NMR-Spektren (C₆D₆, 298 K, 300 MHz) in vordefinierten zeitlichen Abständen aufgenommen. Ein erstes ¹H-NMR-Spektren wurde zum Zeitpunkt 0 h, also vor dem Starten der Hydrosilylierungsreaktion, aufgenommen. Nach etwa 0,25 h, 0,5 h, 1 h, 2 h, 4 h, 8 h und 24 h wurden weitere ¹H-NMR-Spektren aufgenommen.

Die Reaktionsgleichung für die Hydrosilylierung von 1-Octen lautet wie folgt:

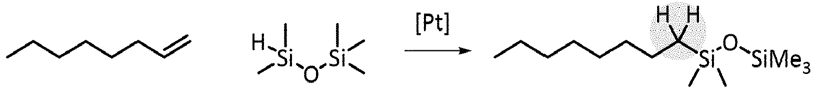

Der Umsatz wurde jeweils auf Basis der C*H*₂-Gruppe des Produktes (im Produktmolekül grau hinterlegt) mit einer Verschiebung von 0,60 ppm bestimmt. Im Rahmen der Berechnung der Umsätze wurden sämtliche vorgenannten ¹H-NMR-Spektren. berücksichtigt. In Tabelle 1 sind die berechneten Umsätze der Hydrosilylierungsreaktion von 1-Octen mit Pentamethyldisiloxan unter Einsatz von 5 ppm [PtMe₃(GuaMe)] als Präkatalysator aufgeführt (vgl. Spalte 2). Unter identischen Bedingungen wurde die Hydrosilylierungsreaktion von 1-Octen mit Pentamethyldisiloxan unter Verwendung von 5 ppm [PtMe₃(CpMe)] durchgeführt. Die berechneten Umsätze sind ebenfalls in Tabelle 1 angegeben (vgl. Spalte 3).

**Tabelle 1:**

| Zeit in h | Umsatz in % | |
|---|---|---|
| | [PtMe₃(GuaMe)] | [PtMe₃(CpMe)] |
| 0 | 0 | 0 |
| 0,25 | 96,7 | 30,1 |
| 0,5 | 98,2 | 37,7 |
| 1 | 100 | 42,7 |
| 2 | 100 | 60,2 |
| 4 | 100 | 85,4 |
| 8 | 100 | 98,3 |
| 24 | 100 | 99,7 |

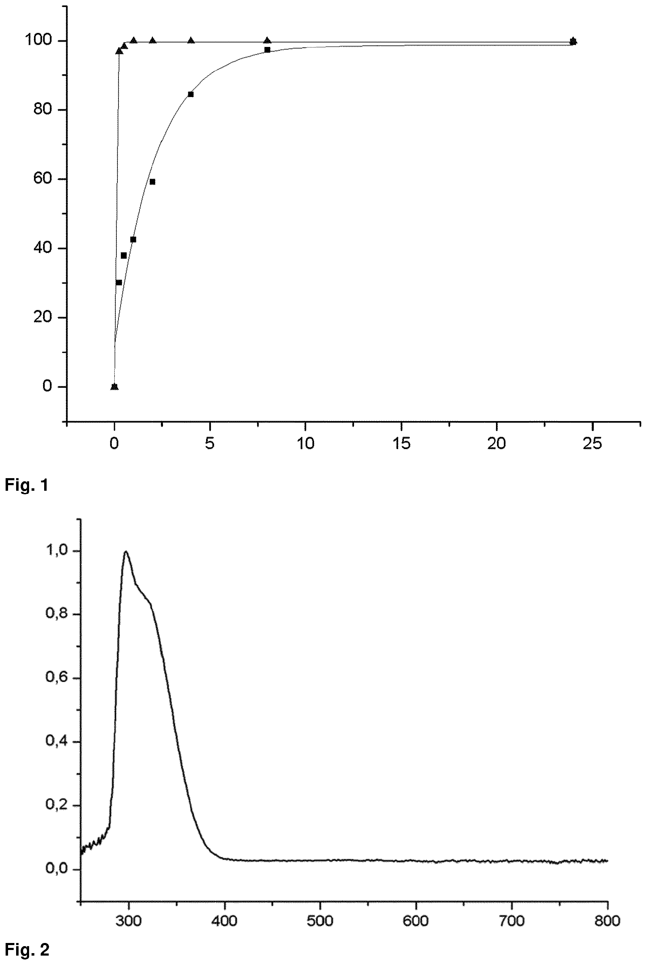

Die grafische Auswertung der in **Tabelle 1** aufgeführten Ergebnisse ist in **Fig. 1** dargestellt. Dabei ist auf der Abszisse die Zeit in Stunden (h) aufgetragen, und auf der Ordinate der Umsatz in Prozent (%). Die dreieckigen Datenpunkte stellen die bei Einsatz von [PtMe₃(GuaMe)] als Präkatalysator berechneten Umsätze dar. Die quadratischen Datenpunkte zeigen die berechneten Umsätze an, welche mit [PtMe₃(CpMe)] als Präkatalysator erzielt wurden.

Es lässt sich feststellen, dass die Hydrosilylierungsreaktion unter Einsatz von 5 ppm [PtMe₃(GuaMe)] als Präkatalysator bereits nach etwa 1 h fast vollständig abgelaufen ist. Mithin wird eine sehr zufriedenstellende Reaktionsgeschwindigkeit erreicht, welche - unter identischen Bedinungen - insbesondere höher als diejenige des als Hydrosilylierungskatalysator bekannten Pt(IV)-Komplexes [PtMe₃(CpMe)] ist. Mit anderen Worten: Der Präkatalysator [PtMe₃(GuaMe)] zeigt eine höhere Aktivität als [PtMe₃(CpMe)].

Vorteilhafterweise liegt die Pt(IV)-Verbindung [PtMe₃(GuaMe)] als Flüssigkeit vor und zeigt eine Absorption im Vis-Bereich. Das UV/Vis-Spektrum von [PtMe₃(GuaMe)] ist in **Fig. 2** dargestellt. Auf der Abszisse ist die Wellenlänge in nm abgetragen, auf der

Ordinate die Absorption in willkürlicher Einheit (engl. *arbitrary unit, a. u.*)*.* Bei > 340 nm wird eine Schulter beobachtet. Diese wird durch die beiden olefinischen Doppelbindungen des Siebenrings hervorgerufen, die mit dem aromatischen System des Fünfrings in Konjugation stehen. Eine Verunreinigung durch neutrales Guajazulen, gegebenenfalls gebildet durch Abstraktion eines H-Atoms in 8-Position des Dihydroguajazulenid-Liganden, ist im sichtbaren Bereich (blau-grün) des in **Fig. 2** gezeigten Spektrums nicht erkennbar.

Dass der Komplex [PtMe₃(GuaMe)] eine Absorption im Vis-Bereich zeigt, stellt einen weiteren Vorteil dieser hier als Präkatalysator eingesetzten Pt(IV)-Verbindung dar. Denn bei lichtinduzierten Platin-katalysierten Hydrosilylierungsreaktionen ist regelmäßig die Verwendung von UV/Vis-Licht vorgesehen, wodurch üblicherweise besondere Sicherheitsmaßnahmen erforderlich sind, um das Hautkrebsrisiko zu reduzieren. Solche Sicherheitsmaßnahmen sind bei Verwendung von [PtMe₃(GuaMe)] nicht zwingend notwendig.

Nach gängiger Lehrmeinung führt die Photolyse eines bekannten, ein Cyclopentadienyl-Anion enthaltenden Präkatalysators, wie z. B. [PtMe₃(Cp)] und [PtMe₃(CpMe)], in Gegenwart eines Silans, wie z. B. Pentamethyldisiloxan, zur Bildung eines Platin-Kolloids als aktivem Hydrosilylierungskatalysator. (L. D. Boardman, Organometallics 1992, 11, 4194 - 4201) Boardman gibt für die Hydrosilylierungsreaktion von 1-Octen mit Pentamethyldisiloxan (äquimolare Mischung) eine Präkatalysatorkonzentration von 10 ppm Platin als [PtMe₃(Cp)] an.

Bei Verwendung des im Rahmen der vorliegenden Erfindung erstmals dargestellten Präkatalysators [PtMe₃(GuaMe)] wird bereits bei 50 % der in der Literatur gewählten Katalysatorkonzentration, also bei einer Präkatalysatorkonzentration von 5 ppm Platin als [PtMe₃(GuaMe)], ein vollständiger Umsatz der Substrate 1-Octen und Pentamethyldisiloxan beobachtet, und zwar bereits nach etwa 1 h.

Ein weiterer Vorteil der vorliegend als Präkatalysator eingesetzten Verbindung [PtMe₃(GuaMe)] gegenüber Cyclopentadienyl-Anionen enthaltenden Pt(IV)-Verbindungen besteht darin, dass das zu dessen Herstellung erforderliche Guajazulen unter Verwendung nachwachsender Rohstoffe anstelle von Erdöl synthetisiert wird. Infolgedessen ist die Darstellung des Pt(IV)-Komplexes [PtMe₃(GuaMe)] vergleichsweise nachhaltig, einfach und kostengünstig realisierbar.

Mithin stellt der im Rahmen der vorliegenden Erfindung als Präkatalysator für die Hydrosilylierungsreaktion von 1-Octen mit Pentamethyldisiloxan eingesetzte Halb*sandwich*-Komplex [PtMe₃(GuaMe)] eine relativ nachhaltige und kostengünstige Alternative zu vorbekannten Hydrosilylierungs-Präkatalysatoren wie [PtMe₃(Cp)] und [PtMe₃(CpMe)] dar.

### Arbeitsvorschriften zur Synthese von Li(GuaMe) und [PtMe₃(GuaMe)]

(GuaMe)⁻ = C₁₅H₁₉⁻
= 7-*iso*-Propyl-1,4-dimethyl-8-methyl-dihydroazulenyl-Anion (Gua-8-Me)¹⁻ oder
7-*iso*-Propyl-1,4-dimethyl-6-methyl-dihydroazulenyl-Anion (Gua-6-Me)¹⁻

### Materialien und Methoden

Alle Reaktionen wurden unter Standard-Inertgasbedingungen durchgeführt. Die eingesetzten Lösungsmittel und Reagenzien wurden nach Standardprozeduren gereinigt und getrocknet.

Alle kernresonanzspektroskopischen Messungen wurden an einem Gerät des Typs Bruker AV II 300, Bruker AV II HD 300, DRX 400 oder AV III 500 durchgeführt. ¹³C-NMR-Spektren wurden standardmäßig ¹H-breitbandentkoppelt bei 300 K gemessen. ¹H- und ¹³C-NMR-Spektren wurden auf das entsprechende Restprotonensignal des Lösungsmittels als interner Standard kalibriert: ¹H: DMSO[d₆]: 2,50 ppm, C₆D₆: 7,16 ppm (s); ¹³C: DMSO[d₆]: 39,52 ppm; C₆D₆: 128,0 ppm (t). Die chemischen Verschiebungen werden in ppm angegeben und beziehen sich auf die δ-Skala. Alle Signale werden entsprechend ihres Aufspaltungsmusters mit den folgenden Abkürzungen versehen: s (Singulett), d (Dublett), dd (Doppeldublett), q (Quartett), sept (Septett).

Hochaufgelöste LIFDI-Massenspektren wurden mittels eines AccuTOF-GCv-TOF-Massenspektrometers (JEOL) erhalten.

UV/Vis-Spektren wurden mit einem Avantes AvaSpec-2048-Spektrophotometer in 10-mm-Küvetten in Cyclohexan mit Konzentrationen von 10 µM bei einer Scanrate von 600 nm/min bei Raumtemperatur aufgenommen.

Die thermogravimetrischen Untersuchungen wurden mit einer DSC-TGA 3 (Fa. Mettler Toledo) in einem Handschuhkasten durchgeführt. Die Proben wurden in Aluminiumtiegeln mit einer Heizrate von 10 K/min bis zur Endtemperatur erhitzt. Die Zersetzungstemperaturen wurden anhand der Daten der DSC-TGA ermittelt. Die Auswertung der erhaltenen Spektren erfolgte mit STARe Software der Fa. Mettler Toledo.

### Beispiel 1: Darstellung von Li(GuaMe)

Das Edukt Li(GuaMe) wurde in Anlehnung an die von Edelmann und Mitarbeitern für Lithium-7-*iso*-propyl-1,4,8-trimethyl-dihydroazulenid beschriebene Synthese hergestellt (J. Richter, P. Liebing, F. T. Edelmann, Inorg. Chim. Acta 2018, 475,18 - 27):

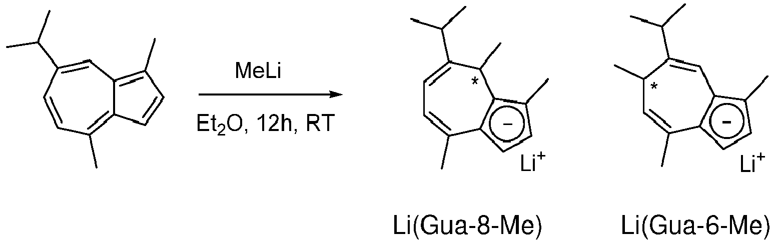

Guajazulen (4,00 g, 20,2 mmol) in 40 mL Diethylether wurde mit MeLi (1,59 M in Diethylether, 12,68 mL) bei 0 °C versetzt. Die Mischung wurde auf Raumtemperatur erwärmt und wurde für weitere 12 h gerührt. Während dieser Zeit verschwand die blaue Farbe und es bildete sich eine braune Suspension. Das ausgefallene Produkt wurde durch Filtration isoliert, mit Diethylether (3 x 20 mL) gewaschen und im Vakuum getrocknet, um Li(GuaMe) als cremefarbenen, hoch luft- und feuchtigkeitsempfindlichen Feststoff (3,35 g, 15,2 mmol, 75 %) zu erhalten.

¹H-NMR (300,1 MHz, DMSO-d₆): *δ* = 5,42 (d, ³*J_{HH}* = 3,5 Hz, 1H), 5,37 (d, ³*J_{HH}* = 3,5 Hz, 1H), 5,33 (d, ³*J_{HH}* = 7,0 Hz, 1H), 4,84 (d, ³*J_{HH}* = 6,8 Hz, 1H), 3,41 (q, ³*J_{HH}* = 7,2 Hz, 1H), 2,34 (sept, ³*J_{HH}* = 6,7 Hz, 1H), 2,02 (s, 3H), 1,99 (s, 3H), 1,04 (d, ³*J_{HH}* = 6,7 Hz, 3H), 1,00 (d, ³*J_{HH}* = 6,7 Hz, 3H), 0,70 (d, ³*J_{HH}* = 6,9 Hz, 3H) ppm; ¹³C-NMR (300,1 MHz, DMSO-d₆): *δ*= 140,1 (s, 1C), 135,4 (s, 1C), 121,3 (s, 1C), 120,3 (s, 1C), 117,7 (s, 1C), 108,7 (s, 1C), 106,4 (s, 1C), 106,2 (s, 1C), 100,6 (s, 1C), 37,0 (s, 1C), 33,5 (s, 1C), 24,2 (s, 1C), 23,2 (s, 1C), 22,7 (s, 1C), 20,0 (s, 1C), 13,9 (s, 1C)**.Anmerkung:** Die Verbindung Li(GuaMe) wurde als Gemisch der beiden Regioisomere Lithium-7-*iso*-propyl-1,4,8-trimethyl-dihydroazulenid (Li(Gua-8-Me)) und Lithium-7-*iso*-propyl-1,4,6-trimethyl-dihydroazulenid (Li(Gua-6-Me)) erhalten. Laut ¹H-NMR-spektroskopischer Analyse bestand das isolierte Isomerengemisch aus 12 mol% - 15 mol% Li(Gua-6-Me) und 85 mol% - 88 mol% Li(Gua-8-Me).

### Beispiel 2: Darstellung von [PtMe₃(GuaMe)]

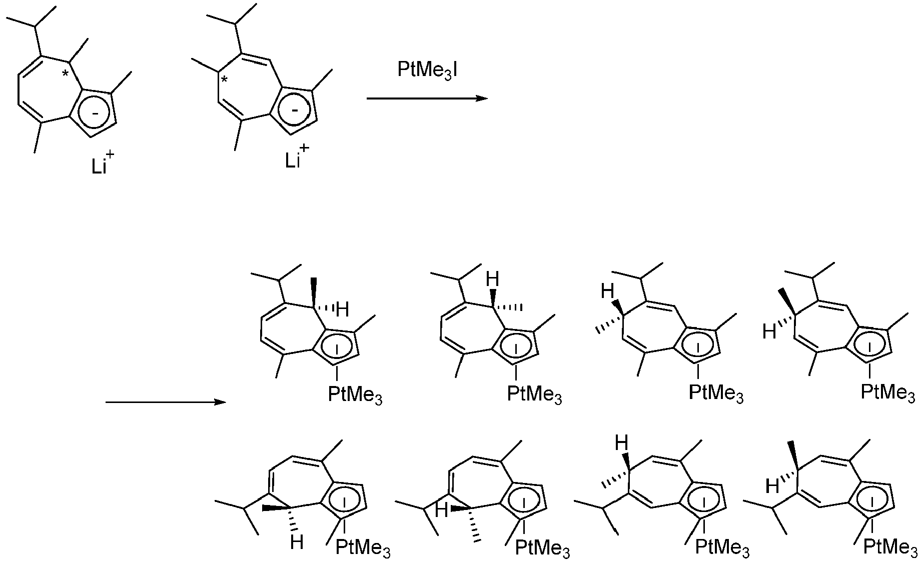

Li(GuaMe) (243 mg, 1,10 mmol) und [PtMe₃I]₄ (405 mg, 0,28 mmol) wurden in Diethylether (20 mL) suspendiert, und die Suspension wurde für 30 min bei 40 °C, dann für 4 h bei Raumtemperatur gerührt. Es wurde eine gelbe Lösung erhalten. Das Lösungsmittel wurde im Vakuum entfernt. Der ölige Rückstand wurde in Pentan (40 mL) aufgenommen, anorganische Salze und ggf. nicht umgesetzte Edukte wurden mittels Filtration abgetrennt. Das Lösungsmittel des Filtrats wurde im Vakuum entfernt, und [PtMe₃(GuaMe)] wurde als gelbgrünes Öl in guter Ausbeute (386 mg, 0,85 mmol, 77%) erhalten. Das ¹H-NMR-Spektrum zeigt ein *d.r.* von 68% (Haupt-Diastereomer, Fraktion 2) : 32% (Neben-Diastereomer, Fraktion 1). Die Verbindung lässt sich beispielsweise an einen Sublimationsfinger (Aceton/Trockeneis, -78 °C) kondensieren. Eine weitere Aufreinigung des Produktes kann mittels Säulenchromatographie (Silica oder Al₂O₃ (neutral), Hexan) erfolgen.

### Fraktion 1 ([PtMe₃(Gua-8-endo-Me)], Neben-Diastereomer):

¹H-NMR (300,1 MHz, DMSO-*d*₆): *δ*= 6,03 (dd, ³*J_{HH}* = 2,8 Hz, 1H), 5,48 (d, ³*J_{HH}* = 7,1 Hz, 1H), 5,43 (d, ³*J_{HH}* = 2,7 Hz, 1H), 5,38 (d, ³*J_{HH}* = 2,6 Hz, 1H), 3,17 (q, ³*J_{HH}* = 6,8 Hz, 1H), 2,28 (sept, ³*J_{HH}* = 5,4 Hz, 1H), 1,93 (s, 3H), 1,92 (s, 3H), 1,03 (d, ³*J_{HH}* = 2,5 Hz, 3H), 1,01 (d, ³*J_{HH}* = 2,3 Hz, 3H), 0,58 (s, *²J_{PtH}* = 40,6 Hz, 9H) ppm; ¹³C-NMR (300,1 MHz, DMSO-d₆): *δ*= 149,0 (s, 1C), 126,9 (s, 1C), 123,5 (s, 1C), 118,1 (s, 1C), 116,6 (s, 1C), 110,1 (s, 1C), 109,3 (s, 1C), 92,4 (s, 1C), 86,1 (s, 1C), 36,8 (s, 1C), 31,5 (s, 1C), 22,7 (s, 1C), 21,9 (s, 1C), 21,6 (s, 1C), 19,4 (s, 1C), 9,5 (s, 1C), -13,4 (s, ¹*J_{PtC}* = 359 Hz, 3C).

### Fraktion 2 ([PtMe₃(Gua-8-exo-Me)], Haupt-Diastereomer):

¹H-NMR (300,1 MHz, DMSO-*d*₆): *δ*= 6,06 (dd, ³*J_{HH}* = 2,6 Hz, 1H), 5,51 (d, ³*J_{HH}* = 6,7 Hz, 1H), 5,48 (d, ³*J_{HH}* = 2,6 Hz, 1H), 5,28 (d, ³*J_{HH}* = 2,6 Hz, 1H), 3,36 (q, ³*J_{HH}* = 7,0 Hz, 1H), 2,39 (sept, ³*J_{HH}* = 6,5 Hz, 1H), 1,98 (s, 3H), 1,92 (s, 3H), 1,02 (d, ³*J_{HH}* = 2,6 Hz, 3H), 0,99 (d, ³*J_{HH}* = 1,9 Hz, 3H), 0,80 (s, *²J_{PtH}* = 40,3 Hz, 9H) ppm; ¹³C-NMR (300,1 MHz, DMSO-d₆): *δ*= 150,8 (s, 1C), 128,9 (s, 1C), 124,9 (s, 1C), 120,0 (s, 1C), 117,9 (s, 1C), 109,9 (s, 1C), 108,7 (s, 1C), 91,3 (s, 1C), 83,2 (s, 1C), 36,4 (s, 1C), 30,9 (s, 1C), 21,7 (s, 1C), 21,6 (s, 1C), 21,5 (s, 1C), 20,2 (s, 1C), 10,1 (s, 1C), -15,28 (s, ¹*J_{PtC}* = 359 Hz, 3C).

**TGA** (Ts = 25 K, T_{E} = 600 K, 10 K/min): Stufen: 1, 3%-Abbau: 180,0 °C, Gesamtmasseabbau: 50,7%.

### Anmerkung:

Die ausgehend von Lithium-7-*iso*-propyl-1,4,6-trimethyl-dihydroazulenid Li(Gua-6-Me) darstellbaren Diastereomere sind weder mittels ¹H-NMR-Spektroskopie detektierbar noch isolierbar. Lediglich die Dünnschichtchromatographie liefert Hinweise auf die vier möglichen Diastereomere [PtMe₃(Gua-6-*endo*-Me)], [PtMe₃(Gua-6-*exo*-Me)], [PtMe₃(Gua-8-*endo*-Me)] und [PtMe₃(Gua-8-*exo*-Me)].

### Beispiel 3: Darstellung von a) [PtMe₃(Gua-8-Me)-CH₂-(Gua-6-Me)PtMe₃] und b) [PtMe₃(Gua-8-Me)-CH₂-(Gua-8-Me)PtMe₃]

Li(GuaMe) (243 mg, 1,10 mmol) und [PtMe₃I]₄ (405 mg, 0,28 mmol) wurden in Diethylether (20 mL) suspendiert, und die Suspension wurde für 30 min bei 40 °C, dann für 4 h bei Raumtemperatur gerührt. Es wurde eine gelbe Lösung erhalten. Das Lösungsmittel wurde im Vakuum entfernt. Der ölige Rückstand wurde in Pentan (40 mL) aufgenommen, anorganische Salze und ggf. nicht umgesetzte Edukte wurden mittels Filtration abgetrennt. Das Lösungsmittel des Filtrats wurde im Vakuum entfernt, und [PtMe₃(GuaMe)] wurde als gelbgrünes Öl in guter Ausbeute (386 mg, 0,85 mmol, 77%) erhalten.

Das ¹H-NMR-Spektrum des gelbgrünen Öls in DMSO-*d*₆ (Auswertung vgl. **Beispiel** 2) zeigt ein *d.r.* von 68% ([PtMe₃(Gua-8-*exo*-Me)]) : 32% (PtMe₃(Gua-8-*endo*-Me)]). Die beiden Diastereomere [PtMe₃(Gua-6-*endo*-Me)] und [PtMe₃(Gua-6-*exo*-Me)] sind mittels ¹H-NMR-Spektroskopie nicht detektierbar.

### a) [PtMe₃(Gua-8-Me)-CH₂-(Gua-6-Me)PtMe₃]

Die mittels ¹H-NMR-Spektroskopie untersuchte Probe wurde drei Tage lang unter Tageslichteinstrahlung bei Raumtemperatur gelagert.

Als Ergebnis wurden für die Röntgenstrukturanalyse geeignete Kristalle der dimeren Verbindung **[PtMe₃(Gua-8-Me)-CH₂-(Gua-6-Me)PtMe₃]** erhalten. Zudem wurde eine toluolische Lösung eines Kristalls massenspektrometrisch untersucht. Eine Hochauflösung eines LIFDI (FD+)-Spektrums zeigt den zugehörigen Peak bei m/z = 890,36474 (m/z berechnet für [C₃₇H₅₆Pt₂]⁺: 890,36775), und zwar in Übereinstimmung mit dem berechneten Isotopenmuster.

### b) [PtMe₃(Gua-8-Me)-CH₂-(Gua-8-Me)PtMe₃]

Das gelbgrüne Öl wurde einer Sublimation (Aceton/Trockeneis, -78 °C) sowie einer Säulenchromatographie (Silica oder Al₂O₃ (neutral), Hexan) unterzogen. Eine Probe des isolierten Diastereomerengemisches bestehend aus [PtMe₃(Gua-8-exo-Me)] und [PtMe₃(Gua-8-*endo*-Me)] wurde in DMSO gelöst. Die Lösung wurde drei Tage lang unter Tageslichteinstrahlung bei Raumtemperatur gelagert.

Ein LIFDI (FD+)-Spektrum (in Toluol) einer Probe der DMSO-Lösung zeigt einen Peak bei m/z = 890,35 zeigt (m/z berechnet für [C₃₇H₅₆Pt_{2]}⁺: 890,36775) und dient als Nachweis für die Bildung von [PtMe₃(Gua-8-Me)-CH₂-(Gua-8-Me)PtMe₃].

### Beispiel 4: Photohydrosilylierung von 1-Octen mit Pentamethyldisiloxan unter Verwendung von [PtMe₃(GuaMe)] als Präkatalysator

### NMR-Experimente mit 5 ppm Pt:

### Stammlösung (1,78 M):

500 mg 1-Octen (4,45 mmol), 661 mg Pentamethyldisiloxan (4,45 mmol), 2,5 mL C₆D₆
**Pt-Komplex-Lösung** (0,0889 mM = 0,0005 mol% im Vergleich zur Stammlösung): 0,403 mg [PtMe₃(GuaMe)] (vgl. Beispiel 2), 5 mL C₆D₆

0,25 mL **Stammlösung** und 0,25 mL **Pt-Komplex-Lösung** wurden in ein NMR-Röhrchen gefüllt. Das Röhrchen wurde geschüttelt und dann 5 min mit UV-Licht (Osram Ultra Vitalux, 300 W, 220 V, Pflanzenlampe) bestrahlt. Es wurden ¹H-NMR-Spektren zum Zeitpunkt 0 h und nach etwa 0,25 h, 0,50 h, 1 h, 2 h, 4 h, 8 h und 24 h aufgenommen.

Die Erfindung ist nicht auf eine der vorbeschriebenen Ausführungsformen beschränkt, sondern in vielfältiger Weise abwandelbar.

Man erkennt, dass die Erfindung ein Verfahren zur Herstellung von Komplexen der Edelmetalle , insbesondere von Platin, betrifft, welche wenigstens einen Organo-dihydroazulenyl-Liganden aufweisen. Gegenstand der Erfindung sind außerdem Komplexe der Edelmetalle, insbesondere von Platin, welche wenigstens einen Organo-dihydroazulenyl-Liganden aufweisen. Ferner betrifft die Erfindung die Verwendung der vorgenannten Metallkomplexe als Präkatalysatoren und/oder Katalysatoren in einer chemischen Reaktion oder als Präkursorverbindungen zur Herstellung einer Schicht, welche ein Edelmetall, insbesondere Platin, enthält, oder einer Metallschicht bestehend aus einem Edelmetall, insbesondere Platin, insbesondere auf wenigstens einer Oberfläche eines Substrats. Außerdem ist Gegenstand der Erfindung ein Substrat, insbesondere erhältlich nach einem solchen Verfahren. Zudem ist Gegenstand der Erfindung eine vernetzbare Silikonzusammensetzung, umfassend wenigstens eine Verbindung mit aliphatischen Kohlenstoff-Kohlenstoff-Mehrfachbindungen, wenigstens eine Verbindung mit Si-gebundenen Wasserstoffatomen und wenigstens einen Platin(IV)-Komplex des vorgenannten Typs. Weiterhin betrifft die Erfindung neue Alkalimetall-organodihydroazulenide, welche zur Herstellung von Metallkomplexen des vorgenannten Typs verwendbar sind.

Mit dem hier beschriebenen Verfahren sind Komplexe der Edelmetalle, insbesondere von Platin, einfach, reproduzierbar und vergleichsweise kostengünstig in hoher Reinheit und guter Ausbeute herstellbar. Das Verfahren ist - mit vergleichbarer Ausbeute und Reinheit der Zielverbindungen - auch im industriellen Maßstab durchführbar. Die mittels des weiter oben beschriebenen Verfahrens erhältlichen Metallkomplexe stellen eine relativ kostengünstige und insbesondere nachhaltige Alternative zu Cyclopentadienyl-Liganden enthaltenden Metallkomplexen dar. Und zwar insbesondere hinsichtlich einer Verwendung zur Herstellung vernetzbarer Silikonzusammensetzungen, mittels derer die Herstellung von Silikonelastomeren besonders einfach, sicher und kostengünstig realisierbar ist, als Präkatalysatoren und/oder Katalysatoren in chemischen Reaktionen. Die Platin(IV)-Komplexe, insbesondere [PtMe₃(GuaMe)], sind beispielsweise vorteilhaft als Präkatalysatoren und/oder Katalysatoren in lichtinduzierten Platin-katalysierten Hydrosilylierungsreaktionen, der Hydrierung ungesättigter Verbindungen und Polymerisationsreaktionen, bei denen die Aktivierung durch ultraviolette oder sichtbare Strahlung erfolgt, einsetzbar. Außerdem eignen sich die Platin(IV)-Komplexe, insbesondere [PtMe₃(GuaMe)], besonders als Präkursorverbindungen zur Herstellung qualitativ hochwertiger Substrate, welche wenigstens eine Platin enthaltende Schicht oder wenigstens eine Platinschicht auf wenigstens einer Oberfläche aufweisen. Weiterhin wird durch die vorliegende Erfindung das Spektrum an Alkalimetall-organo-dihydroazuleniden erweitert, welche zur Herstellung von Metallkomplexen, insbesondere des vorgenannten Typs, verwendbar sind.

Sämtliche aus den Ansprüchen, der Beschreibung und den Figuren hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Verbindung gemäß der allgemeinen Formel 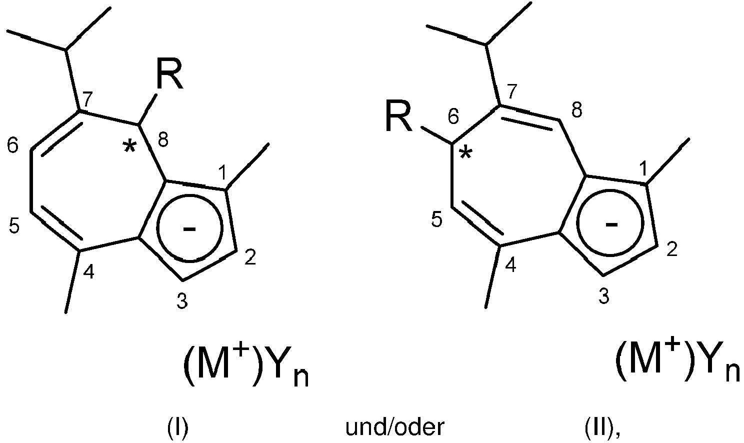 wobei
- M⁺ ein Alkalimetallkation ist,
- R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
- Y ein Neutralligand ist, welcher über wenigstens ein Donoratom an M⁺ gebunden oder koordiniert ist, wobei H₂O ausgenommen ist,
und
- *n* = 0, 1, 2, 3 oder 4 ist,
wobei
die Verbindungen Lithium-7-*iso*-propyl-1,4,8-trimethyl-dihydroazulenid und Lithium-7-*iso*-propyl-1,4-dimethyl-8-phenyl-dihydroazulenid und deren THF-Addukte und DME-Addukte ausgenommen sind.

2. Verbindung nach Anspruch 1, wobei
i. eine isomerenreine Verbindung gemäß der allgemeinen Formel I oder der allgemeinen Formel II vorliegt
oder
ii. ein Isomerengemisch vorliegt, welches ein erstes Regioisomer gemäß Formel I und ein zweites Regioisomer gemäß Formel II enthält.

3. Verbindung nach Anspruch 2, wobei ein Isomerenverhältnis erstes Regioisomer:
zweites Regioisomer ≥ 80 : 20 und < 90 : 10 beträgt.

4. Verbindung nach einem der Ansprüche 1 bis 3, wobei das Alkalimetallkation M⁺ ausgewählt ist aus der Gruppe bestehend aus Li+, Na⁺ und K+.

5. Verbindung nach einem der Ansprüche 1 bis 4, wobei der Neutralligand Y
a) ein aprotisch-polares Lösungsmittel ist
oder
b) ein Kronenether ist,
welcher ausgewählt ist aus der Gruppe bestehend aus makrocyclischen Polyethern und deren Aza-, Phospha- und Thia-Derivaten,
wobei ein Innendurchmesser des Kronenethers und ein lonenradius des Alkalimetallkations M⁺ miteinander korrespondieren.

6. Verwendung einer Verbindung gemäß der allgemeinen Formel I und/oder der allgemeinen Formel II gemäß einem der Ansprüche 1 bis 5 zur Herstellung eines Platin(IV)-Komplexes gemäß der allgemeinen Formel 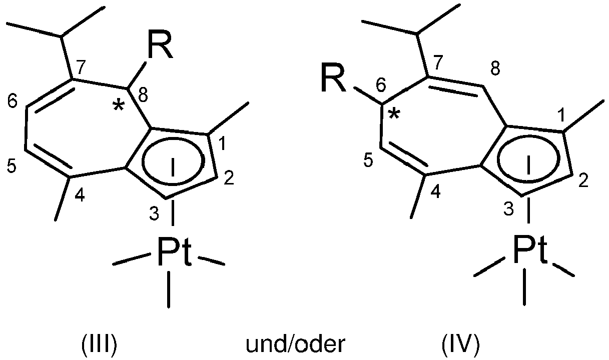 wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten.

7. Verfahren zur Herstellung eines Platin(IV)-Komplexes gemäß der allgemeinen Formel 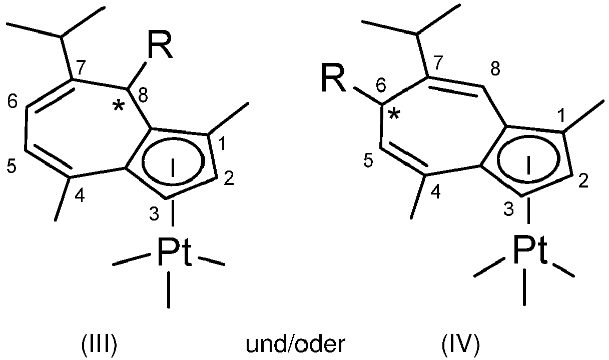 wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
unter Verwendung
einer Verbindung gemäß der allgemeinen Formel I und/oder der allgemeinen Formel II gemäß einem der Ansprüche 1 bis 5,
umfassend die Schritte:
A. Zurverfügungstellung
- der Verbindung gemäß der allgemeinen Formel I und/oder der allgemeinen Formel II
und
- einer Platinvorstufe,
B. Synthese des Platin(IV)-Komplexes gemäß der allgemeinen Formel III und/oder der allgemeinen Formel IV
unter Verwendung der Verbindung gemäß der allgemeinen Formel I und/oder der allgemeinen Formel II als Edukt
in einem Lösungsmittel S_{P},
C. Optionale Isolierung des in Schritt B. synthetisierten Platin(IV)-Komplexes gemäß der allgemeinen Formel III und/oder der allgemeinen Formel IV.

8. Verfahren nach Anspruch 7, wobei das Lösungsmittel S_{P} wenigstens ein Lösungsmittel umfasst, welches ausgewählt ist aus der Gruppe bestehend aus aprotisch-polaren Lösungsmitteln, aliphatischen Kohlenwasserstoffen, aromatischen Kohlenwasserstoffen, siliciumorganischen Verbindungen, und Mischungen davon.

9. Verfahren nach Anspruch 7 oder 8, wobei die Synthese in Schritt B. wenigstens eine salzmetathetische Umsetzung umfasst.

10. Verfahren nach einem der Ansprüche 7 bis 9, wobei die in Schritt A. zur Verfügung zu stellende Platinvorstufe ausgewählt ist aus der Gruppe bestehend aus Trimethylplatin(IV)-iodid, Trimethylplatin(IV)-bromid und Trimethylplatin(IV)-chlorid, und Mischungen davon, wobei die Zurverfügungstellung
i. als Lösung, welche die Platinvorstufe und ein Lösungsmittel S_{A} umfasst, oder
ii. als Feststoff
erfolgt.

11. Platin(IV)-Komplex gemäß der allgemeinen Formel 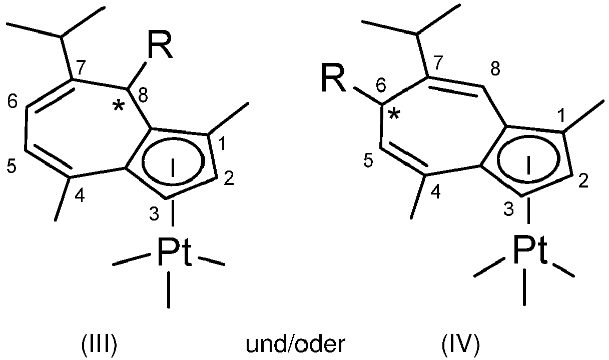 oder
Lösung oder Suspension, umfassend einen Platin(IV)-Komplex gemäß der allgemeinen Formel 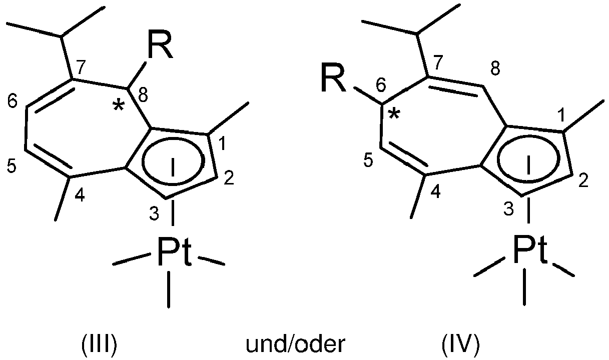 und ein Lösungsmittel, welches insbesondere mit dem Lösungmittel S_{P} mischbar oder identisch ist,
wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten,
erhalten oder erhältlich nach einem Verfahren gemäß einem der Ansprüche 7 bis 10.

12. Platin(IV)-Komplex gemäß der allgemeinen Formel 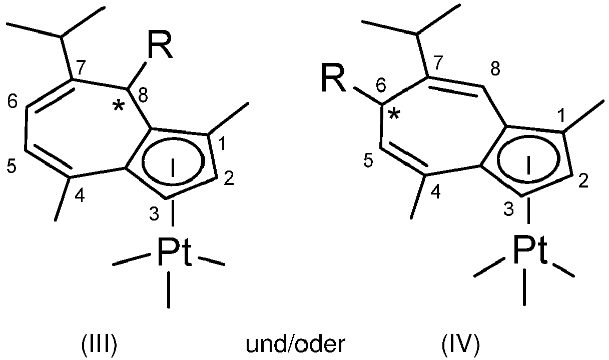 oder
Lösung oder Suspension, umfassend einen Platin(IV)-Komplex gemäß der allgemeinen Formel 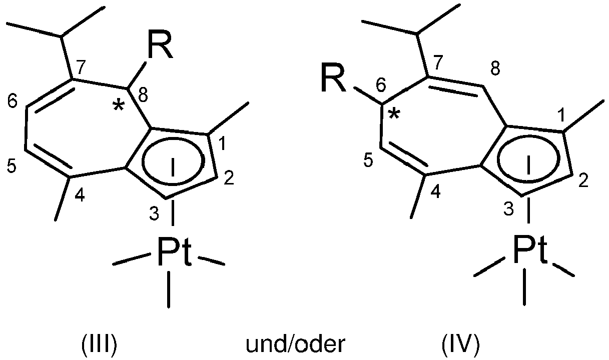 und ein Lösungsmittel,
wobei
R ausgewählt ist aus der Gruppe bestehend aus primären, sekundären, tertiären Alkyl-, Alkenyl- und Alkinylresten mit 1 bis 10 Kohlenstoffatomen, cyclischen Alkylresten mit 3 bis 10 Kohlenstoffatomen, einem Benzylrest, einkernigen Arylresten, mehrkernigen Arylesten, einkernigen Heteroarylresten und mehrkernigen Heteroarylresten.

13. Platin(IV)-Komplex oder Lösung oder Suspension nach Anspruch 11 oder 12, wobei ein Diastereomerengemisch vorliegt umfassend
- ein erstes Diastereomer gemäß Formel III.D1 und ein zweites Diastereomer gemäß Formel III.D2, 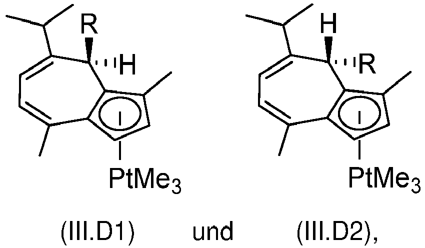 und/oder
- ein drittes Diastereomer gemäß Formel IV.D3 und ein viertes Diastereomer gemäß Formel IV.D4, 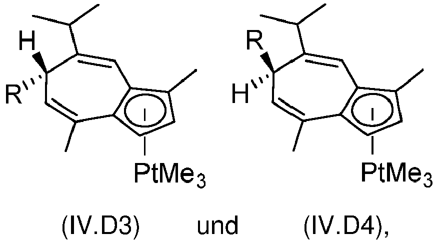 wobei jedes Diastereomer als ein Enantiomerengemisch vorliegt.

14. Platin(IV)-Komplex oder Lösung oder Suspension nach einem der Ansprüche Anspruch 11 bis 13, wobei wenigstens ein Diastereomer als ein Racemat vorliegt.

15. Platin(IV)-Komplex oder Lösung oder Suspension nach Anspruch 13 oder 14, wobei ein Gemisch zweier Diastereomere vorliegt und wobei ein Diastereomerenverhältnis
- erstes Diastereomer: zweites Diastereomer
oder
- drittes Diastereomer: viertes Diastereomer
≥ 60 : 40 und < 90 : 10 beträgt.

16. Platin(IV)-Komplex oder Lösung oder Suspension nach einem der Ansprüche 13 bis 15, wobei ein Diastereomerengemisch vorliegt, bestehend aus dem ersten Diastereomer gemäß Formel III.D1 und dem zweiten Diastereomer gemäß Formel III.D2, 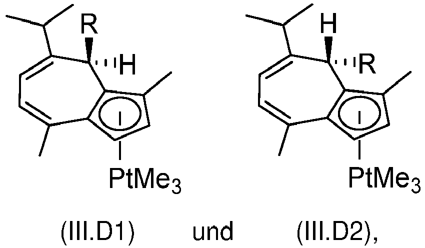 wobei jedes Diastereomer als ein Enantiomerengemisch vorliegt.

17. Platin(IV)-Komplex gemäß Formel 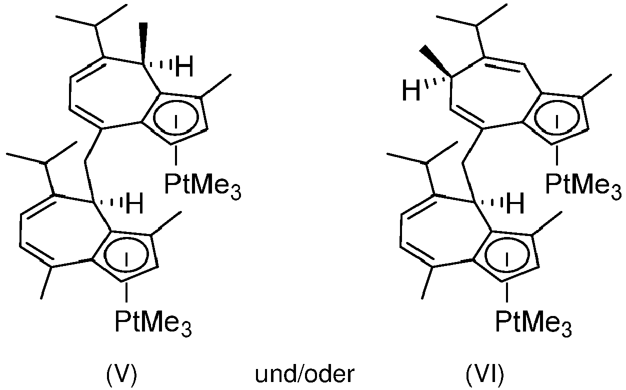 oder
Lösung oder Suspension, umfassend einen Platin(IV)-Komplex gemäß Formel V und/oder Formel VI und ein Lösungsmittel S_{D}, welches ein aprotisch-unpolares oder ein aprotisch-polares Lösungsmittel umfasst oder ist.

18. Verwendung wenigstens eines Platin(IV)-Komplexes nach einem der Ansprüche 11 bis 17 oder wenigstens einer Lösung oder Suspension nach einem der Ansprüche 11 bis 17, als
i. Präkatalysator oder Präkatalysator enthaltende Lösung oder Suspension in einer chemischen Reaktion
und/oder
ii. Katalysator oder Katalysator enthaltende Lösung oder Suspension in einer chemischen Reaktion
und/oder
iii. Präkursorverbindung oder Präkursorverbindung enthaltende Lösung oder Suspension zur Herstellung wenigstens einer Schicht bestehend aus Platin oder wenigstens einer Schicht, welche Platin enthält, auf wenigstens einer Oberfläche eines Substrats.

19. Verfahren zur Durchführung einer chemischen Reaktion unter Verwendung wenigstens eines Platin(IV)-Komplexes nach einem der Ansprüche 11 bis 17 oder wenigstens einer Lösung oder Suspension nach einem der Ansprüche 11 bis 17,
umfassend die Schritte:
A) Zurverfügungstellung
des wenigstens einen Platin(IV)-Komplexes gemäß der allgemeinen Formel III und/oder IV und/oder gemäß Formel V und/oder VI
oder
der wenigstens einen Lösung oder Suspension,
umfassend einen Platin(IV)-Komplex gemäß der allgemeinen Formel III und/oder IV und ein Lösungsmittel, welches insbesondere mit dem Lösungmittel S_{P} mischbar oder identisch ist, und/oder umfassend einen Platin(IV)-Komplex gemäß Formel V und/oder VI und ein Lösungsmittel S_{D}, welches ein aprotisch-unpolares oder ein aprotisch-polares Lösungsmittel umfasst oder ist,
und
B) Durchführung der chemischen Reaktion unter Verwendung des wenigstens einen Platin(IV)-Komplexes als Präkatalysator und/oder als Katalysator.

20. Verfahren zur Herstellung
i. wenigstens einer Schicht bestehend aus Platin
oder
ii. wenigstens einer Schicht, welche Platin enthält,
auf wenigstens einer Oberfläche eines Substrats unter Verwendung wenigstens eines Platin(IV)-Komplexes nach einem der Ansprüche 11 bis 16 oder wenigstens einer Lösung oder Suspension nach einem der Ansprüche 11 bis 16,
umfassend die Schritte:
A) Zurverfügungstellung
des wenigstens einen Platin(IV)-Komplexes gemäß der allgemeinen Formel III und/oder IV
oder
der wenigstens einen Lösung oder Suspension,
umfassend einen Platin(IV)-Komplex gemäß der allgemeinen Formel III und/oder IV und ein Lösungsmittel, welches insbesondere mit dem Lösungmittel S_{P} mischbar oder identisch ist,
und
B) Abscheidung
i. der wenigstens einen Schicht bestehend aus Platin
oder
ii. der wenigstens einen Schicht, welche Platin enthält,
auf der wenigstens einen Oberfläche des Substrats unter Verwendung des wenigstens einen Platin(IV)-Komplexes gemäß der allgemeinen Formel III und/oder IV als Präkursorverbindung.

21. Substrat, welches auf wenigstens einer Oberfläche
i. wenigstens eine Schicht bestehend aus Platin,
oder
ii. wenigstens eine Schicht, welche Platin enthält,
aufweist,
wobei die wenigstens eine Schicht bestehend aus Platin oder die wenigstens eine Schicht, welche Platin enthält, herstellbar oder hergestellt ist unter Verwendung
wenigstens eines Platin(IV)-Komplexes nach einem der Ansprüche 11 bis 16 oder wenigstens einer Lösung oder Suspension nach einem der Ansprüche 11 bis 16.

22. Vernetzbare Silikonzusammensetzung, umfassend
i. wenigstens eine Verbindung ausgewählt aus der Gruppe bestehend aus Verbindungen vom Typ (a), Verbindungen vom Typ (b) und Verbindungen vom Typ (c), wobei
- Verbindungen vom Typ (a) organische Verbindungen und siliciumorganische Verbindungen sind, umfassend jeweils wenigstens zwei Reste mit aliphatischen Kohlenstoff-Kohlenstoff-Mehrfachbindungen,
- Verbindungen vom Typ (b) siliciumorganische Verbindungen sind, umfassend jeweils wenigstens zwei Si-gebundene Wasserstoffatome, und
- Verbindungen vom Typ (c) siliciumorganische Verbindungen sind, umfassend jeweils SiC-gebundene Reste mit aliphatischen Kohlenstoff-Kohlenstoff-Mehrfachbindungen und Si-gebundene Wasserstoffatome, wobei die Silikonzusammensetzung wenigstens eine Verbindung mit aliphatischen Kohlenstoff-Kohlenstoff-Mehrfachbindungen und wenigstens eine Verbindung mit Si-gebundenen Wasserstoffatomen enthält,
und
ii. wenigstens einen Platin(IV)-Komplex nach einem der Ansprüche 11 bis 17.

23. Verbindung nach einem der Ansprüche 1 bis 5 oder Verwendung nach Anspruch 6 oder Verfahren nach einem der Ansprüche 7 bis 10 oder Platin(IV)-Komplex oder Lösung oder Suspension nach einem der Ansprüche 11 bis 17 oder Verwendung nach Anspruch 18 oder Verfahren nach Anspruch 19 oder 20 oder Substrat nach Anspruch 21 oder vernetzbare Silikonzusammensetzung nach Anspruch 22, wobei R = Me ist.
